(19) Europäisches Patentamt
European Patent Office
Office européen des brevets

(11) EP 2 752 191 A1

(12) **EUROPEAN PATENT APPLICATION**

(43) Date of publication:
**09.07.2014 Bulletin 2014/28**

(21) Application number: **13305008.8**

(22) Date of filing: **07.01.2013**

(51) Int Cl.:
***A61K 31/404*** (2006.01) ***A61K 31/455*** (2006.01)
***A61K 31/44*** (2006.01) ***A61P 35/00*** (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **SANOFI**
**75008 Paris (FR)**

(72) Inventors:
- **Debussche, Laurent**
**75008 PARIS (FR)**
- **Nicolas, Jean-Paul**
**75008 PARIS (FR)**
- **Rowley, Steve**
**BRIDGEWATER, NJ New Jersey NJ 08807 (US)**
- **Watters, James**
**BRIDGEWATER, NJ New Jersey NJ 08807 (US)**
- **Windenberger, Fanny**
**75008 PARIS (FR)**

(74) Representative: **Bouron, Estelle et al**
**Sanofi**
**Département Brevets**
**54, rue La Boétie**
**75008 Paris (FR)**

(54) **Compositions and methods using hdm2 antagonist and mek inhibitor**

(57) Methods of causing growth inhibition of cancer cells are provided, wherein the methods comprise contacting cancer cells with an HDM2 antagonist and a MEK inhibitor. Compositions in which the HDM2 antagonist and MEK inhibitor are combined also are provided.

EP 2 752 191 A1

**Description**

BACKGROUND

HDM2 negatively regulates p53 activity by binding to the transactivation domain of p53 and by acting as a p53-specific E3 ubiquitin ligase. In response to diverse stresses, p53 can induce cell cycle arrest and apoptosis.

Compound (1)

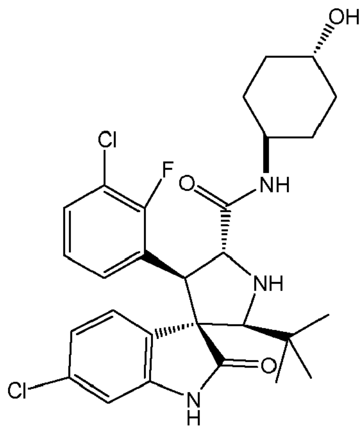

is a selective antagonist of HDM2. (WO2012/065022). Binding of Compound (1) to HDM2 disrupts the interaction between HDM2 and p53 thereby inhibiting the ability of HDM2 to bind to and inhibit p53.

Compound (4), N-((S)-2,3-dihydroxypropyl)-3-(2-fluoro-4-iodo-phenylamino)isonicotinamide (also referred to as Pimasertib, MSC1936369, or AS703026) is a novel, allosteric inhibitor of MEK. It possesses relatively high potency and selectivity, having no activity against 217 kinases or 90 non-kinase targets when tested at 10 μM. (WO06/045514).

Tumor cells treated with inhibitors of MEK kinases typically respond via inhibition of phosphorylation of ERK, down-regulation of Cyclin D, induction of G1 arrest, and finally undergoing apoptosis. The effect of MEK inhibition on tumor growth in selected mutant xenograft tumors that express mutant B-raf and selected Ras mutant tumors are described in D. B. Solit et al., Nature 2006; 439: 358-362.

Predominant toxicities reported for MEK inhibitors in human patients include diarrhea, fatigue, rash, vomiting, nausea, and reversible visual disturbances. (Haura et al., Clin Cancer Res (2010) 16(8):2450-2457). In particular, similar to epidermal growth factor receptor inhibitors, dermatologic toxicities such as papulopustular rash, xerosis, and pruritus are associated with MEK inhibitors. (Baladula et al., Invest New Drugs (2011) 29:1114-1121).

There remains a need for a cancer therapy that is more effective in inhibiting cell proliferation and tumor growth while minimizing patient toxicity. There is a particular need for an HDM2 antagonist and/or MEK inhibitor therapy that is made more efficacious without substantially increasing, or even maintaining or decreasing, the dosages of HDM2 antagonist and/or MEK inhibitor traditionally used in the art.

**SUMMARY**

Provided herein are compositions for the treatment of patients having cancer and methods of using the compositions that employ an HDM2 antagonist in combination with an inhibitor of MEK pathway signaling.

In a first aspect, methods for treating a subject having cancer are provided comprising administering to the subject an effective amount of Compound (1):

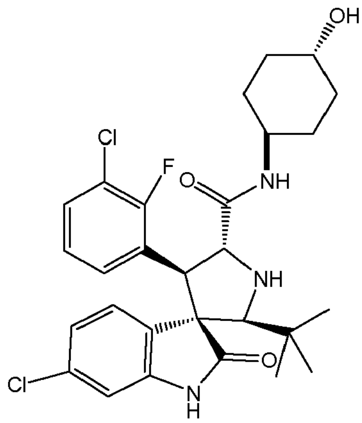

or a pharmaceutically acceptable salt thereof and Compound (4)

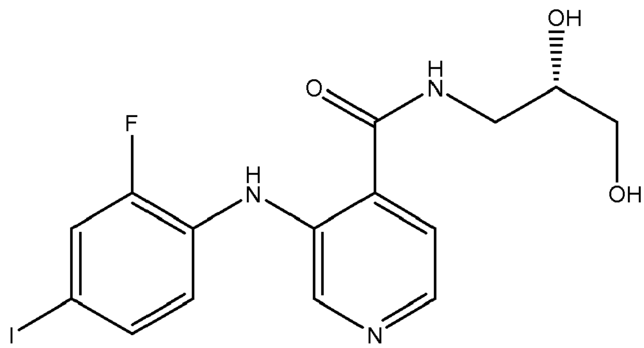

or a pharmaceutically acceptable salt thereof. In some embodiments, Compound (1) and/or Compound (4) are administered with a pharmaceutically acceptable carrier. In some embodiments, Compound (1) and Compound (4) are administered in either separate or single dosage forms. In some embodiments, Compound (1) and Compound (4) are administered at different times. In some embodiments, Compound (1) and Compound (4) are administered at substantially the same time. In some embodiments, the effective amount achieves a synergistic effect in the treatment of the subject in need thereof.

In a second aspect, a combination is provided for use in treating a subject having cancer, the combination comprising a therapeutically effective amount of Compound (1), or a pharmaceutically acceptable salt thereof, and Compound (4), or a pharmaceutically acceptable salt thereof. In one embodiment, the combination is in separate dosage forms, while in another embodiment, the combination is in a single dosage form. In a third aspect, a use of a combination comprising a therapeutically effective amount of Compound (1), or a pharmaceutically acceptable salt thereof, and Compound (4), or a pharmaceutically acceptable salt thereof, for the preparation of a medicament for use in treatment of lymphoma is provided. In one embodiment, the combination is in separate dosage forms, while in another embodiment, the combination is in a single dosage form.

In some embodiments of the second and third aspect, the therapeutically effective amount achieves a synergistic effect in the treatment of the subject in need thereof.

In a fourth aspect, a composition comprising Compound (1), or a pharmaceutically acceptable salt thereof, and Compound (4), or a pharmaceutically acceptable salt thereof is provided. The composition can further comprise a pharmaceutically acceptable carrier.

In a fifth aspect, kits are provided comprising: (A) Compound (1), or a pharmaceutically acceptable salt thereof; (B) Compound (4), or a pharmaceutically acceptable salt thereof; and (C) instructions for use. In some embodiments, the instructions are for the use of Compounds (1) and (4) in the treatment of cancer.

In some embodiments of aspects four and five, a therapeutically effective amount of Compound (1) and Compound (4) provided. In some embodiments, a therapeutically effective amount achieves a synergistic effect in the treatment of the subject in need thereof.

In some embodiments of aspects one through five, the cancer expresses mutant Nras. In one example, the mutant Nras expressing cancer is melanoma. In another example, the Nras mutant expressing cancer is bladder cancer. In other embodiments, the cancer expresses mutant Braf. In one example, the Braf mutant expressing cancer is melanoma.

## BRIEF DESCRIPTION OF THE DRAWINGS

FIG. 1 shows a table of the mean synergy score and False Discovery Rate (FDR) for HDM2 antagonist Compounds (1), (2), and (3) in combination with MEK inhibitor Compounds (6), (7), (8), (9), and (10), in the indicated cell lines (A: 769-P, B: A375, C: BPH1, D:D32, E: Cake-1, F: DBTRG-05MG, G: DK-MG, .H: H4, I: HCT-116, J: HT-1197, **K:** JAR, **L:** LNCaP-sa, **M:** MCF7, **N:** MG-63, **O:** MV-4-11, **P:** RKO, **Q:** SJSA-1, **R:** SK-N-AS, **S:** SR, and **T:** SW48).

FIG. 2 shows: A) percent growth inhibition of C32 cells when treated with the indicated amounts of Compound (1) in combination with Compound (6); B) the level of growth inhibition of C32 cells versus drug concentration where ♦ is Compound (1), ■ is Compound (6), and ▲ is Compound (1) in combination with Compound (6).

FIG 3 shows: A) percent growth inhibition of MV4-11 cells when treated with the indicated amounts of Compound (1) in combination with Compound (4), and B) the level of growth inhibition of MV4-11 cells versus drug concentration where ♦ is Compound (1), ■ is Compound (6), and ▲ is Compound (1) in combination with Compound (6).

FIG. 4 shows: A) percent growth inhibition of A375 cells when treated with the indicated amounts of Compound (1) in combination with Compound (6), and B) an isobologram of growth inhibition of A375 cells treated with the indicated amounts of Compound (1) in combination with Compound (6) compared to additive growth inhibition.

FIG. 5 shows: A) percent growth inhibition of Vemurafenib resistant A375 cells when treated with the indicated amounts of Compound (1) in combination with Compound (6), and B) an isobologram of growth inhibition of Vemurafenib resistant A375 cells treated with the indicated amounts of Compound (1) in combination with Compound (6) compared to additive growth inhibition.

FIG. 6 shows an isobologram of growth inhibition of C8161 cells treated with the Compound (1) in combination with Compound (4) (A) or with Compound (5) compared to additive growth inhibition where the y-axis is [Compound (4) or (5)]/IC50 of Compound (4) or (5), respectively and the x-axis is [Compound (1)]/IC50 of Compound (1); circles and triangles represent a first and second experiment, respectively.

FIG. 7 shows an isobologram of growth inhibition ofHCT116 cells treated with Compound (1) in combination with Compound (4) (A) or with Compound (5) (B) where the y-axis is [Compound (4) or (5)]/IC50 of Compound (4) or (5), respectively and the x-axis is [Compound (1)]/IC50 of Compound (1); circles, triangles, squares, and astrices represent experiments 1-4, respectively.

FIG. 8 shows an isobologram of growth inhibition of SK-MEL-103 cells treated with Compound (1) in combination with Compound (4) (A) or with Compound (5) (B) where the y-axis is [Compound (4) or (5)]/IC50 of Compound (4) or (5), respectively and the x-axis is [Compound (1)]/IC50 of Compound (1); circles, triangles, and squares represent experiments 1-3, respectively.

FIG. 9 shows an isobologram of growth inhibition of UACC-62 cells treated with Compound (1) in combination with Compound (4) (A) or with Compound (5) (B) where the y-axis is [Compound (4) or (5)]/IC50 of Compound (4) or (5), respectively and the x-axis is [Compound (1)]/IC50 of Compound (1); circles, triangles, and squares represent experiments 1-3, respectively.

FIG. 10 shows an isobologram of growth inhibition ofHT-1197 cells treated with Compound (1) in combination with Compound (4) (A) or with Compound (5) (B) where the y-axis is [Compound (4) or (5)]/IC40 of Compound (4) or (5), respectively and the x-axis is [Compound (1)]/IC40 of Compound (1); triangles, squares, and astrices represent experiments 1-3, respectively.

FIG. 11 shows an isobologram of growth inhibition of KU-19-19 cells treated with Compound (1) in combination with Compound (4) (A) or with Compound (5) (B) where the y-axis is [Compound (4) or (5)]/IC50 of Compound (4) or (5), respectively and the x-axis is [Compound (1)]/IC50 of Compound (1); circles, triangles, squares, and astrices represent experiment 1-4, respectively.

FIG. 12 shows an isobologram of growth inhibition of A375 cells treated with Compound (1) in combination with Compound (4) (A) or with Compound (5) (B) where the y-axis is [Compound (4) or (5)]/IC50 of Compound (4) or (5), respectively and the x-axis is [Compound (1)]/IC50 of Compound (1); circles and triangles represent experiments 1 and 2, respectively.

FIG. 13 shows an isobologram of growth inhibition C32 cells treated with Compound (1) in combination with Compound (4) (A) or with Compound (5) (B) where the y-axis is [Compound (4) or (5)]/IC50 of Compound (4) or (5), respectively and the x-axis is [Compound (1)]/IC50 of Compound (1); circles, triangles, and squares represent experiments 1-3, respectively.

FIG. 14 shows an isobologram of growth inhibition SK-MEL-147 cells treated with Compound (1) in combination with Compound (4) (A) or with Compound (5) (B) where the y-axis is [Compound (4) or (5)]/IC50 of Compound (4) or (5), respectively and the x-axis is [Compound (1)]/IC50 of Compound (1); circles, triangles, and squares represent

experiments 1-3, respectively.

FIG. 15 shows an isobologram of growth inhibition DV90 cells treated with Compound (1) in combination with Compound (4) (A) or with Compound (5) (B) where the y-axis is [Compound (4) or (5)]/IC50 of Compound (4) or (5), respectively and the x-axis is [Compound (1)]/IC50 of Compound (1); circles, triangles, and squares represent experiments 1-3, respectively.

FIG. 16 shows an isobologram of growth inhibition H460 cells treated with Compound (1) in combination with Compound (4) (A) or with Compound (5) (B) where the y-axis is [Compound (4) or (5)]/IC50 of Compound (4) or (5), respectively and the x-axis is [Compound (1)]/IC50 of Compound (1); circles, triangles, and squares represent experiments 1-3, respectively.

FIG. 17 shows an isobologram of growth inhibition UM-UC-3 cells treated with Compound (1) in combination with Compound (4) (A) or with Compound (5) (B) where the y-axis is [Compound (4) or (5)]/IC50 of Compound (4) or (5), respectively and the x-axis is [Compound (1)]/IC50 of Compound (1); circles and triangles represent experiments 1 and 2, respectively.

## DETAILED DESCRIPTION

Compositions and methods of use thereof for treatment of Nras mutant expressing bladder cancer or melanoma and for the treatment of mutant Braf expressing melanoma are provided herein that employ an HMD2 antagonist in combination with a MEK inhibitor. The compositions provided herein afford better efficacy and/or potency than the single agent components of the compositions. As shown herein for the first time, Compound (1) demonstrates synergistic activity in combination with Compound (4) in causing growth inhibition of Nras mutant expressing bladder cancer cells, mutant Nras expressing melanoma cells, and mutant Braf expressing melanoma cells.

Methods for treating a subject having cancer such as Nras mutant expressing bladder cancer or melanoma and for the treatment of mutant Braf expressing melanoma are provided. In one embodiment, the methods comprise administering to the patient a therapeutically effective amount of an HDM2 antagonist and MEK inhibitor as further described herein.

In some embodiments, the methods and compositions comprise a HDM2 antagonist having the formula (1):

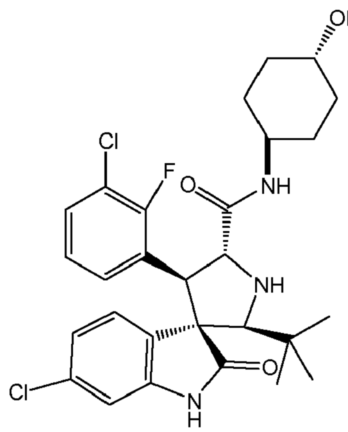

(1).

The compound according to formula (1) is referred to herein as "Compound (1)". The preparation and properties of Compound (1) are provided in, for example, International Patent Publication No. WO2012/065022, particularly Example 11, p. 169-171 therein. The entire contents of WO2012/065022 are incorporated herein by reference.

In some embodiments, the methods and compositions comprise a MEK inhibitor having the formula (4):

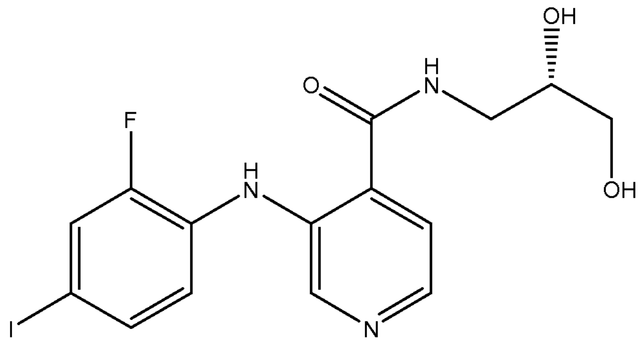

(4)

The compound according to formula (4) is referred to herein as "Compound (4)". The preparation and properties of Compound (4), also referred to as Pimasertib, are provided in, for example, International Patent Publication No. WO06/045514, particularly Example 115 and Table 1 therein. The entire contents of WO06/045514 are incorporated herein by reference.

In some embodiments, the compounds described herein are unsolvated. In other embodiments, one or both of the compounds used are in solvated form. As known in the art, the solvate can be any ofpharmaceutically acceptable solvent, such as water, ethanol, and the like. In general, the presence of a solvate or lack thereof does not have a substantial effect on the efficacy of Compounds (1) or (4) described above. Although the Compounds (1) and (4) are depicted in their neutral forms, in some embodiments, these compounds are used in a pharmaceutically acceptable salt form. The salt can be obtained by any of the methods well known in the art. A "pharmaceutically acceptable salt" of the compound refers to a salt that is pharmaceutically acceptable and that retains pharmacological activity. It is understood that the pharmaceutically acceptable salts are non-toxic. Additional information on suitable pharmaceutically acceptable salts can be found in Remington's Pharmaceutical Sciences, 17th ed., Mack Publishing Company, Easton, PA, 1985, or S. M. Berge, et al., "Pharmaceutical Salts," J. Pharm. Sci., 1977; 66:1-19, both of which are incorporated herein by reference.

Examples of pharmaceutically acceptable acid addition salts include those formed with inorganic acids such as hydrochloric acid, hydrobromic acid, sulfuric acid, nitric acid, phosphoric acid, as well as those salts formed with organic acids, such as acetic acid, trifluoroacetic acid, propionic acid, hexanoic acid, cyclopentanepropionic acid, glycolic acid, pyruvic acid, lactic acid, oxalic acid, maleic acid, malonic acid, succinic acid, fumaric acid, tartaric acid, citric acid, benzoic acid, cinnamic acid, 3-(4-hydroxybenzoyl)benzoic acid, mandelic acid, methanesulfonic acid, ethanesulfonic acid, 1,2-ethanedisulfonic acid, 2-hydroxyethanesulfonic acid, benzenesulfonic acid, 4-chlorobenzenesulfonic acid, 2-naphthalenesulfonic acid, 4-toluenesulfonic acid, camphorsulfonic acid, glucoheptonic acid, 4,4'-methylenebis-(3-hydroxy-2-ene-1-carboxylic acid), 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid, p-toluenesulfonic acid, and salicylic acid.

In some embodiments of the methods and uses provided herein, Compound (1) is administered simultaneously with Compound (4). Simultaneous administration typically means that both compounds enter the patient at precisely the same time. However, simultaneous administration also includes the possibility that Compound (1) and Compound (4) enter the patient at different times, but the difference in time is sufficiently miniscule that the first administered compound is not provided the time to take effect on the patient before entry of the second administered compound. Such delayed times typically correspond to less than 1 minute, and more typically, less than 30 seconds.

In one example, wherein the compounds are in solution, simultaneous administration can be achieved by administering a solution containing the combination of compounds. In another example, simultaneous administration of separate solutions, one of which contains the Compound (1) and the other of which contains Compound (4), can be employed. In one example wherein the compounds are in solid form, simultaneous administration can be achieved by administering a composition containing the combination of compounds.

In other embodiments, Compound (1) and Compound (4) are not simultaneously administered. In one set of embodiments, the Compound (1) is administered before Compound (4). In another set of embodiments, Compound (4) is administered before Compound (1). The time difference in non-simultaneous administrations can be greater than 1 minute, and can be, for example, precisely, at least, up to, or less than 5 minutes, 10 minutes, 15 minutes, 30 minutes, 45 minutes, 60 minutes, two hours, three hours, six hours, nine hours, 12 hours, 24 hours, 36 hours, or 48 hours. In another embodiment, the first administered compound is provided time to take effect on the patient before the second administered compound is administered. Generally, the difference in time does not extend beyond the time for the first administered compound to complete its effect in the patient, or beyond the time the first administered compound is completely or substantially eliminated or deactivated in the patient.

In some embodiments, one or both of the Compounds (1) and (4) are administered in a therapeutically effective (i.e., therapeutic) amount or dosage. A "therapeutically effective amount" is an amount of Compound (1) or Compound (4) that, when administered to a patient by itself, effectively treats the cancer (for example, inhibits tumor growth, stops tumor growth, or causes tumor regression). An amount that proves "therapeutically effective amount" in a given instance, for a particular subject, may not be effective for 100% of subjects similarly treated for the disease or condition under consideration, even though such dosage is deemed a "therapeutically effective amount" by skilled practitioners. The amount of the compound that corresponds to a therapeutically effective amount is affected by the type of cancer, stage of the cancer, the age of the patient being treated, and other facts.

In other embodiments, one or both of the Compounds (1) and (4) are administered in a sub-therapeutically effective amount or dosage. A sub-therapeutically effective amount is an amount of Compound (1) or (4) that, when administered to a patient by itself, does not completely inhibit over time the biological activity of the intended target.

Whether administered in therapeutic or sub-therapeutic amounts, the combination of Compounds (1) and (4) should be effective in treating, for example mutant Nras expressing melanoma mutant Nras expressing bladder cancer, and mutant Braf expressing melanoma. For example, a sub-therapeutic amount of Compound (4) can be an effective amount if, when combined with Compound (1), the combination is effective in the treatment of mutant Nras expressing melanoma mutant Nras expressing bladder cancer, or mutant Braf expressing melanoma.

The combination of compounds provided herein exhibits a synergistic effect (i.e., greater than additive effect) in the treatment of the cancer. The term "synergistic effect" as used herein, refers to action of two agents such as, for example, Compound (1) and Compound (4), producing an effect, for example, slowing the symptomatic progression of cancer or symptoms thereof, which is greater than the simple addition of the effects of each drug administered by themselves. A synergistic effect can be calculated, for example, using suitable methods such as the Sigmoid-Emax equation (Holford, N. H. G. and Scheiner, L. B., Clin. Pharmacokinet. 6: 429-453 (1981)), the equation of Loewe additivity (Loewe, S. and Muischnek, H., Arch. Exp. Pathol Pharmacol. 114: 313-326 (1926)) and the median-effect equation (Chou, T. C. and Talalay, P., Adv. Enzyme Regul. 22: 27-55 (1984)). Each equation referred to above can be applied to experimental data to generate a corresponding graph to aid in assessing the effects of the drug combination. The corresponding graphs associated with the equations referred to above are the concentration-effect curve, isobologram curve and combination index curve, respectively.

In different embodiments, depending on the combination and the effective amounts used, the combination of compounds can inhibit cancer growth. In some embodiments, the combination achieves cancer stasis. In some embodiments, the combination achieves substantial or complete cancer regression.

As used herein, the term "about" generally indicates a possible variation of no more than 10%, 5%, or 1% of a value. For example, "about 25 mg/kg" will generally indicate, in its broadest sense, a value of 22.5-27.5 mg/kg, i.e., 25 $\pm$ 10 mg/kg.

While the amounts of Compounds (1) and (4) should result in the effective treatment of a cancer, the amounts, when combined, are preferably not excessively toxic to the patient (i.e., the amounts are preferably within toxicity limits as established by medical guidelines). In some embodiments, either to prevent excessive toxicity and/or provide a more efficacious treatment of the cancer, a limitation on the total administered dosage is provided. Typically, the amounts considered herein are per day; however, half-day and two-day or three-day cycles also are considered herein.

Different dosage regimens may be used to treat the cancer. In some embodiments, a daily dosage, such as any of the exemplary dosages described above, is administered once, twice, three times, or four times a day for three, four, five, six, seven, eight, nine, or ten days. Depending on the stage and severity of the cancer, a shorter treatment time (e.g., up to five days) may be employed along with a high dosage, or a longer treatment time (e.g., ten or more days, or weeks, or a month, or longer) may be employed along with a low dosage. In some embodiments, a once- or twice-daily dosage is administered every other day. In some embodiments, each dosage contains both Compound (1) and Compound (4) to be delivered as a single dosage, while in other embodiments, each dosage contains either Compound (1) or Compound (4) to be delivered as separate dosages.

Examples of types of cancers to be treated with the present invention include, for example, mutant Nras expressing melanoma, mutant Nras expressing bladder cancer, and mutant Braf expressing melanoma.

The subject considered herein is typically a human, for example a human patient. However, the subject can be any mammal for which cancer treatment is desired. Thus, the methods described herein can be applied to both human and veterinary applications.

The term "treating" or "treatment", as used herein, indicates that the method has, at the least, mitigated abnormal cellular proliferation. For example, the method can reduce the rate of cancer growth in a patient, or prevent the continued growth of a cancer, or even reduce the overall reach of the cancer

Compounds (1) and (4), or their pharmaceutically acceptable salts or solvate forms, in pure form or in an appropriate pharmaceutical composition, can be administered via any of the accepted modes of administration or agents known in the art. The compounds can be administered, for example, orally, nasally, parenterally (intravenous, intramuscular, or subcutaneous), topically, transdermally, intravaginally, intravesically, intracistemally, or rectally. The dosage

form can be, for example, a solid, semi-solid, lyophilized powder, or liquid dosage forms, such as for example, tablets, pills, soft elastic or hard gelatin capsules, powders, solutions, suspensions, suppositories, aerosols, or the like, preferably in unit dosage forms suitable for simple administration of precise dosages. A particular route of administration is oral, particularly one in which a convenient daily dosage regimen can be adjusted according to the degree of severity of the disease to be treated.

In one aspect, a composition is provided that comprises Compound (1) and Compound (4). In some embodiments, the composition is in the form of a solid (e.g., a powder or tablet) including Compound (1) and Compound (4) in solid form, and optionally, one or more auxiliary (e.g., adjuvant) or pharmaceutically active compounds in solid form. In other embodiments, the composition further includes any one or combination of pharmaceutically acceptable carriers (i.e., vehicles or excipients) known in the art, thereby providing a liquid dosage form.

As discussed above, Compound (1) and Compound (4) of the combination therapy can be administered in a single unit does or separate dosage forms. Accordingly, the pharmaceutically acceptable carriers and excipients described throughout the application can be combined with Compounds (1) and (4) in a single unit dose, as well as individually combined with Compounds (1) and (4) when these compounds are administered separately.

Auxiliary and adjuvant agents may include, for example, preserving, wetting, suspending, sweetening, flavoring, perfuming, emulsifying, and dispensing agents. Prevention of the action of microorganisms is generally provided by various antibacterial and antifungal agents, such as, parabens, chlorobutanol, phenol, sorbic acid, and the like. Isotonic agents, such as sugars, sodium chloride, and the like, may also be included. Prolonged absorption of an injectable pharmaceutical form can be brought about by the use of agents delaying absorption, for example, aluminum monostearate and gelatin. The auxiliary agents also can include wetting agents, emulsifying agents, pH buffering agents, and antioxidants, such as, for example, citric acid, sorbitan monolaurate, triethanolamine oleate, butylated hydroxytoluene, and the like.

Dosage forms suitable for parenteral injection may comprise physiologically acceptable sterile aqueous or nonaqueous solutions, dispersions, suspensions or emulsions, and sterile powders for reconstitution into sterile injectable solutions or dispersions. Examples of suitable aqueous and nonaqueous carriers, diluents, solvents or vehicles include water, ethanol, polyols (propyleneglycol, polyethyleneglycol, glycerol, and the like), suitable mixtures thereof, vegetable oils (such as olive oil) and injectable organic esters such as ethyl oleate. Proper fluidity can be maintained, for example, by the use of a coating such as lecithin, by the maintenance of the required particle size in the case of dispersions and by the use of surfactants.

Solid dosage forms for oral administration include capsules, tablets, pills, powders, and granules. In such solid dosage forms, the active compound is admixed with at least one inert customary excipient (or carrier) such as sodium citrate or dicalcium phosphate or (a) fillers or extenders, as for example, starches, lactose, sucrose, glucose, mannitol, and silicic acid, (b) binders, as for example, cellulose derivatives, starch, alignates, gelatin, polyvinylpyrrolidone, sucrose, and gum acacia, (c) humectants, as for example, glycerol, (d) disintegrating agents, as for example, agar-agar, calcium carbonate, potato or tapioca starch, alginic acid, croscarmellose sodium, complex silicates, and sodium carbonate, (e) solution retarders, as for example paraffin, (f) absorption accelerators, as for example, quaternary ammonium compounds, (g) wetting agents, as for example, cetyl alcohol, and glycerol monostearate, magnesium stearate and the like (h) adsorbents, as for example, kaolin and bentonite, and (i) lubricants, as for example, talc, calcium stearate, magnesium stearate, solid polyethylene glycols, sodium lauryl sulfate, or mixtures thereof. In the case of capsules, tablets, and pills, the dosage forms also may comprise buffering agents.

Solid dosage forms as described above can be prepared with coatings and shells, such as enteric coatings and others well-known in the art. They can contain pacifying agents and can be of such composition that they release the active compound or compounds in a certain part of the intestinal tract in a delayed manner. Examples of embedded compositions that can be used are polymeric substances and waxes. The active compounds also can be in microencapsulated form, if appropriate, with one or more of the above-mentioned excipients.

Liquid dosage forms for oral administration include pharmaceutically acceptable emulsions, solutions, suspensions, syrups, and elixirs. Such dosage forms are prepared, for example, by dissolving, dispersing, etc., the HDM2 antagonist or the MEK inhibitor compound described herein, or a pharmaceutically acceptable salt thereof, and optional pharmaceutical adjuvants in a carrier, such as, for example, water, saline, aqueous dextrose, glycerol, ethanol and the like; solubilizing agents and emulsifiers, as for example, ethyl alcohol, isopropyl alcohol, ethyl carbonate, ethyl acetate, benzyl alcohol, benzyl benzoate, propyleneglycol, 1,3- butyleneglycol, dimethyl formamide; oils, in particular, cottonseed oil, groundnut oil, corn germ oil, olive oil, castor oil and sesame oil, glycerol, tetrahydrofurfuryl alcohol, polyethyleneglycols and fatty acid esters of sorbitan; or mixtures of these substances, and the like, to thereby form a solution or suspension.

Suspensions, in addition to the active compounds, may contain suspending agents, as for example, ethoxylated isostearyl alcohols, polyoxyethylene sorbitol and sorbitan esters, microcrystalline cellulose, aluminum metahydroxide, bentonite, agar-agar and tragacanth, or mixtures of these substances, and the like.

Compositions for rectal administrations are, for example, suppositories that can be prepared by mixing the compounds described herein with, for example, suitable non- irritating excipients or carriers such as cocoa butter,

polyethyleneglycol or a suppository wax, which are solid at ordinary temperatures but liquid at body temperature and therefore, melt while in a suitable body cavity and release the active component therein.

Dosage forms for topical administration may include, for example, ointments, powders, sprays, and inhalants. The active component is admixed under sterile conditions with a physiologically acceptable carrier and any preservatives, buffers, or propellants as can be required. Ophthalmic formulations, eye ointments, powders, and solutions also can be employed.

Generally, depending on the intended mode of administration, the pharmaceutically acceptable compositions will contain about 1% to about 99% by weight of the compounds described herein, or a pharmaceutically acceptable salt thereof, and 99% to 1% by weight of a pharmaceutically acceptable excipient. In one example, the composition will be between about 5% and about 75% by weight of a compounds described herein, or a pharmaceutically acceptable salt thereof, with the rest being suitable pharmaceutical excipients.

Actual methods of preparing such dosage forms are known, or will be apparent, to those skilled in this art. Reference is made, for example, to Remington's Pharmaceutical Sciences, 18th Ed., (Mack Publishing Company, Easton, Pa., 1990).

In some embodiments, the composition includes one or more other anti-cancer compounds. For example, administered compositions can comprise standard of care agents for the type of cancers selected for treatment.

In another aspect, kits are provided. Such kits can comprise package(s) comprising compounds or compositions as described herein. In one embodiment, kits comprise Compound (1), or a pharmaceutically acceptable salt thereof, and Compound (4) or a pharmaceutically acceptable salt thereof.

The phrase "package" means any vessel containing compounds or compositions presented herein. In some embodiments, the package can be a box or wrapping. Packaging materials for use in packaging pharmaceutical products are well-known to those of skill in the art. Examples of pharmaceutical packaging materials include, but are not limited to, bottles, tubes, inhalers, pumps, bags, vials, containers, syringes, bottles, and any packaging material suitable for a selected formulation and intended mode of administration and treatment.

The kit also can contain items that are not contained within the package but are attached to the outside of the package, for example, pipettes.

Kits can contain instructions for administering compounds or compositions of the invention to a patient. Kits also can comprise instructions for approved uses of compounds herein by regulatory agencies, such as the United States Food and Drug Administration. Kits also can contain labeling or product inserts for the inventive compounds. The package(s) and/or any product insert(s) may themselves be approved by regulatory agencies. The kits can include compounds in the solid phase or in a liquid phase (such as buffers provided) in a package. The kits also can include buffers for preparing solutions for conducting the methods, and pipettes for transferring liquids from one container to another.

Examples have been set forth below for the purpose of illustration and to describe certain specific embodiments of the invention. However, the scope of the claims is not to be in any way limited by the examples set forth herein.

EXAMPLE 1

HDM2-p53 Inhibitors

Three HDM2-p53 inhibitors, Compounds

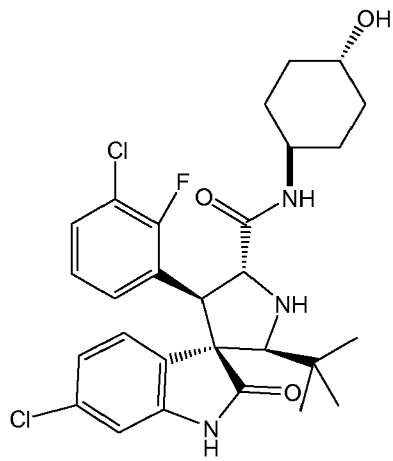

(1),

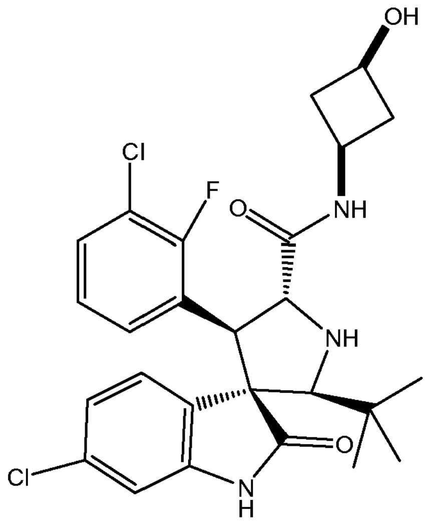
(2), and
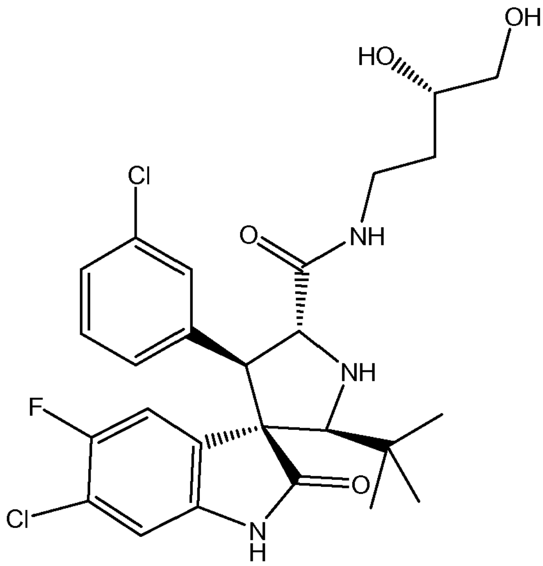
(3),

were examined in combination with MEK inhibitors

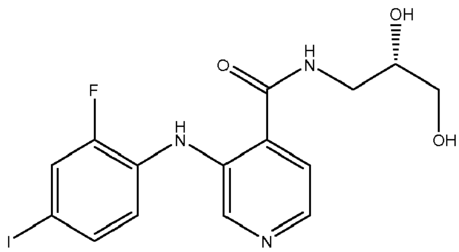
(4),
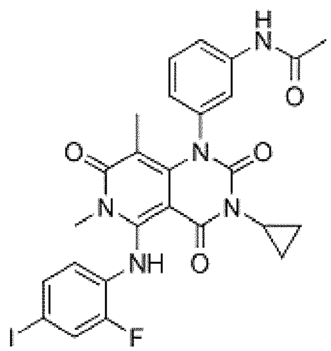
(5),

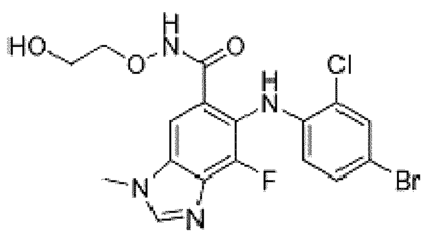
(6),
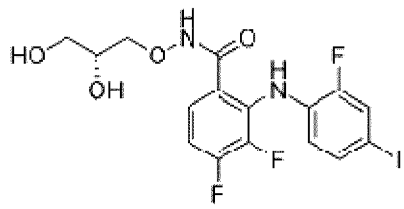
(7),

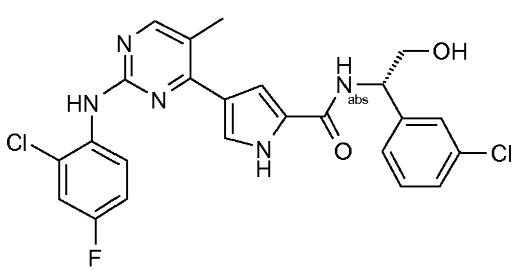
(8),

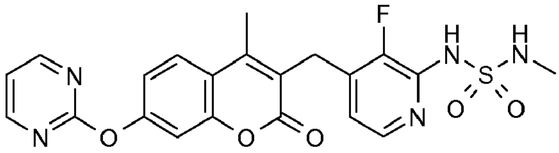

(9),

and

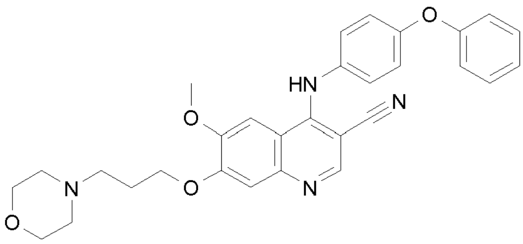

(10).

The preparation and properties of Compound (5), also referred to as Trametinib, GSK1120212, GSK212, and JTP-74057), are provided in, for example, in published PCT application WO2005/121142, the contents of which are incorporated herein by reference.

The preparation and properties of Compound (6), also referred to as AZD6244, Selumetinib, and ARRY-142886) are provided in, for example, in published PCT application WO03/077914, particularly Example 10 therein, the contents of which are incorporated herein by reference.

The preparation and properties of Compound (7), also referred as PD0325901 (N-(2,3-dihydroxy-propoxy)-3,4-difluoro-2-(2-fluoro-4-iodo-phenylamino)-benzamide) are provided in, for example, in published PCT application WO2002006213, particularly Example 85 therein, the contents of which are incorporated herein by reference.

The preparation and properties of Compound (8), also referred as also referred to as 4-[2-(2-chloro-4-fluorophenylamino)-5-methylpyrimidin-4-yl]-N-[1(S)-(3-chlorophenyl)-2-hydroxyethyl]-1H-pyrrole-2-carboxamide are provided in, for example, in Aronov, et al, J.Med. Chem. (2009) 52:6362-6368, the contents of which are incorporated herein by reference.

The preparation and properties of Compound (9), also referred to as RO-5126766 (N-[3-fluoro-4-[4-methyl-2-oxo-7-(pyrimidin-2-yloxy)-2H-1-benzopyran-3-ylmethyl]pyridin-2-yl]-N'-methylsulfamide), are provided in, for example, in published US patent application US2010/0004233, particularly paragraphs [1780]-[1793] therein, the contents of which are incorporated herein by reference.

The preparation and properties of Compound (10), also referred to as (6-methoxy-7-[3-(4-morpholinyl)propoxy]-4-(4-phenoxyphenylamino)quinoline-3-carbonitrile), are provided in, for example, in Zhang, et al, Bioor. Med. Chem. Let. (2000) 10:2825-2828, the contents of which are incorporated herein by reference.

The binding affinity of Compounds (1), (2), and (3) for MDM2 was determined as described in published PCT application WO2012/065022, Examples 2 and 3, and shown herein in Table 1.

Table 1

| COMPOUND | $IC_{50}$ (MDM2) |
|---|---|
| (1) | 8.3 ± 3.0 nM |
| (2) | 5.6 ± 1.7 nM |
| (3) | 104 nM |

The combinations of HDM2-p53 inhibitor and MEK inhibitor were examined in a panel of twenty cell lines (Table 2). The panel of cell lines included cell lines that were induced to undergo apoptosis upon exposure to Compound (1) (apoptosis), as well as cell lines where exposure to Compound (1) resulted in inhibition of cell proliferation but not apoptosis (stasis), and cell lines that did not undergo apoptosis or experience stasis upon exposure to Compound (1) (resistant).

A 36-point combination dose response matrix over a treatment time of seventy-two hours was used. The method by which this screen was performed is described below.

Table 2

| Cell Line | Tissue | Tumor Type | Effect of Cpd (1) on Cells in vitro | Ras pathway status |
|---|---|---|---|---|
| BPH1 | Prostate | Hyperplasia | resistant | |
| MG-63 | Bone | Osteosarcoma | resistant | |
| SK-N-AS | Adrenal | Neuroblastoma | resistant | |
| 769-P | Kidney | Renal Cell Adenocarcinoma | stasis | |
| C32 | Skin | Melanoma | stasis | Braf mut |
| Caki-1 | Kidney | Carcinoma | stasis | |
| H4 | Brain | Neuroglioma | stasis | |
| HCT-116 | Colorectal | Carcinoma, Colorectal | stasis | Kras mut |
| HT-1197 | Bladder | Carcinoma | stasis | Nras mut |
| SR | | Analplastic large Lymphomacell lymphoma | stasis | |
| SW48 | Colorectal | Adenocarcinoma | stasis | EGFR mut |
| MCF7 | Breast | Adenocarcinoma | stasis | |
| LNCaP | Prostate | AdenoCarcinoma | stasis | |
| RKO | Colorectal | Carcinoma, Colorectal | apoptosis | Braf mut |
| A375 | Melanoma | Melanoma | apoptosis | Brafmut |
| DBTRG-05MG | Brain | Glioblastoma | apoptosis | Braf mut |
| DK-MG[1] | Brain | Glioblastoma | apoptosis | |
| JAR[1] | Placenta | Choriocarcinoma | apoptosis | |
| MV-4-11 | Leukemia | Acute Myelogenous Leukemia | apoptosis | Flt3ITD |
| SJSA-1[1] | Bone | Sarcoma | apoptosis | |

1. HDM2 amplified

**Cell Culture**

Cells were thawed from a liquid nitrogen preserved state. Once cells had been expanded and divided at their expected doubling times, cells were seeded in complete media (Table 3) at 100 cells per well in black 1536 tissue culture treated plates. Assay plates were centrifuged for 2 min at 335 x g, incubated at room temperature for 20 min, and incubated at 37° C for 24 hours before treatment.

Table 3

| Cell Line | Growth Conditions |
|---|---|
| 769-P | RPMI WITH 10% FBS |
| A375 | DMEM, 10% FBS |
| BPH1 | RPMI, 20% FBS, |
| C32 | Eagles MEM 10% FBS |
| Caki-1 | McCoy's 5A, 10% FBS |
| DBTRG-05MG | RPMI, 10% FBS |
| DK-MG[1] | RPMI, 10% FBS |
| H4 | DMEM, 10% FBS |
| HCT-116 | McCoy's 5A, 10% FBS |
| HT-1197 | Eagles MEM 10% FBS |
| JAR[1] | RPMI, 10% FBS |

(continued)

| Cell Line | Growth Conditions |
|---|---|
| LNCaP | DMEM, 10% FBS |
| MCF7 | Eagles MEM 10% FBS, 0.01 mg/ml bovine insulin |
| MG-63 | Eagles MEM 10% FBS |
| MV-4-11 | Iscoves, 10% FBS |
| RKO | Eagles MEM 10% FBS |
| SJSA-1[1] | RPMI, 10% FBS |
| SK-N-AS | DMEM, 10% FBS, 0.1 mM NEAA |
| SR | RPMI, 10% FBS |
| SW48 | RPMI, 10% FBS, 5% $CO_2$ |

ATP levels were used as a measure of cell number (cell growth). At the time of treatment, ATP levels were measured in an untreated set of assay plates (Tzero plates). ATP levels were measured by adding ATPLite (Perkin Elmer) according to manufacturer's instructions. The Tzero plates were read using ultra-sensitive luminescence on Envision Plate Readers. Assay plates receiving treatment were incubated with compound for 72 h at 37° C with 5% $CO_2$. After 72 h, plates were developed for endpoint analysis using ATPLite.

Assay plates were accepted for analysis if they passed the following quality control standards: relative luciferase values were consistent throughout the entire experiment, Z' factor scores were greater than 0.6, untreated/vehicle controls behaved consistently on the plate. The calculation for synergy score is provided below.

**Calculation of Growth Inhibition**

Growth Inhibition (GI) was used as a measure of cell viability. As described above, ATP levels were measured at the time of dosing (T0) and after 72 hours (T72). The effect of vehicle alone on cell viability was determined by measuring ATP levels at T0 and 72 hours after treatment of cells with vehicle alone (T72).

GI was calculated by applying the following test and equation:

$$\text{If } T < V_0 : 100^* \,(1\text{- } T\text{- } V_0 \,/\, V_0)$$

$$\text{If } T \geq V_0 : 100 \,^*\, (1\text{- } T\text{- } V_0 \,/\, V\text{- } V_0)$$

Where T is the signal measure for a test article, V is the vehicle-treated control measure, and $V_0$ is the vehicle control measure at time zero. This formula is derived from the Growth Inhibition calculation used in the National Cancer Institute's NCI-60 high throughput screen, as described on the world wide web at dtp.nci.nih.gov/branches/btb/ivclsp.html. A GI reading of 0% represents no growth inhibition-that is, cells treated with compound had similar levels of ATP as cells treated with vehicle and measured at 72 hours. A GI of 100% represents complete growth inhibition-that is, cells treated with compound had similar levels of ATP as cells treated with vehicle and measured at T0. A GI of 100% where cell numbers have not increased during the treatment period is indicative of a cytostatic effect for compounds reaching a plateau at this effect level. A GI of 200% represents complete death of all cells in the culture well which is indicative of an apoptotic effect.

For each cell line, the dose-response curve was measured with each HDM2-p53 antagonist and each MEK inhibitor as single agents and in pair wise combinations. Using the potency values from the single agent dose-response curves, the compounds were assigned to different groups of similar potency. Multiple doses above and below the $EC_{50}$ of a given HDM2-p53 antagonist or MEK inhibitor were measured.

**Data Normalization**

In each well of a plate, ATP levels were measured by fluorescence assay as described above. ATP levels are proportional to the number of remaining live cells. This raw data was then normalized in two steps. First, background subtraction was performed by subtracting a mean intensity of negative control wells in which cells were dosed with

dimethyl sulfoxide (DMSO). Second, a dynamic range normalization was performed by dividing the background-subtracted data by the mean intensity of positive control wells in which cells were dosed with Staurosporine. The resulting normalized intensity, now with plate-specific effects removed, was used to calculate the Growth Inhibition measure described above.

A dose response matrix was constructed where one axis corresponded to one of the three HDM2-p53 antagonists, and the other axis corresponded to one of the MEK inhibitors. The high doses were approximately the given compound's $EC_{90}$ and were diluted by a constant factor to generate the lower dosage levels. The lowest dose was 0, i.e., the edges of the matrix correspond to another measure of single-agent behavior of each of the compounds. In addition, compounds were combined with themselves in a self-cross analysis for each cell line as described below.

## Synergy Scores

Synergy in general means that two or more compounds, in a given biological context, have a combined effect that is significantly greater than would be expected from the combinations of their single-agent effects. Synergy can be measured in a variety of ways; the methods for measuring synergy involve stating a null hypothesis about the expected single-agent effects, and computing a summary score of the differences between observations and the null hypothesis. A variety of synergy scores in these three broad families were considered.

Loewe Synergy, where the null hypothesis is that the two compounds being evaluated are actually the same compound (a self-cross). The effect of combining the two compounds is a function of the sum of concentrations of the two compounds. Significant synergy means the compounds have different effects which work together to create an effect.

Bliss Synergy, where the null hypothesis is that the two compounds affect different targets, independently producing the result phenotype so that their effects add like the probability of independent events. Significant synergy means that the compounds somehow work together to produce a net effect, rather than acting independently.

Gaddum Synergy, also known as Highest Single Agent Synergy, where the null hypothesis is that there is competitive binding between the two compounds for the same target, and only the compound with the highest binder affinity causes an effect. Significant synergy means that something other than competitive binding is occurring, in which the compounds produce their combined effect in independent ways.

Chalice software (Zalicus) was used to perform the bulk of the synergy calculations.

## Positive Calling Criteria

Criteria were imposed to define how much synergy was significant. This was done in four steps:

1: The HDM2-p53 antagonists were self-crossed (each HDM2-p53 antagonist was tested in combination with itself). Synergy scores for each compound measured with itself were determined in each cell line. The resulting synergy scores were typical of false positives (a given compound cannot exhibit synergy with itself). A parametric distribution was fitted to each such population of self-cross synergy scores. For a nonlinear version of Loewe synergy, a gamma distribution was fitted. For most other synergy scores, a normal distribution was fitted, based on the appearance of the histogram of synergy scores and a Shapirio-Wilk test of normality.

For a false positive rate alpha, a true cross of two distinct compounds were required to have generated a synergy score in the alpha-th tail of the self-cross distribution. Alpha levels of 0.25% to 1% (corresponding to an equivalent normal distribution tail of 2 sigma or 3 sigma), were chosen, depending on the permissiveness of the synergy score. In this way, pairs of compounds in each cell line whose synergy was statistically significantly different from the false positives measured by self-crosses were identified. The p-value measured how far out in the tail of the false positive distribution the potential synergy was.

2: All of the compound pairs were further analyzed using a multiple hypothesis test correction for the group. A Benjamini-Höchberg correction was used, which changed the p-values of step (1) into False Discovery Rates (FDR). A cutoff of 5% or less was imposed on the FDR to obtain an initial set of MEK inhibitors showing synergistic activity in combination with one or more of the HDM2-p53 antagonists.

3: The initial set of MEK inhibitors showing synergistic activity in combination with one or more of the HDM2-p53 antagonists were further filtered by selecting MEK inhibitors that showed synergy with both compound (1) and (2). Compound (3) was not included in the analysis. In order to be declared a positive, it was required that both HDM2-p53 antagonist compounds (1) and (2) had an FDR < 5% with the same MEK inhibitor in the same cell line. Positives were reported by the maximum of the FDR for Compound(1) and Compound (2) (the most stringent call) and the mean of the synergy scores for compounds (1) and (2) (the consensus view).

4: Venn diagram of compound pairs and cell lines that were called as positives was examined for each of the synergy scores used.

Thus, a final set of compound pair/cell line positives was chosen based on the nonlinear Loewe synergy, demanding it be in the 5% tail of the self-cross distribution, have a FDR of < 5%, and that Compound (1) and Compound (2) agree on the positive.

**Enrichment Analysis of Positive Annotation**

FIG. 1 shows a table of the mean synergy score for Compounds (1) and (2) in combination with the indicated MEK inhibitor in the indicated cell lines. The grey color shows the maximum FDR with Compound (1) and Compound (2) in the indicated cell line. As shown in FIG. 1, MEK inhibitors (6), (7), (8), (9), and (10) showed statistically significant Loewe synergy with both Compound (1) and Compound (2) at an FDR < 5% in some cell lines. The cell lines in which MEK inhibitors synergized with Compounds (1) and (2) appeared to have mutations that activate the RAS pathway signaling such as mutations or Nras, Braf, FLT3-ITD, or EGFR.

FIG. 2 shows effect of the combination of Compound (1) and Compound (6) on C32 cells. FIG. 2A shows the mean synergy scores of the combination of Compounds (1) and (6) at the indicated concentrations. FIG. 2B shows percent growth inhibition as a function of concentration of Compound (1), where Compound (6) is not present or is present at 1.1 uM. As shown in FIG. 2, Compound (1) and Compound (6) induce cytostasis of C32 cells when used as single agents. However, the combination of Compound (1) and (6) induced significant cell death.

FIG. 3 shows effect of the combination of Compound (1) and Compound (6) on MV4-11 cells. FIG. 2A shows the mean synergy scores of the combination of Compounds (1) and (6) at the indicated concentrations. FIG. 2B shows percent growth inhibition as a function of concentration of Compound (1), where Compound (6) is not present or is present at 3.3 uM. As shown in FIG. 3, Compound (1) induces cytotoxicity in MV4-11 cells and Compound (6) induces cytostasis of MV4-11 cells when used as single agents. However, the combination of Compound (1) and (6) resulted in a cytotoxicity at lower concentrations of Compound (1).

FIG. 4 shows effect of the combination of Compound (1) and Compound (6) on A375 cells. FIG. 4A shows the mean synergy scores of the combination of Compounds (1) and (6) at the indicated concentrations. FIG. 4B shows an isobologram of growth inhibition of A375 cells treated with the indicated amounts of Compound (1) in combination with Compound (6), compared to additive growth inhibition. As shown in FIG. 4. the combination of Compounds (1) and (6) displayed synergy in inhibition of A375 cell proliferation. The Loewe Synergy Score for the treatment of A375 cells with the combination of Compounds (1) and (5) is 2.7.

FIG. 5 shows effect of the combination of Compound (1) and Compound (5) on Vemurafenib resistant A375 cells. FIG. 5A shows the mean synergy scores of the combination of Compounds (1) and (5) at the indicated concentrations. FIG. 5B shows an isobologram of growth inhibition of Vemurafenib resistant A375 cells treated with the indicated amounts of Compound (1) in combination with Compound (5), compared to additive growth inhibition. As shown in FIG. 5. the combination of Compounds (1) and (5) displayed synergy in inhibition of Vemurafenib resistant A375 cell proliferation. The Loewe Synergy Score for the treatment of Vemurafenib resistant A375 cells with the combination of Compounds (1) and (5) is 2.7.

**Example 2:** Ray Design Analysis

**Experimental conditions for ATP viability assays:**

Cells were seeded in 384 well microplates in 47.5 μL of culture medium as described in Table 4 and incubated for 4 hours at 37° C with 5 % CO2. After incubation, 2.5 μL of a 20x concentrated solution of individual test compounds (for single agent tests) or mixtures of test compounds (for combination tests) were added (see "Cell Treatment" section). The microplates are returned to the incubator for 96 h. After incubation, 30 μL of a mix of lysis reagent and luciferase/luciferin was added (CellTiterGlo, Promega) in each well, the microplates were incubated for 1 h at room temperature and the luminescence emitted was measured with a Microbeta Trilux counter (PerkinElmer).

**Table 4**

| Cell line name | Culture medium | Cell seeding density |
|---|---|---|
| A-375 | DMEM, 10% FBS | 500 |
| C32 | MEM alpha, 10% FBS | 6000 |
| C8161 | DMEM, 10% FBS | 600 |
| DV-90 | RPMI 1640, 10% FBS | 1000 |
| HCT116 | DMEM, 10% FBS | 800 |
| HT-1197 | MEM alpha, 10% FBS | 1000 |
| KU-19-19 | RPMI 1640, 10% FBS | 600 |

(continued)

| Cell line name | Culture medium | Cell seeding density |
|---|---|---|
| NCI-H460 | DMEM, 10% FBS | 300 |
| SKMEL-103 | RPMI 1640, 10% FBS | 1500 |
| SK-MEL-147 | RPMI 1640, 10% FBS | 1500 |
| UACC-62 | RPMI 1640, 10% FBS | 1000 |
| UM-UC-3 | MEM alpha, 10% FBS | 600 |

**Cell Treatment:**

A combination dose response matrix was generated with Compound (1) and Compound (4) or Compound (5) in a 384 well plate. Compound (1) was diluted to generate 14 concentrations and Compound (4) (or Compound (5)) was diluted to generate 18 concentrations. All combinations of concentrations were made in a 14x18 matrix (252 wells). Two identical plates were prepared, allowing duplicate determination of the effect of each combination.

In addition, the activity of each of Compound (1), Compound (4) or Compound (5) as a single agent was determined in quadruplicate at the same concentrations, respectively, used in the combination matrix. Calculation of percent inhibition I%: Percent inhibition was calculated for each well according to the formula

$$I\%=100*(T-X)/(T-B)$$

Where X = the measured value of the well,

T = the average value of the control wells containing cells treated with vehicle (DMSO 0.1 %) and B = the average value of blank wells without cells.

The 1% values were then used in nonlinear regression analysis to calculate the IC50 of the rays as described in the "Statistical Methodology" section.

**Experimental design**

ray design was used to validate findings of synergy. Compound (1) and Compound (4) or Compound (5) were evaluated at several fixed proportions of the compounds in combination. (R. Straetemans, Biometrical journal 47 -2005). The ray design included one ray for each single agent and 19 combination rays. The ray with Compound (1) alone had 14 concentrations of Compound (1), the ray with Compound (4) or Compound (5) alone had 18 concentrations of Compound (4) or Compound (5), and the combination rays had between 7 and 14 concentrations of each compound.

Between two and four experiments were performed with each cell line in order to take into account any experimental variability. For each experiment, two 384-well plates were used to obtain duplicates of combination rays and quadruplicates for single agent rays.

**Statistical methodology**

Absolute ICX is defined as the concentration of compound where 1% is equal to X%. This measurement allows determining the potential synergistic combinations using the statistical method described hereunder.

The relative potency p is first estimated as $\rho=\frac{ICX_A}{ICX_B}$ where $ICX_A$ is the ICX of the compound A (here Compound 1) and $ICX_B$ is the ICX of the compound B (here Compound 4 or Compound 5). This effective fraction for the ray i is then calculated as $f_i=\frac{1}{c_i \cdot \rho+1}$ where $c_i=\frac{[B]}{[A]}$ is the constant ratio of the concentrations of the compounds A and B in the mixture.

Among the combination rays, all the rays for which the effective fraction is in the range 0.05-0.95 are analyzed.

A global non linear model using the NLMIXED procedure of the software SAS V9.2 was applied to fit simultaneously the concentration-responses curves for each ray. The model used is a 4-parameter logistic model corresponding to the following equation:

$$Y_{ikj} = E\min_i + \frac{(E\max_i - E\min_i)}{1 + \exp\left[-m_i \log(\frac{Conc_{ij}}{IC50_i})\right]} + \varepsilon_{ijk}$$

Where $Y_{ijk}$ is the percentage of inhibition for the $k^{th}$ replicate of the $j^{th}$ concentration in the $i^{th}$ ray
$Conc_{ij}$ is the $j^{th}$ mixture concentration (sum of the concentrations of compound A and compound B) in the $i^{th}$ ray
$Emin_i$ is the minimum effect obtained from $i^{th}$ ray
$Emax_i$ is the maximum effect obtained from $i^{th}$ ray
$IC50_i$ is the IC50 obtained from $i^{th}$ ray
$m_i$ is the slope of the curve adjusted with data from $i^{th}$ ray
$\varepsilon_{ijk}$ is the residual for the $k^{th}$ replicate of the $j^{th}$ concentration in the $i^{th}$ ray, $\varepsilon_{ijk} \sim N(0, \sigma^2)$

Emin, Emax and/or slope were shared whenever it was possible without degrading the quality of the fit. The combination index Ki of each ray and its 95% confidence interval were then estimated using the following equation based on the Loewe additivity model:

$$\frac{C_A}{ICX_A} + \frac{C_B}{ICX_B} = K_i$$

where $ICX_A$ and $ICX_B$ are the concentrations of compound A and compound B necessary to obtain X% of inhibition for each compound alone and $C_A$ and $C_B$ are the concentrations of compound A and compound B in the mixture necessary to obtain X% of inhibition. For the combinations studied here, IC50 (or IC40 for compounds with a low activity) have been used to calculate Ki.

Additivity for a given Ray was defined as a 95% confidence interval of the Ki that included 1. Significant synergy for a given Ray was defined as a 95% confidence interval of the Ki having an upper bound of less than 1. Significant antagonism was defined as a 95% confidence interval of the Ki having a lower bound of higher than 1.

The isobolograms shown in FIGs. 6-17 allow visualization of the position of each Ray. The line shown in FIGs. 6-17 connects the point (0,1) to the point (1,0). All rays below the line correspond to potential synergy, whereas rays above the line correspond to potential antagonism.

In the isobolograms shown in FIGs 6-9, and 11-17, the x-axis is [Compound (1)]/IC50[Compound (1)] and the y-axis is [Compound (4)]/IC50[Compound(4)] (FIG. A) or [Compound (5)]/IC50[Compound(5)] (FIG. B). In the isobolograms shown in FIG. 10 the x-axis is [Compound (1)]/IC40[Compound (1)] and the y-axis is [Compound (4)]/IC40[Compound(4)] (FIG. 10A) or [Compound (5)]/IC40[Compound(5)] (FIG. 10B).

Synergy was confirmed where the upper bound of the confidence interval of the combination index (Ki) was lower than 1 for the majority of rays. Trend to synergy was confirmed where the combination index (Ki) was lower than 1 (but the confidence interval of the combination index (Ki) included 1) for the majority of rays. Additivity was confirmed where the confidence interval of the combination index (Ki) included 1 for the majority of rays. Trend to antagonism was found where the combination index (Ki) was higher than 1 (but the confidence interval of the combination index (Ki) included 1) for the majority of rays. Antagonism was found where the lower bound of the confidence interval of the combination index (Ki) was higher than 1 for the majority of rays.

**Compound (1) shows a trend toward antagonism with Compound (4) and additivity with Compound (5) in an Nras mutant melanoma cell line (C8161).**

Table 5 shows the IC50 estimations for each compound and the relative potency of Compounds (1) and (4) on C8161 cells when used as single agents.

**Table 5**

| Experiment | Compound 1 | Compound 4 | Relative potency |
|---|---|---|---|
| N1 **(IC50) N1 (IC50)** | 7.74E+01 [6.16E+01 ; 9.72E+01] | 1.89E+02 [1.38E+02 ; 2.60E+02] | 0.41 |
| N2 **(IC50) N2 (IC50)** | 1.63E+02 [9.31E+01;2.84E+02] | 2.87E+02 [2.24E+02 ; 3.68E+02] | 0.57 |

Table 6 shows the interaction characterization of Compounds (1) and (4) in C8161 cells.

**Table 6**

| Experiment | Ray | f values | Ki (confidence interval at 95%) | Interaction characterization |
|---|---|---|---|---|
| N2 | Ray 5 | 0.05 | 1.2284 [0.8518 ; 1.7716] | Additivity |
| N1 | Ray 5 | 0.07 | 1.3302 [0.7936 ; 2.2295] | Additivity |
| N2 | Ray 6 | 0.15 | 1.4596 [0.9350 ; 2.2784] | Additivity |
| N1 | Ray 6 | 0.20 | 1.4464 [0.8138 ; 2.5707] | Additivity |
| N2 | Ray 7 | 0.35 | 1.4928 [1.0483 ; 2.1259] | Antagonism |
| N1 | Ray 7 | 0.42 | 1.3625 [0.9226 ; 2.0121] | Additivity |
| N2 | Ray 8 | 0.64 | 1.1827 [0.6624 ; 2.1119] | Additivity |
| N1 | Ray 8 | 0.71 | 1.2192 [0.8524 ; 1.7439] | Additivity |
| N2 | Ray 9 | 0.85 | 1.2099 [0.3842 ; 3.8105] | Additivity |
| N1 | Ray 9 | 0.89 | 1.2529 [0.8711 ; 1.8019] | Additivity |
| N2 | Ray 10 | 0.95 | 0.7767 [0.4027 ; 1.4984] | Additivity |
| N1 | Ray 10 | 0.96 | 1.2059 [0.8505 ; 1.7098] | Additivity |

Table 7 shows the IC50 estimations for each compound and the relative potency of Compounds (1) and (5) on C8161 cells when used as single agents.

**Table 7**

| Experiment | Compound 1 | Compound 5 | Relative potency |
|---|---|---|---|
| **N1 (IC50)** | 1.42E+02 [1.15E+02 ; 1.75E+02] | 2.80E+01 [2.13E+01 ; 3.68E+01] | 5.07 |
| **N2 (IC50)** | 9.19E+01 [6.52E+01 ; 1.29E+02] | 3.00E+01 [1.83E+01 ; 4.92E+01] | 3.06 |

Table 8 shows the interaction characterization of Compounds (1) and (4) in C8161 cells.

**Table 8**

| Experiment | Ray | f values | Ki (confidence interval at 95%) | Interaction characterization |
|---|---|---|---|---|
| N1 | Ray 7 | 0.06 | 0.7853 [0.4884 ; 1.2627] | Additivity |
| N3 | Ray 6 | 0.09 | 1.0619 [0.4659 ; 2.4204] | Additivity |
| N1 | Ray 8 | 0.16 | 0.7728 [0.4591 ; 1.3008] | Additivity |
| N3 | Ray 7 | 0.25 | 1.3380 [0.6550 ; 2.7333] | Additivity |
| N1 | Ray 9 | 0.40 | 1.0063 [0.6789 ; 1.4917] | Additivity |
| N3 | Ray 8 | 0.52 | 1.0316 [0.5872 ; 1.8122] | Additivity |
| N1 | Ray 10 | 0.66 | 0.9705 [0.6897 ; 1.3655] | Additivity |
| N3 | Ray 9 | 0.77 | 1.0863 [0.6502 ; 1.8150] | Additivity |
| N1 | Ray 11 | 0.87 | 0.7584 [0.5434 ; 1.0584] | Additivity |
| N3 | Ray 10 | 0.92 | 0.7807 [0.4402 ; 1.3846] | Additivity |
| N1 | Ray 12 | 0.95 | 1.1543 [0.8403 ; 1.5857] | Additivity |
| N3 | Ray 11 | 0.97 | 0.6960 [0.3887 ; 1.2465] | Additivity |

FIG. 6 shows an isobologram of growth inhibition of C8161 cells treated with Compound (1) in combination with Compound (4) (FIG. 6A) or with Compound (5) (FIG. 6B), compared to additive growth inhibition. As shown in Tables 6 and 8, and FIG. 6, despite the Nras mutant status of C8161 melanoma cells, Compound (1) in combination with Compound (4) showed a trend toward antagonsism and Compound (1) in combination with (5) showed an additive effect on C8161 cells.

**Compound (1) shows synergy with Compounds (4) and (5) in HCT116 Cells**

Table 9 shows the IC50 estimations for each compound alone and the relative potency of Compounds (1) and (4) on HCT116 cells.

**Table 9**

| Experiment | Compound 1 | Compound 4 | Relative potency |
|---|---|---|---|
| **N1 (IC50)** | 2.96E+02 [1.41E+02 ; 3.63E+02] | 1.50E+02 [1.20E+02 ; 1.86E+02] | 1.98 |
| **N2 (IC50)** | 2.96E+02 [2.33E+02 ; 3.77E+02] | 1.59E+02 [1.21E+02 ; 2.09E+02] | 1.86 |
| **N3 (IC50)** | 5.30E+02 [3.56E+02 ; 7.89E+02] | 4.21E+01 [2.91E+01 ; 6.10E+02] | 12.58 |
| **N4 (IC50)** | 4.92E+02 [3.07E+02 ; 7.87E+02] | 6.76E+01 [4.18E+01 ; 1.10E+02] | 7.28 |

Table 10 shows the interaction characterization of Compounds (1) and (4) in HCT116 cells.

**Table 10**

| Experiment | Ray | f values | Ki (confidence interval at 95%) | Interaction characterization |
|---|---|---|---|---|
| N4 | Ray 7 | 0.04 | 1.0245 [0.4100 ; 2.5596] | Additivity |
| N1 | Ray 6 | 0.05 | 0.5864 [0.3866 ; 0.8896] | Synergy |
| N2 | Ray 6 | 0.05 | 0.7371 [0.3785 ; 1.4354] | Additivity |
| N3 | Ray 8 | 0.07 | 1.6414 [0.8931 ; 3.0168] | Additivity |
| N4 | Ray 8 | 0.12 | 0.9923 [0.4493 ; 2.1919] | Additivity |
| N1 | Ray 7 | 0.13 | 0.5293 [0.3563 ; 0.7864] | Synergy |
| N2 | Ray 7 | 0.14 | 1.7545 [1.1893 ; 2.5883] | Antagonism |
| N3 | Ray 9 | 0.21 | 1.0670 [0.6146 ; 1.8524] | Additivity |
| N4 | Ray 9 | 0.31 | 0.6700 [0.3251 ; 1.3808] | Additivity |
| N1 | Ray 8 | 0.34 | 0.5892 [0.4156 ; 0.8354] | Synergy |
| N2 | Ray 8 | 0.35 | 0.9372 [0.6154 ; 1.4272] | Additivity |
| N3 | Ray 10 | 0.44 | 0.6197 [0.3725 ; 1.0310] | Additivity |
| N4 | Ray 10 | 0.58 | 0.4061 [0.1945 ; 0.8481] | Synergy |
| N1 | Ray 9 | 0.63 | 0.6492 [0.4759 ; 0.8856] | Synergy |
| N2 | Ray 9 | 0.64 | 0.7973 [0.5390 ; 1.1795] | Additivity |
| N3 | Ray 11 | 0.73 | 0.4591 [0.2767 ; 0.7616] | Synergy |
| N4 | Ray 11 | 0.82 | 0.2574 [0.1085 ; 0.6103] | Synergy |
| N1 | Ray 10 | 0.84 | 0.7309 [0.5345 ; 0.9995] | Synergy |
| N2 | Ray 10 | 0.84 | 0.7431 [0.4950 ; 1.1155] | Additivity |
| N3 | Ray 12 | 0.89 | 0.5369 [0.321 ; 0.8958] | Synergy |
| N4 | Ray 12 | 0.93 | 0.2159 [0.0810 ; 0.5753] | Synergy |
| N1 | Ray 11 | 0.94 | 0.5160 [0.3649 ; 0.7298] | Synergy |
| N2 | Ray 11 | 0.95 | 0.5015 [0.3092 ; 0.8133] | Synergy |
| N3 | Ray 13 | 0.96 | 0.6990 [0.4083 ; 1.1968] | Additivity |

Table 11 shows the IC50 estimations for each compound alone and the relative potency of Compounds (1) and (5) on HCT116 cells.

**Table 11**

| Experiment | Compound 1 | Compound 5 | Relative potency |
|---|---|---|---|
| **N1 (IC50) N1 (IC50)** | 3.66E+02 [2.95E+02 ; 4.56E+02] | 1.37E+01 [1.08E+01 ; 1.73E+01] | 26.84 |
| **N2 (IC50)** | 2.43E+02 [2.01E+03 ; 2.95E+02] | 1.04E+01 [8.38E+00 ; 1.28E+01] | 23.49 |
| **N3 (IC50)** | 6.42E+02 [4.90E+02 ; 8.42E+02] | 1.96E+01 [1.49E+01 ; 2.59E+01] | 32.75 |

Table 12 shows the interaction characterization of Compounds (1) and (5) in HCT116 cells.

Table 12

| Experiment | Ray | f values | Ki (confidence interval at 95%) | Interaction characterization |
|---|---|---|---|---|
| N3 | Ray 8 | 0.03 | 0.4099 [0.2339 ; 0.7186] | Synergy |
| N1 | Ray 8 | 0.04 | 0.7768 [0.5208 ; 1.1586] | Additivity |
| N2 | Ray 8 | 0.04 | 0.7355 [0.5100 ; 1.0608] | Additivity |
| N3 | Ray 9 | 0.09 | 0.4684 [0.2814 ; 0.7798] | Synergy |
| N1 | Ray 9 | 0.11 | 0.8389 [0.5625 ; 1.2512] | Additivity |
| N2 | Ray 9 | 0.12 | 0.8544 [0.6081 ; 1.2005] | Additivity |
| N3 | Ray 10 | 0.23 | 0.4153 [0.2621 ; 0.6580] | Synergy |
| N1 | Ray 10 | 0.27 | 0.6508 [0.4369 ; 0.9696] | Synergy |
| N2 | Ray 10 | 0.30 | 0.6879 [0.4947 ; 0.9566] | Synergy |
| N3 | Ray 11 | 0.50 | 0.5036 [0.3375 ; 0.7514] | Synergy |
| N1 | Ray 11 | 0.55 | 0.4888 [0.3421 ; 0.6983] | Synergy |
| N2 | Ray 11 | 0.59 | 0.6420 [0.4635 ; 0.8892] | Synergy |
| N3 | Ray 12 | 0.75 | 0.4661 [0.3036 ; 0.7156] | Synergy |
| N1 | Ray 12 | 0.79 | 0.7794 [0.5613 ; 1.0821] | Additivity |
| N2 | Ray 12 | 0.81 | 0.9862 [0.7260 ; 1.3396] | Additivity |
| N3 | Ray 13 | 0.91 | 0.7491 [0.4957 ; 1.1321] | Additivity |
| N1 | Ray 13 | 0.93 | 0.8746 [0.6058 ; 1.2625] | Additivity |
| N2 | Ray 13 | 0.93 | 1.1598 [0.8454 ; 1.5912] | Additivity |

FIG. 7 shows an isobologram of growth inhibition of HCT116 cells treated with the indicated amounts of Compound (1) in combination with Compound (4) (FIG. 7A) or Compound (5) (FIG. 7B), compared to additive growth inhibition. As shown in Tables 10 and 12, and FIG. 7, Compound (1) in combination with Compound (4) or (5) showed a synergistic effect on HCT116 cells.

**Compound (1) shows synergy with Compounds (4) and (5) in an Nras mutant melanoma cell line, SK-MEL-103.**

Table 13 shows the IC50 estimations for each compound alone and the relative potency of Compounds (1) and (4) on SK-MEL-103 cells.

Table 13

| Experiment | Compound 1 | Compound 4 | Relative potency |
|---|---|---|---|
| **N1 (IC50)** | 2.60E+02 [2.11E+02 ; 3.20E+02] | 2.85E+01 [2.30E+01 ; 3.53E+01] | 9.1 |
| **N2 (IC50)** | 3.41E+02 [2.62E+02 ; 4.43E+02] | 3.16E+01 [2.38E+01 ; 4.18E+01] | 10.8 |
| **N3 (IC50)** | 2.44E+02 [2.10E+02 ; 2.83E+02] | 3.40E+01 [2.77E+01 ; 4.17E+01] | 7.2 |

Table 14 shows the interaction characterization of Compounds (1) and (4) in SK-MEL-103 cells.

Table 14

| Experiment | Ray | f values | Ki (confidence interval at 95%) | Interaction characterization |
|---|---|---|---|---|
| N1 | **Ray 7** | 0.03 | 0.5733 [0.3874 ; 0.8483] | Synergy |
| N3 | **Ray 7** | 0.04 | 0.7592 [0.5434 ; 1.0606] | Additivity |
| N2 | **Ray 8** | 0.08 | 0.9332 [0.5787 ; 1.5047] | Additivity |
| N1 | **Ray 8** | 0.10 | 0.7038 [0.4942 ; 1.0022] | Additivity |
| N3 | **Ray 8** | 0.12 | 0.8963 [0.6406 ; 1.2542] | Additivity |
| N2 | **Ray 9** | 0.24 | 0.7623 [0.4920 ; 1.1811] | Additivity |
| N1 | **Ray 9** | 0.27 | 0.6873 [0.4967 ; 0.9511] | Synergy |
| N3 | **Ray 9** | 0.32 | 0.5607 [0.4173 ; 0.7534] | Synergy |
| N2 | **Ray 10** | 0.48 | 0.6409 [0.4261 ; 0.9640] | Synergy |

(continued)

| Experiment | Ray | f values | Ki (confidence interval at 95%) | Interaction characterization |
|---|---|---|---|---|
| N1 | **Ray 10** | 0.52 | 0.6075 [0.4364 ; 0.8457] | Synergy |
| N3 | **Ray 10** | 0.58 | 0.6164 [0.4760 ; 0.7983] | Synergy |
| N2 | **Ray 11** | 0.76 | 0.5139 [0.3397 ; 0.7776] | Synergy |
| N1 | **Ray 11** | 0.79 | 0.6928 [0.5006 ; 0.9588] | Synergy |
| N3 | **Ray 11** | 0.82 | 0.6929 [0.5351 ; 0.8971] | Synergy |
| N2 | **Ray 12** | 0.90 | 0.5738 [0.3787 ; 0.8694] | Synergy |
| N1 | **Ray 12** | 0.92 | 0.7326 [0.5095 ; 1.0536] | Additivity |
| N3 | **Ray 12** | 0.93 | 0.7716 [0.6085 ; 0.9785] | Synergy |
| N2 | **Ray 13** | 0.97 | 0.6644 [0.4281 ; 1.0311] | Additivity |

Table 15 shows the IC50 estimations for each compound alone and the relative potency of Compounds (1) and (5) on SK-MEL-103 cells.

**Table 15**

| Experiment | Compound 1 | Compound 5 | Relative potency |
|---|---|---|---|
| **N1 (IC50)** | 3.60E+02 [2.82E+02 ; 4.60E+02] | 4.83E+00 [3.79E+00 ; 6.16E+00] | 74.6 |
| **N2 (IC50)** | 3.63E+02 [3.00E+02 ; 4.39E+02] | 4.58E+00 [3.79E+00 ; 5.54E+00] | 79.1 |
| **N3 (IC50)** | 3.59E+02 [2.82E+02 ; 4.58E+02] | 4.47E+00 [3.50E+00 ; 5.73E+00] | 80.3 |

Table 16 shows the interaction characterization of Compounds (1) and (5) in SK-MEL-103 cells.

**Table 16**

| Experiment | Ray | f values | Ki (confidence interval at 95%) | Interaction characterization |
|---|---|---|---|---|
| N3 | **Ray 9** | 0.04 | 0.7550 [0.4853 ; 1.1747] | Additivity |
| N2 | **Ray 9** | 0.04 | 0.6809 [0.4798 ; 0.9664] | Synergy |
| N1 | **Ray 9** | 0.04 | 0.9716 [0.5780 ; 1.6332] | Additivity |
| N3 | **Ray 10** | 0.11 | 0.7894 [0.527 ; 1.1816] | Additivity |
| N2 | **Ray 10** | 0.11 | 0.6393 [0.4555 ; 0.8974] | Synergy |
| N1 | **Ray 10** | 0.12 | 0.5086 [0.3376 ; 0.7662] | Synergy |
| N3 | **Ray 11** | 0.29 | 0.5741 [0.4014 ; 0.8209] | Synergy |
| N2 | **Ray 11** | 0.30 | 0.5522 [0.4075 ; 0.7485] | Synergy |
| N1 | **Ray 11** | 0.31 | 0.5301 [0.3522 ; 0.7979] | Synergy |
| N3 | **Ray 12** | 0.55 | 0.6952 [0.5030 ; 0.9610] | Synergy |
| N2 | **Ray 12** | 0.56 | 0.6971 [0.5211 ; 0.9326] | Synergy |
| N1 | **Ray 12** | 0.57 | 0.6277 [0.4384 ; 0.8987] | Synergy |
| N3 | **Ray 13** | 0.81 | 0.8010 [0.565 ; 1.1340] | Additivity |
| N2 | **Ray 13** | 0.81 | 0.6753 [0.4990 ; 0.9139] | Synergy |
| N1 | **Ray 13** | 0.82 | 0.7837 [0.5361 ; 1.1457] | Additivity |
| N3 | **Ray 14** | 0.93 | 0.9801 [0.6760 ; 1.4209] | Additivity |
| N2 | **Ray 14** | 0.93 | 0.7425 [0.5427 ; 1.0159] | Additivity |
| N1 | **Ray 14** | 0.93 | 0.7598 [0.4911 ; 1.1755] | Additivity |

FIG. 8 shows an isobologram of growth inhibition of SK-MEL-103 cells treated with the indicated amounts of Compound (1) in combination with Compound (4) (FIG. 8A) or with Compound (5) (FIG. 8B), compared to additive growth inhibition. As shown in Tables 14 and 16, and FIG. 8, the Compound (1) in combination with Compound (4) or Compound (5) showed a synergistic effect on SK-MEL-103 cells.

**Compound (1) shows a trend to synergy with Compounds (4) and (5) in UACC-62 cells.**

Table 17 shows the IC50 estimations for each compound alone and the relative potency of Compounds (1) and (4) on UACC-62 cells.

**Table 17**

| Experiment | Compound 1 | Compound 4 | Relative potency |
|---|---|---|---|
| **N1 (IC50)** | 2.05E+02 [1.51E+02 ; 2.78E+02] | 9.87E+00 [7.75E+00; 1.26E+01] | 20.8 |
| **N2 (IC50)** | 3.44E+02 [2.66E+02 ; 4.45E+02] | 5.16E+00 [4.17E+00 ; 6.39E+00] | 66.72 |
| **N3 (IC50)** | 1.07E+02 [4.05E+01;2.84E+02] | 9.29E+00 [6.57E+00 ; 1.31E+01] | 11.55 |

Table 18 shows the interaction characterization of Compounds (1) and (4) in UACC-62 cells.

**Table 18**

| Experiment | Ray | f values | Ki (confidence interval at 95%) | Interaction characterization |
|---|---|---|---|---|
| N1 | Ray 8 | 0.05 | 0.6078 [0.3961 ; 0.9326] | synergy |
| N2 | Ray 9 | 0.05 | 1.2738 [0.8904 ; 1.8222] | additivity |
| N3 | Ray 8 | 0.08 | 1.0897 [0.5725 ; 2.0740] | additivity |
| N2 | Ray 10 | 0.13 | 1.0951 [0.7723 ; 1.5529] | additivity |
| N1 | Ray 9 | 0.14 | 0.5185 [0.3286 ; 0.8182] | synergy |
| N3 | Ray 9 | 0.22 | 0.8687 [0.4733 ; 1.5945] | additivity |
| N1 | Ray 10 | 0.32 | 0.6805 [0.4350 ; 1.0647] | additivity |
| N2 | Ray 11 | 0.33 | 0.7797 [0.5547 ; 1.0960] | additivity |
| N3 | Ray 10 | 0.46 | 0.4797 [0.2268 ; 1.0147] | additivity |
| N2 | Ray 12 | 0.60 | 0.6365 [0.4473 ; 0.9056] | synergy |
| N1 | Ray 11 | 0.62 | 0.5638 [0.3493 ; 0.9098] | synergy |
| N3 | Ray 11 | 0.74 | 0.3222 [0.1202 ; 0.8642] | synergy |
| N1 | Ray 12 | 0.83 | 0.9596 [0.6085 ; 1.5132] | additivity |
| N2 | Ray 13 | 0.83 | 0.6258 [0.4250 ; 0.9216] | synergy |
| N3 | Ray 12 | 0.90 | 0.4484 [0.1384 ; 1.4521] | additivity |
| N1 | Ray 13 | 0.94 | 0.9395 [0.5671 ; 1.5562] | additivity |
| N2 | Ray 14 | 0.94 | 0.5851 [0.3870 ; 0.8846] | synergy |
| N3 | Ray 13 | 0.97 | 0.7522 [0.2092 ; 2.7049] | additivity |

Table 19 shows the IC50 estimations for each compound alone and the relative potency of Compounds (1) and (5) on UACC-62 cells.

**Table 19**

| Experiment | Compound 1 | Compound 5 | Relative potency |
|---|---|---|---|
| **N1 (IC50)** | 1.95E+02 [1.54E+02 ; 2.47E+02] | 8.35E-01 [6.76E-01 ; 1.03E+00] | 233.36 |
| **N2 (IC50)** | 1. 12E+02 [7.23E+01 ; 1.72E+02] | 1.14E+00 [8.02E-01 ; 1.63E+00] | 98.25 |
| **N3 (IC50)** | 1.32E+02 [9.87E+01 ; 1.77E+02] | 1.03E+00 [8.06E-01 ; 1.31E+00] | 128.15 |

Table 20 shows the interaction characterization of Compounds (1) and (5) in UACC-62 cells.

Table 20

| Experiment | Ray | f values | Ki (confidence interval at 95%) | Interaction characterization |
|---|---|---|---|---|
| N2 | Ray 9 | 0.03 | 0.4761 [0.2605 ; 0.8702] | Synergy |
| N1 | Ray 10 | 0.04 | 0.8046 [0.5605 ; 1.1551] | Additivity |
| N3 | Ray 10 | 0.07 | 0.4425 [0.2921 ; 0.6704] | synergy |
| N2 | Ray 10 | 0.09 | 0.4787 [0.2705 ; 0.8470] | Synergy |
| N1 | Ray 11 | 0.12 | 0.6280 [0.4411 ; 0.8942] | Synergy |
| N3 | Ray 11 | 0.21 | 0.6226 [0.4178 ; 0.9278] | synergy |
| N2 | Ray 11 | 0.25 | 0.4913 [0.2781 ; 0.8680] | Synergy |
| N1 | Ray 12 | 0.30 | 0.8162 [0.5788 ; 1.1510] | Additivity |
| N3 | Ray 12 | 0.44 | 0.7460 [0.4982 ; 1.1173] | additivity |
| N2 | Ray 12 | 0.51 | 0.6003 [0.3398 ; 1.0604] | Additivity |
| N1 | Ray 13 | 0.59 | 0.7101 [0.5015 ; 1.0054] | Additivity |
| N3 | Ray 13 | 0.72 | 0.8353 [0.5321 ; 1.3110] | additivity |
| N2 | Ray 13 | 0.77 | 0.8851 [0.4676 ; 1.6753] | Additivity |
| N1 | Ray 14 | 0.81 | 0.5655 [0.3815 ; 0.8381] | Synergy |
| N3 | Ray 14 | 0.89 | 1.0538 [0.6566 ; 1.6912] | additivity |
| N2 | Ray 14 | 0.91 | 0.7710 [0.3855 ; 1.5421] | Additivity |
| N1 | Ray 15 | 0.93 | 0.5147 [0.3444 ; 0.7694] | Synergy |
| N3 | Ray 15 | 0.96 | 0.9601 [0.6005 ; 1.5351] | additivity |

FIG. 9 shows an isobologram of growth inhibition of UACC-62 cells treated with the indicated amounts of Compound (1) in combination with Compound (4) (FIG. 9A) or Compound (5) (FIG. 9B), compared to additive growth inhibition. As shown in Tables 18 and 20, and FIG. 9, Compound (1) in combination with Compound (4) or Compound (5) showed a trend toward synergy on UACC-62 cells.

**Compound (1) shows synergy with Compounds (4) and (5) in HT-1197 cells.**

Table 21 shows the IC50 estimations for each compound alone and the relative potency of Compounds (1) and (4) on HT-1197 cells.

Table 21

| Experiment | Compound 1 | Compound 4 | Relative potency |
|---|---|---|---|
| **N1 (IC40)** | 3.68E+02 [2.04E+02 ; 6.63E+02] | 3.81E+02 [1.98E+02 ; 7.35E+02] | 0.97 |
| **N2 (IC40)** | 4.10E+02 [2.10E+02 ; 8.00E+02] | 7.30E+01 [3.79E+01 ; 1.41E+02] | 5.61 |
| **N3 (IC40)** | 6.07E+02 [1.93E+02 ; 1.91E+03] | 1.30E+02 [5.25E+01 ; 3.20E+02] | 4.68 |

Table 22 shows interaction characteristics of Compounds (1) and (4) in HT-1197 cells.

Table 22

| Experiment | Ray | f values | Ki (confidence interval at 95%) | Interaction characterization |
|---|---|---|---|---|
| N2 | **Ray 7** | 0.05 | 0.3150 [0.1005 ; 0.9873] | Synergy |
| N3 | **Ray 7** | 0.06 | 0.7615 [0.1413 ; 4.1039] | Additivity |
| N1 | **Ray 6** | 0.09 | 0.0724 [0.0242 ; 0.2164] | Synergy |
| N2 | **Ray 8** | 0.15 | 0.4372 [0.1668 ; 1.1462] | Additivity |
| N3 | **Ray 8** | 0.18 | 0.4460 [0.0992 ; 2.0060] | Additivity |
| N1 | **Ray 7** | 0.24 | 0.0285 [0.0110 ; 0.0734] | Synergy |
| N2 | **Ray 9** | 0.37 | 0.3530 [0.1568 ; 0.7949] | Synergy |
| N3 | **Ray 9** | 0.42 | 0.2049 [0.0444 ; 0.9469] | Synergy |
| N1 | **Ray 8** | 0.51 | 0.0531 [0.0216 ; 0.1306] | Synergy |

(continued)

| Experiment | Ray | f values | Ki (confidence interval at 95%) | Interaction characterization |
|---|---|---|---|---|
| N2 | **Ray 10** | 0.64 | 0.2341 [0.1043 ; 0.5256] | Synergy |
| N3 | **Ray 10** | 0.68 | 0.0377 [0.0101 ; 0.1414] | Synergy |
| N1 | **Ray 9** | 0.78 | 0.0805 [0.0354 ; 0.1829] | Synergy |
| N2 | **Ray 11** | 0.86 | 0.1562 [0.0659 ; 0.3703] | Synergy |
| N3 | **Ray 11** | 0.88 | 0.0499 [0.0131 ; 0.1907] | Synergy |
| N1 | **Ray 10** | 0.92 | 0.0640 [0.0249 ; 0.1648] | Synergy |
| N2 | **Ray 12** | 0.95 | 0.2129 [0.0875 ; 0.5177] | Synergy |
| N2 | **Ray 12** | 0.96 | 0.1648 [0.0370 ; 0.7334] | Synergy |
| N1 | **Ray 11** | 0.97 | 0.1867 [0.0745 ; 0.4678] | Synergy |

Table 23 shows the IC50 estimations for each compound alone and the relative potency of Compounds (1) and (4) on HT-1197 cells.

**Table 23**

| Experiment | Compound 1 | Compound 5 | Relative potency |
|---|---|---|---|
| **N1 (IC40)** | 8.91E+02 [3 59E+02 ; 2.21E+03] | 8.31E+01 [4.05E+01 ; 1.70E+02] | 10.7 |
| **N2 (IC40)** | 6.13E+02 [2.56E+02 ; 1.47E+03] | 1.03E+00 [4.20E+00 ; 2.50E+01] | 59.5 |
| **N3 (IC40)** | 7.30E+02 [4.53E+02 ; 1.18E+03] | 1.61E+01 [9.15E+00 ; 2.82E+01] | 45.34 |
| **N4 (IC40)** | 6.02E+02 [3.74E+02 ; 9.68E+02] | 1.40E+01 [7.93E+00 ; 2.48E+01] | 43 |

Table 24 shows interaction characteristics of Compounds (1) and (5) in HT-1197 cells.

**Table 24**

| Experiment | Ray | f values | Ki (confidence interval at 95%) | Interaction characterization |
|---|---|---|---|---|
| N2 | Ray 9 | 0.05 | 2.0559 [0.5657 ; 7.4714] | Additivity |
| N3 | Ray 9 | 0.07 | 0.2116 [0.0829 ; 0.5403] | synergy |
| N4 | Ray 9 | 0.07 | 0.2840 [0.1189 ; 0.6783] | synergy |
| N1 | Ray 8 | 0.08 | 0.4962 [0.1566 ; 1.5728] | Additivity |
| N2 | Ray 10 | 0.14 | 0.7021 [0.2170 ; 2.2724] | Additivity |
| N3 | Ray 10 | 0.18 | 0.1550 [0.0708 ; 0.3392] | synergy |
| N4 | Ray 10 | 0.19 | 0.1819 [0.0832 ; 0.3980] | synergy |
| N1 | Ray 9 | 0.24 | 0.4944 [0.1796 ; 1.3613] | Additivity |
| N2 | Ray 11 | 0.36 | 0.4624 [0.1758 ; 1.2159] | Additivity |
| N3 | Ray 11 | 0.42 | 0.0947 [0.0470 ; 0.1910] | synergy |
| N4 | Ray 11 | 0.44 | 0.1379 [0.0678 ; 0.2807] | synergy |
| N1 | Ray 10 | 0.48 | 0.3983 [0.1617 ; 0.9813] | Synergy |
| N2 | Ray 12 | 0.63 | 0.5800 [0.2000 ; 1.6815] | Additivity |
| N3 | Ray 12 | 0.69 | 0.0878 [0.0427 ; 0.1804] | synergy |
| N4 | Ray 12 | 0.70 | 0.1580 [0.0787 ; 0.3172] | synergy |
| N1 | Ray 11 | 0.76 | 0.2001 [0.0632 ; 0.6334] | Synergy |
| N2 | Ray 13 | 0.85 | 0.9292 [0.2921 ; 2.9557] | Additivity |
| N3 | Ray 13 | 0.88 | 0.1227 [0.0555 ; 0.2712] | synergy |
| N4 | Ray 13 | 0.89 | 0.2154 [0.1088 ; 0.4264] | synergy |
| N1 | Ray 12 | 0.90 | 0.2923 [0.0875 ; 0.9765] | Synergy |
| N2 | Ray 14 | 0.94 | 1.0114 [0.2919 ; 3.5040] | Additivity |
| N3 | Ray 14 | 0.96 | 0.1820 [0.0783 ; 0.4229] | synergy |
| N4 | Ray 14 | 0.96 | 0.3619 [0.1823 ; 0.7186] | synergy |

(continued)

| Experiment | Ray | f values | Ki (confidence interval at 95%) | Interaction characterization |
|---|---|---|---|---|
| N1 | Ray 13 | 0.97 | 0.2589 [0.0638 ; 1.0500] | Additivity |

FIG. 10 shows an isobologram of growth inhibition of HT-1197 cells treated with the indicated amounts of Compound (1) in combination with Compound (4) (FIG. 10A) or Compound (5) (FIG. 10B), compared to additive growth inhibition. As shown in Table 22 and 24 and FIG. 10, Compound (1) in combination with Compound (4) or Compound (5) showed a synergistic effect on HT-1197 cells.

**Compound (1) shows synergy with Compound (4) and a trend to synergy with Compound (5) in KU-19-19 cells.**

Table 25 shows the IC50 estimations for each compound alone and the relative potency of Compounds (1) and (4) on KU-19-19 cells.

**Table 25**

| Experiment | Compound 1 | Compound 4 | Relative potency |
|---|---|---|---|
| **N1 (IC50)** | 2.14E+02 [1,66E202 ; 2.077E+02] | 4.34E+01 [3.38E+01;5.56E+01] | 4.94 |
| **N2 (IC50)** | 1.78E+02 [1.30E+02 ; 2.44E+02] | 3.30E+01 [2.57E3010E4.023E+OI] | 5.40 |
| **N3 (IC50)** | 2.57E+02 [2.06E+02 ; 3.19E+02] | 2.27E+01 [1.90E+01 ; 2.71E+01] | 11.3 |
| **N4 (IC50)** | 2.63E+02 [1.96E+02 ; 3.51E+02] | 2.99E+01 [2.32E+01 ; 3.85E+01] | 8.79 |

Table 26 shows the interaction characterization of Compounds (1) and (4) in KU-19-19 cells.

**Table 26**

| Experiment | Ray | f values | Ki (confidence interval at 95%) | Interaction characterization |
|---|---|---|---|---|
| N1 | Ray 7 | 0.06 | 2.0177 [1.1799 ; 3.4504] | Antagonism |
| N2 | Ray 7 | 0.05 | 0.5005 [0.3188 ; 0.7860] | Synergy |
| N3 | Ray 8 | 0.08 | 1.1001 [0.828 ; 1.4602] | additivity |
| N4 | Ray 8 | 0.10 | 0.8438 [0.5576 ; 1.2771] | additivity |
| N2 | Ray 8 | 0.16 | 0.5013 [0.3150 ; 0.7978] | Synergy |
| N1 | Ray 8 | 0.17 | 0.6707 [0.428 ; 1.0501] | Additivity |
| N3 | Ray 9 | 0.23 | 0.9098 [0.7001 ; 1.1822] | additivity |
| N4 | Ray 9 | 0.27 | 0.6537 [0.4459 ; 0.9583] | synergy |
| N2 | Ray 9 | 0.38 | 0.6125 [0.3899 ; 0.9622] | Synergy |
| N1 | Ray 9 | 0.40 | 0.6864 [0.4628 ; 1.0180] | Additivity |
| N3 | Ray 10 | 0.47 | 0.6994 [0.5401 ; 0.9056] | synergy |
| N4 | Ray 10 | 0.53 | 0.4986 [0.3424 ; 0.7260] | synergy |
| N2 | Ray 10 | 0.65 | 0.3878 [0.2425 ; 0.6201] | Synergy |
| N1 | Ray 10 | 0.67 | 0.7675 [0.5348 ; 1.1014] | Additivity |
| N3 | Ray 11 | 0.75 | 0.5599 [0.4220 ; 0.7429] | synergy |
| N4 | Ray 11 | 0.79 | 0.4680 [0.3084 ; 0.7104] | synergy |
| N2 | Ray 11 | 0.86 | 0.2520 [0.1560 ; 0.4070] | Synergy |
| N1 | Ray 11 | 0.87 | 0.4106 [0.2679 ; 0.6292] | Synergy |
| N3 | Ray 12 | 0.90 | 0.5630 [0.4159 ; 0.7621] | synergy |
| N4 | Ray 12 | 0.92 | 0.5323 [0.3356 ; 0.8441] | synergy |
| N1 | Ray 12 | 0.95 | 0.7081 [0.4705 ; 1.0656] | Additivity |
| N2 | Ray 12 | 0.95 | 0.9759 [0.6214 ; 1.5325] | Additivity |
| N3 | Ray 13 | 0.97 | 0.6125 [0.4421 ; 0.8487] | synergy |
| N4 | Ray 13 | 0.97 | 0.6048 [0.3705 ; 0.9874] | synergy |

Table 27 shows the IC50 estimations for each compound alone and the relative potency of Compounds (1) and (5) on KU-19-19 cells.

**Table 27**

| Experiment | Compound 1 | Compound 5 | Relative potency |
|---|---|---|---|
| **N1 (IC50)** | 2.80E+02 [2.07E+02 ; 3.79E+02] | 2.60E+01 [1.95E+01 ; 3.46E+01] | 10.77 |
| **N2 (IC50)** | 3.38+02 [2.20E+02 ; 5.19E+02] | 3.04E+00 [2.07E+00 ; 4.48E+00] | 111.18 |
| **N3 (IC50)** | 2.82E+02 [2.12E+02 ; 3.76E+02] | 2.42E+00 [1.98E+00 ; 2.95E+00] | 116.62 |
| **N4 (IC50)** | 1.94E+02 [1.53E+02 ; 2.47E+02] | 2.33E+00 [1.94E+00 ; 2.79E+00] | 83.34 |

Table 28 shows the interaction characterization of Compounds (1) and (5) in KU-19-19 cells.

**Table 28**

| Experiment | Ray | f values | Ki (confidence interval at 95%) | Interaction characterization |
|---|---|---|---|---|
| N2 | Ray 10 | 0.08 | 1.7399 [0.7995 ; 3.7864] | additivity |
| N3 | Ray 10 | 0.08 | 0.8535 [0.6111 ; 1.1920] | additivity |
| N1 | Ray 8 | 0.09 | 1.0924 [0.6788 ; 1.7580] | additivity |
| N4 | Ray 10 | 0.11 | 0.8316 [0.6105 ; 1.1327] | additivity |
| N3 | Ray 11 | 0.22 | 0.6394 [0.4607 ; 0.8873] | synergy |
| N2 | Ray 11 | 0.23 | 1.4696 [0.7745 ; 2.7885] | additivity |
| N1 | Ray 9 | 0.24 | 0.8856 [0.5602 ; 1.3999] | additivity |
| N4 | Ray 11 | 0.29 | 0.7753 [0.5830 ; 1.0311] | additivity |
| N3 | Ray 12 | 0.46 | 0.5302 [0.3765 ; 0.7467] | synergy |
| N2 | Ray 12 | 0.47 | 1.5136 [0.8200 ; 2.7941] | additivity |
| N1 | Ray 10 | 0.48 | 0.8336 [0.5448 ; 1.2755] | additivity |
| N4 | Ray 12 | 0.55 | 0.8118 [0.6135 ; 1.0742] | additivity |
| N3 | Ray 13 | 0.74 | 0.4581 [0.307 ; 0.6816] | synergy |
| N2 | Ray 13 | 0.75 | 1.0034 [0.5070 ; 1.9860] | additivity |
| N1 | Ray 11 | 0.76 | 0.4396 [0.2727 ; 0.7087] | synergy |
| N4 | Ray 13 | 0.80 | 0.8254 [0.6023 ; 1.1312] | additivity |
| N2 | Ray 14 | 0.90 | 0.9596 [0.4619 ; 1.9938] | additivity |
| N1 | Ray 12 | 0.90 | 0.6701 [0.4117 ; 1.0907] | additivity |
| N3 | Ray 14 | 0.90 | 0.4489 [0.2910 ; 0.6922] | synergy |
| N4 | Ray 14 | 0.92 | 0.8246 [0.5809 ; 1.1705] | additivity |
| N1 | Ray 13 | 0.97 | 0.7644 [0.4450 ; 1.3130] | additivity |
| N2 | Ray 15 | 0.97 | 0.7806 [0.3535 ; 1.7238] | additivity |
| N3 | Ray 15 | 0.97 | 0.4715 [0.3011 ; 0.7383] | synergy |
| N4 | Ray 15 | 0.98 | 0.6628 [0.4572 ; 0.9608] | synergy |

FIG. 11 shows an isobologram of growth inhibition of KU-19-19 cells treated with the indicated amounts of Compound (1) in combination with Compound (4) (FIG. 11A) or Compound (5) (FIG. 11B), compared to additive growth inhibition. As shown in Tables 26 and 28, and FIG. 11, Compound (1) in combination with Compound (4) showed a synergistic effect on KU-19-19 cells and Compound (1) in combination with Compound (5) showed a trend to synergy.

**Compound (1) shows additivity with Compound (4) and Compound (5) in a Braf mutant melanoma cell line, A375.**

Table 29 shows the IC50 estimations for each compound alone and relative potency of Compounds (1) and (4) on A375 cells.

Table 29

| Experiment | Compound 1 | Compound 4 | Relative potency |
|---|---|---|---|
| **N1 (IC50)** | 3.10E+02 [2.67E+02 ; 3.60E+02] | 1.01E+01 [8.64E+00 ; 1.18E+01] | 30.7 |
| **N2 (IC50)** | 2.88E+02 [2.41E+02 ; 3.45E+02] | 1.12E+01 [9.32E+00 ; 1.35E+01] | 25.7 |

Table 30 shows the interaction characteristics of Compounds (1) and (4) in A375 cells.

Table 30

| Experiment | Ray | f values | Ki (confidence interval at 95%) | Interaction characterization |
|---|---|---|---|---|
| N1 | **Ray 8** | 0.03 | 1.0838 [0.8505 ; 1.3812] | Additivity |
| N2 | **Ray 8** | 0.04 | 0.9608 [0.6980 ; 1.3228] | Additivity |
| N1 | **Ray 9** | 0.10 | 0.9830 [0.7610 ; 1.2697] | Additivity |
| N2 | **Ray 9** | 0.11 | 1.1334 [0.8346 ; 1.5392] | Additivity |
| N1 | **Ray 10** | 0.25 | 1.0991 [0.8656 ; 1.3957] | Additivity |
| N2 | **Ray 10** | 0.28 | 1.1329 [0.8649 ; 1.4839] | Additivity |
| N1 | **Ray 11** | 0.52 | 1.1522 [0.9368 ; 1.4171] | Additivity |
| N2 | **Ray 11** | 0.56 | 1.028 [0.7835 ; 1.3487] | Additivity |
| N1 | **Ray 12** | 0.76 | 0.9289 [0.7305 ; 1.1811] | Additivity |
| N2 | **Ray 12** | 0.80 | 1.2189 [0.9297 ; 1.5982] | Additivity |
| N1 | **Ray 13** | 0.92 | 0.7160 [0.5463 ; 0.9384] | Synergy |
| N2 | **Ray 13** | 0.93 | 1.0725 [0.7907 ; 1.4548] | Additivity |

Table 31 shows the IC50 estimations for each compound alone and relative potency of Compounds (1) and (5) on A375 cells.

Table 31

| Experiment | Compound 1 | Compound 5 | Relative potency |
|---|---|---|---|
| **N1 (IC50)** | 2.64E+02 [2.38E+02 ; 2.92E+02] | 7.01E-01 [6.32E-01 ; 7.77E-01] | 376.3 |
| **N2 (IC50)** | 2.89E+02 [2.41E+02 ; 3.47E+02] | 9.26E-01 [7.81E-01 ; 1.10E+00] | 312.3 |

Table 32 shows the interaction characteristics of Compounds (1) and (5) in A375 cells.

Table 32

| Experiment | Ray | f values | Ki (confidence interval at 95%) | Interaction characterization |
|---|---|---|---|---|
| N2 | **Ray 10** | 0.03 | 1.8132 [1.3613 ; 2.4153] | Antagonism |
| N1 | **Ray 11** | 0.08 | 1.1294 [0.9616 ; 1.3265] | Additivity |
| N2 | **Ray 11** | 0.10 | 0.8676 [0.6326 ; 1.1899] | Additivity |
| N1 | **Ray 12** | 0.21 | 1.2616 [1.0814 ; 1.4717] | Antagonism |
| N2 | **Ray 12** | 0.24 | 1.1801 [0.9184 ; 1.5164] | Additivity |
| N1 | **Ray 13** | 0.47 | 1.1666 [1.0061 ; 1.3528] | Antagonism |
| N2 | **Ray 13** | 0.52 | 1.1023 [0.8396 ; 1.4471] | Additivity |
| N1 | **Ray 14** | 0.73 | 1.2623 [1.0831 ; 1.4711] | Antagonism |
| N2 | **Ray 14** | 0.76 | 1.1830 [0.9087 ; 1.5399] | Additivity |
| N1 | **Ray 15** | 0.90 | 1.0731 [0.9103 ; 1.2651] | Additivity |
| N2 | **Ray 15** | 0.91 | 0.8740 [0.6238 ; 1.2248] | Additivity |
| N1 | **Ray 16** | 0.96 | 1.1028 [0.9311 ; 1.3063] | Additivity |

FIG. 12 shows an isobologram of growth inhibition of A375 cells treated with the indicated amounts of Compound (1) in combination with Compound (4) (FIG. 12A) or with Compound (5) (FIG. 12B) compared to additive growth inhibition.

As shown in Tables 30 and 32, and FIG. 12, Compound (1) in combination with Compound (4) or (5) showed an additive effect on A375 cells.

**Compound (1) shows synergy with Compounds (4) and (5) in Braf mutant melanoma cells, C32.**

Table 33 shows the IC50 estimations for each compound alone and relative potency of Compounds (1) and (4) on C32 cells.

Table 33

| Experiment | Compound 1 | Compound 4 | Relative potency |
|---|---|---|---|
| **N1 (IC50)** | 8.13E+02 [5.86E+02 ; 1.13E+03] | 1.74E+01 [1.47E+01 ; 2.06E+01] | 46.7 |
| **N2 (IC50)** | 9.62E+02 [6.75E+02 ; 1.37E+03] | 2.36E+01 [2.03E+01 ; 2.74E+01] | 40.8 |
| **N3 (IC50)** | 1.45E+03 [1.19E+03 ; 1.77E+03] | 2.27E+01 [2.03E+01 ; 2.54E+01] | 64.1 |

Table 34 shows the interaction characteristics of Compounds (1) and (4) in C32 cells.

**Table 34**

| Experiment | Ray | f values | Ki (confidence interval at 95%) | Interaction characterization |
|---|---|---|---|---|
| N3 | **Ray 9** | 0.05 | 0.7614 [0.6225 ; 0.9313] | Synergy |
| N1 | **Ray 9** | 0.07 | 0.7740 [0.5815 ; 1.0303] | Additivity |
| N2 | **Ray 9** | 0.08 | 0.8382 [0.6282 ; 1.1183] | Additivity |
| N3 | **Ray 10** | 0.14 | 0.6935 [0.5733 ; 0.8388] | Synergy |
| N1 | **Ray 10** | 0.18 | 0.7019 [0.5210 ; 0.9457] | Synergy |
| N2 | **Ray 10** | 0.20 | 0.6164 [0.4593 ; 0.8272] | Synergy |
| N3 | **Ray 11** | 0.34 | 0.5301 [0.4268 ; 0.6585] | Synergy |
| N1 | **Ray 11** | 0.42 | 0.6887 [0.4903 ; 0.9673] | Synergy |
| N2 | **Ray 11** | 0.45 | 0.5207 [0.3702 ; 0.7324] | Synergy |
| N3 | **Ray 12** | 0.61 | 0.5798 [0.4516 ; 0.7444] | Synergy |
| N1 | **Ray 12** | 0.68 | 0.7610 [0.5068 ; 1.1427] | Additivity |
| N2 | **Ray 12** | 0.71 | 0.7183 [0.4802 ; 1.0744] | Additivity |
| N3 | **Ray 13** | 0.84 | 0.6554 [0.4819 ; 0.8912] | Synergy |
| N1 | **Ray 13** | 0.88 | 0.9736 [0.6169 ; 1.5366] | Additivity |
| N2 | **Ray 13** | 0.89 | 0.9132 [0.5504 ; 1.5150] | Additivity |
| N3 | **Ray 14** | 0.94 | 0.7955 [0.5759 ; 1.0988] | Additivity |
| N1 | **Ray 14** | 0.96 | 1.2713 [0.7803 ; 2.0713] | Additivity |
| N2 | **Ray 14** | 0.96 | 1.3182 [0.7815 ; 2.2235] | Additivity |

Table 35 shows the IC50 estimations for each compound alone and relative potency of Compounds (1) and (5) on C32 cells.

**Table 35**

| Experiment | Compound 1 | Compound 5 | Relative potency |
|---|---|---|---|
| **N1 (IC40)** | 8.23E+02 [4.42E+02 ; 1.53E+03] | 1.13E+00 [9.89E-01 ; 1.29E+00] | 727 |
| **N2 (IC40)** | 6.18E+02 [3.44E+02 ; 1.11E+03] | 1.19E+00 [9.79E-01 ; 1.45E+00] | 518.5 |
| **N3 (IC40)** | 2.15E+03 [1.77E+03 ; 2.62E+03] | 8.46E-01 [7.06E-01 ; 1.02E+00] | 2545.0 |
| **N4 (IC40)** | 1.07E+03 [7.24E+02 ; 1.57E+03] | 7.88E-01 [5.93E-01 ; 1.05E+00] | 1353.5 |

Table 36 shows the interaction characteristics of Compounds (1) and (5) in C32 cells.

Table 36

| Experiment | Ray | f values | Ki (confidence interval at 95%) | Interaction characterization |
|---|---|---|---|---|
| N4 | **Ray 11** | 0.02 | 0.7309 [0.4502 ; 1.1867] | Additivity |
| N1 | **Ray 11** | 0.04 | 0.6662 [0.5318 ; 0.8347] | Synergy |
| N3 | **Ray 12** | 0.04 | 0.9525 [0.7258 ; 1.2500] | Additivity |
| N2 | **Ray 11** | 0.06 | 0.7353 [0.5151 ; 1.0497] | Additivity |
| N4 | **Ray 12** | 0.07 | 0.5630 [0.3466 ; 0.9144] | Synergy |
| N1 | **Ray 12** | 0.12 | 0.8010 [0.6482 ; 0.9899] | Synergy |
| N3 | **Ray 13** | 0.12 | 0.6808 [0.5177 ; 0.8954] | Synergy |
| N2 | **Ray 12** | 0.16 | 0.7696 [0.5376 ; 1.1018] | Additivity |
| N4 | **Ray 13** | 0.20 | 0.6761 [0.407 ; 1.1206] | Additivity |
| N3 | **Ray 14** | 0.28 | 0.5832 [0.4382 ; 0.7762] | Synergy |
| N1 | **Ray 13** | 0.31 | 0.6753 [0.5002 ; 0.9118] | Synergy |
| N2 | **Ray 13** | 0.39 | 0.6700 [0.4271 ; 1.0509] | Additivity |
| N4 | **Ray 14** | 0.42 | 0.4637 [0.2750 ; 0.7819] | Synergy |
| N3 | **Ray 15** | 0.57 | 0.6566 [0.4961 ; 0.8690] | Synergy |
| N2 | **Ray 14** | 0.66 | 1.0079 [0.5921 ; 1.7157] | Additivity |
| N4 | **Ray 15** | 0.71 | 0.5161 [0.3036 ; 0.8772] | Synergy |
| N3 | **Ray 16** | 0.80 | 0.8643 [0.6417 ; 1.1639] | Additivity |
| N1 | **Ray 15** | 0.82 | 0.9326 [0.5200 ; 1.6726] | Additivity |
| N2 | **Ray 15** | 0.87 | 1.5207 [0.8099 ; 2.8552] | Additivity |
| N1 | **Ray 16** | 0.93 | 0.6917 [0.3420 ; 1.3991] | Additivity |
| N3 | **Ray 17** | 0.93 | 1.0378 [0.7652 ; 1.4075] | Additivity |

FIG. 13 shows an isobologram of growth inhibition of C32 cells treated with the indicated amounts of Compound (1) in combination with Compound (4) (FIG. 13A) or with Compound (5) (FIG. 13B) compared to additive growth inhibition. As shown in Tables 34 and 36, and FIG. 13, Compound (1) in combination with Compound (4) or Compound (5) showed a synergistic effect on C32 cells.

**Compound (1) shows synergy with Compounds (4) and (5) in SK-MEL-147 cells.**

Table 37 shows the IC50 estimations for each compound alone and the relative potency of Compounds (1) and (4) on SK-MEL-147 cells.

Table 37

| Experiment | Compound 1 | Compound 4 | Relative potency |
|---|---|---|---|
| **N1 (IC50)** | 5.56E+02 [3.97E+02 ; 7.77E+02] | 9.12E+01 [6.45E+01 ; 1.29E+02] | 6.1 |
| **N2 (IC50)** | 8.78E+02 [6.56E+02; 1.17E+03] | 8.24E+01 [5.99E+01 ; 1.13E+02] | 10.7 |
| **N3 (IC50)** | 8.66E+02 [6.57E+02; 1.14E+03] | 8.14E+01 [6.08E+01 ; 1.09E+02] | 10.6 |

Table 38 shows the interaction characteristics of Compounds (1) and (4) in SK-MEL-147 cells.

Table 38

| Experiment | Ray | f values | Ki (confidence interval at 95%) | Interaction characterization |
|---|---|---|---|---|
| N2 | **Ray 7** | 0.03 | 0.8443 [0.4859 ; 1.4671] | Additivity |
| N1 | **Ray 7** | 0.05 | 0.6337 [0.3663 ; 1.0965] | Additivity |
| N2 | **Ray 8** | 0.09 | 0.6180 [0.3662 ; 1.0430] | Additivity |
| N3 | **Ray 8** | 0.09 | 0.6558 [0.3798 ; 1.1323] | Additivity |
| N1 | **Ray 8** | 0.14 | 0.3892 [0.228 ; 0.6636] | Synergy |
| N2 | **Ray 9** | 0.24 | 0.6069 [0.3901 ; 0.9443] | Synergy |

(continued)

| Experiment | Ray | f values | Ki (confidence interval at 95%) | Interaction characterization |
|---|---|---|---|---|
| N3 | **Ray 9** | 0.24 | 0.2878 [0.1692 ; 0.4896] | Synergy |
| N1 | **Ray 9** | 0.35 | 0.4817 [0.2814 ; 0.8248] | Synergy |
| N2 | **Ray 10** | 0.48 | 0.7068 [0.4690 ; 1.0652] | Additivity |
| N3 | **Ray 10** | 0.48 | 0.4086 [0.2603 ; 0.6413] | Synergy |
| N1 | **Ray 10** | 0.62 | 0.4286 [0.2576 ; 0.7131] | Synergy |
| N2 | **Ray 11** | 0.76 | 0.5105 [0.3279 ; 0.7947] | Synergy |
| N3 | **Ray 11** | 0.76 | 0.4263 [0.2726 ; 0.6668] | Synergy |
| N1 | **Ray 11** | 0.85 | 0.4476 [0.2623 ; 0.7640] | Synergy |
| N2 | **Ray 12** | 0.90 | 0.6678 [0.4270 ; 1.0444] | Additivity |
| N3 | **Ray 12** | 0.90 | 0.5595 [0.3526 ; 0.8877] | Synergy |
| N1 | **Ray 12** | 0.94 | 0.5124 [0.2948 ; 0.8908] | Synergy |
| N3 | **Ray 13** | 0.97 | 0.6469 [0.3981 ; 1.0512] | Additivity |

Table 39 shows the IC50 estimations for each compound alone and the relative potency of Compounds (1) and (5) on SK-MEL-147 cells.

**Table 39**

| Experiment | Compound 1 | Compound 5 | Relative potency |
|---|---|---|---|
| **N1 (IC50)** | 9.78E+02 [7.39E+02 ; 1.29E+03] | 6.94E+00 [5.39E+00 ; 8.93E+00] | 141.0 |
| **N2 (IC50)** | 9.68E+02 [7.93E+02 ; 1.18E+03] | 6.94E+00 [5.62E+00 ; 8.57E+] | 139.4 |
| **N3 (IC50)** | 1.08E+03 [8.80E+02 ; 1.32E+03] | 7.42E+00 [6.05E+00 ; 9.10E+00] | 145.2 |

Table 40 shows the interaction characteristics of Compounds (1) and (5) in SK-MEL-147 cells.

**Table 40**

| Experiment | Ray | f values | Ki (confidence interval at 95%) | Interaction characterization |
|---|---|---|---|---|
| N3 | Ray 10 | 0.06 | 0.6569 [0.4573 ; 0.9436] | Synergy |
| N1 | Ray 10 | 0.07 | 0.6580 [0.4227 ; 1.0243] | Additivity |
| N2 | Ray 10 | 0.07 | 0.8604 [0.6040 ; 1.2258] | Additivity |
| N1 | Ray 11 | 0.19 | 0.5333 [0.3346 ; 0.8498] | Synergy |
| N2 | Ray 11 | 0.19 | 0.5885 [0.4167 ; 0.8311] | Synergy |
| N3 | Ray 11 | 0.19 | 0.5846 [0.4240 ; 0.8061] | Synergy |
| N1 | Ray 12 | 0.41 | 0.6460 [0.4447 ; 0.9383] | Synergy |
| N3 | Ray 12 | 0.41 | 0.4436 [0.3235 ; 0.6084] | Synergy |
| N2 | Ray 12 | 0.42 | 0.5921 [0.4299 ; 0.8156] | Synergy |
| N1 | Ray 13 | 0.70 | 0.5642 [0.3659 ; 0.8702] | Synergy |
| N3 | Ray 13 | 0.70 | 0.4736 [0.3484 ; 0.6439] | Synergy |
| N2 | Ray 13 | 0.71 | 0.4888 [0.3511 ; 0.6804] | Synergy |
| N3 | Ray 14 | 0.87 | 0.5728 [0.4078 ; 0.8047] | Synergy |
| N1 | Ray 14 | 0.88 | 0.7568 [0.4829 ; 1.1860] | Additivity |
| N2 | Ray 14 | 0.88 | 0.6583 [0.469 ; 0.9222] | Synergy |
| N1 | Ray 15 | 0.96 | 0.8858 [0.5535 ; 1.4177] | Additivity |
| N2 | Ray 15 | 0.96 | 0.7126 [0.5018 ; 1.0120] | Additivity |
| N3 | Ray 15 | 0.96 | 0.7981 [0.5680 ; 1.1215] | Additivity |

FIG. 14 shows an isobologram of growth inhibition of SK-MEL-147 cells treated with the indicated amounts of Compound (1) in combination with Compound (4) (FIG. 14A) or Compound (5) (FIG. 14B), compared to additive growth inhibition. As shown in Tables 38 and 40, and FIG. 14, the combination of Compound (1) and Compound (4) or (5)

showed a synergistic effect on SK-MEL-147 cells.

**Compound (1) shows additivity with Compound (4) in DV90 cells.**

Table 41 shows the IC50 estimations for each compound used alone and relative potency of Compounds (1) and (4) on DV90 cells.

**Table 41**

| Experiment | Compound 1 | Compound 4 | Relative potency |
|---|---|---|---|
| **N1 (IC50)** | 1.40E+02 [6.90E+01 ; 2.85E+02] | 3.64E+01 [2.10E+01 ; 6.30E+01] | 3.9 |
| **N2 (IC50)** | 1.63E+02 [9.22E+01 ; 2.87E+02] | 1.97E+01 [9.77E+00 ; 3.99E+01] | 8.2 |
| **N3 (IC50)** | 2.54E+02 [1.48E+02 ; 4.35E+02] | 5.55E+01 [3.23E+01 ; 9.52E+01] | 4.6 |

Table 42 shows the interaction characteristics of Compounds (1) and (4) in DV90 cells.

**Table 42**

| Experiment | Ray | f values | Ki (confidence interval at 95%) | Interaction characterization |
|---|---|---|---|---|
| N1 | **Ray 6** | 0.03 | 1.1814 [0.428 ; 3.2561] | Additivity |
| N2 | **Ray 7** | 0.04 | 1.8696 [0.5511 ; 6.3424] | Additivity |
| N3 | **Ray 7** | 0.06 | 0.4777 [0.1699 ; 1.3435] | Additivity |
| N1 | **Ray 7** | 0.07 | 0.7219 [0.2082 ; 2.5032] | Additivity |
| N2 | **Ray 8** | 0.11 | 0.9143 [0.31999; 2.6131] | Additivity |
| N3 | **Ray 8** | 0.18 | 1.4945 [0.5946 ; 3.7567] | Additivity |
| N1 | **Ray 8** | 0.21 | 0.7855 [0.2519 ; 2.4499] | Additivity |
| N2 | **Ray 9** | 0.29 | 1.0474 [0.3818 ; 2.8733] | Additivity |
| N3 | **Ray 9** | 0.42 | 0.6976 [0.2907 ; 1.6745] | Additivity |
| N1 | **Ray 9** | 0.46 | 0.8797 [0.3443 ; 2.2477] | Additivity |
| N2 | **Ray 10** | 0.55 | 0.7820 [0.3678 ; 1.6623] | Additivity |
| N3 | **Ray 10** | 0.69 | 0.4475 [0.1569 ; 1.2760] | Additivity |
| N1 | **Ray 10** | 0.72 | 0.4393 [0.164 ; 1.1715] | Additivity |
| N2 | **Ray 11** | 0.80 | 1.4445 [0.6724 ; 3.1029] | Additivity |
| N3 | **Ray 11** | 0.88 | 0.3989 [0.1488 ; 1.0693] | Additivity |
| N1 | **Ray 11** | 0.90 | 0.4137 [0.1524 ; 1.1232] | Additivity |
| N2 | **Ray 12** | 0.92 | 1.4103 [0.5895 ; 3.3738] | Additivity |
| N3 | **Ray 12** | 0.96 | 0.7001 [0.307 ; 1.5920] | Additivity |

Table 43 shows the IC50 estimations for each compound used alone and relative potency of Compounds (1) and (5) on DV90 cells.

**Table 43**

| Experiment | Compound 1 | Compound 5 | Relative potency |
|---|---|---|---|
| **N1 (IC50)** | 1.39E+02 [8.65E+01 ; 2.23E+02] | 3.23E+00 [2.20E+00 ; 4.72E+00] | 43.1 |
| **N2 (IC50)** | 7.83E+01 [3.74E+01 ; 1.64E+02] | 1.20E+00 [6.47E-01 ; 2.24E+00] | 65.0 |
| **N3 (IC50)** | 2.07E+02 [1.57E+02 ; 2.72E+02] | 9.03E-01 [4.62E-01 ; 1.77E+00] | 228.9 |

Table 44 shows the interaction characteristics of Compounds (1) and (5) in DV90 cells.

Table 44

| Experiment | Ray | f values | Ki (confidence interval at 95%) | Interaction characterization |
|---|---|---|---|---|
| N3 | **Ray 10** | 0.04 | 3.2416 [1.5428 ; 6.8111] | Antagonism |
| N2 | **Ray 9** | 0.05 | 0.4163 [0.1210 ; 1.4320] | Additivity |
| N1 | **Ray 9** | 0.07 | 1.6379 [0.8013 ; 3.3482] | Additivity |
| N2 | **Ray 10** | 0.13 | 0.8221 [0.3180 ; 2.1249] | Additivity |
| N3 | **Ray 11** | 0.13 | 2.6946 [1.3277 ; 5.4687] | Antagonism |
| N1 | **Ray 10** | 0.19 | 0.8337 [0.4408 ; 1.5766] | Additivity |
| N3 | **Ray 12** | 0.30 | 1.6455 [0.9386 ; 2.8849] | Additivity |
| N2 | **Ray 11** | 0.34 | 0.1919 [0.0804 ; 0.4583] | Synergy |
| N1 | **Ray 11** | 0.44 | 0.6070 [0.3066 ; 1.2017] | Additivity |
| N3 | **Ray 13** | 0.59 | 1.0816 [0.6800 ; 1.7205] | Additivity |
| N2 | **Ray 12** | 0.61 | 0.0795 [0.0323 ; 0.1954] | Synergy |
| N1 | **Ray 12** | 0.70 | 0.5166 [0.2706 ; 0.9862] | Synergy |
| N3 | **Ray 14** | 0.81 | 1.2789 [0.8622 ; 1.8971] | Additivity |
| N2 | **Ray 13** | 0.84 | 0.0936 [0.0319 ; 0.2745] | Synergy |
| N1 | **Ray 13** | 0.89 | 0.8042 [0.4102 ; 1.5764] | Additivity |
| N2 | **Ray 14** | 0.94 | 0.1161 [0.0412 ; 0.3278] | Synergy |
| N3 | **Ray 15** | 0.94 | 0.9426 [0.5601 ; 1.5863] | Additivity |
| N1 | **Ray 14** | 0.96 | 1.3607 [0.6770 ; 2.7348] | Additivity |

FIG. 15 shows an isobologram of growth inhibition of DV90 cells treated with the indicated amounts of Compound (1) in combination with Compound (4) (FIG. 15A) or Compound (5) (FIG. 15B), compared to additive growth inhibition. As shown in Tables 42 and 43, and FIG. 15, Compound (1) in combination with Compound (4) showed additivity on DV90 cells.

**Compound (1) shows synergy with Compound (5) in H460 cells.**

Table 45 shows the IC50 estimations for each compound alone and relative potency of Compounds (1) and (4) on H460 cells.

Table 45

| Experiment | Compound 1 | Compound 4 | Relative potency |
|---|---|---|---|
| **N1 (IC50)** | 3.19E+02 [2.61E+02 ; 3.89E+02] | 1.55E+02 [1.07E+02 ; 2.24E+02] | 2.1 |
| **N2 (IC50)** | 1.66E+02 [1.25E+02 ; 2.20E+02] | 8.40E+01 [6.20E+01 ; 1.14E+02] | 2.0 |
| **N3 (IC50)** | 1.36E+02 [9.00E+01 ; 2.07E+02] | 1.04E+02 [6.48E+01 ; 1.68E+02] | 1.3 |

Table 46 shows the interaction characteristics of Compounds (1) and (4) in H460 cells.

Table 46

| Experiment | Ray | f values | Ki (confidence interval at 95%) | Interaction characterization |
|---|---|---|---|---|
| N1 | **Ray 6** | 0.05 | 1.5331 [0.9970 ; 2.3574] | Additivity |
| N2 | **Ray 6** | 0.05 | 0.7694 [0.3938 ; 1.5031] | Additivity |
| N3 | **Ray 6** | 0.07 | 0.7949 [0.3405 ; 1.8555] | Additivity |
| N1 | **Ray 7** | 0.13 | 0.7955 [0.4571 ; 1.3843] | Additivity |
| N2 | **Ray 7** | 0.13 | 1.1173 [0.6732 ; 1.8545] | Additivity |
| N3 | **Ray 7** | 0.19 | 0.7763 [0.3564 ; 1.6910] | Additivity |
| N1 | **Ray 8** | 0.33 | 0.5376 [0.3261 ; 0.8862] | Synergy |
| N2 | **Ray 8** | 0.34 | 1.3456 [0.8795 ; 2.0587] | Additivity |
| N3 | **Ray 8** | 0.43 | 1.7012 [0.8964 ; 3.2285] | Additivity |

(continued)

| Experiment | Ray | f values | Ki (confidence interval at 95%) | Interaction characterization |
|---|---|---|---|---|
| N1 | **Ray 9** | 0.62 | 0.6094 [0.3864 ; 0.9611] | Synergy |
| N2 | **Ray 9** | 0.63 | 0.5693 [0.3675 ; 0.8819] | Synergy |
| N3 | **Ray 9** | 0.72 | 0.4729 [0.2427 ; 0.9214] | Synergy |
| N1 | **Ray 10** | 0.83 | 0.6137 [0.3982 ; 0.9456] | Synergy |
| N2 | **Ray 10** | 0.83 | 1.0664 [0.7122 ; 1.5966] | Additivity |
| N3 | **Ray 10** | 0.88 | 0.4807 [0.2338 ; 0.9886] | Synergy |
| N1 | **Ray 11** | 0.94 | 1.4303 [1.0277 ; 1.9907] | Antagonism |
| N2 | **Ray 11** | 0.94 | 1.0616 [0.6981 ; 1.6144] | Additivity |
| N3 | **Ray 11** | 0.96 | 0.8813 [0.4655 ; 1.6684] | Additivity |

Table 47 shows the IC50 estimations for each compound alone and relative potency of Compounds (1) and (5) on H460 cells.

**Table 47**

| Experiment | Compound 1 | Compound 5 | Relative potency |
|---|---|---|---|
| **N1 (IC50)** | 2.53E+02 [2.15E+02 ; 2.99E+02] | 6.82E+00 [4.95E+00 ; 9.39E+00] | 37.1 |
| **N2 (IC50)** | 3.19E+02 [2.72E+02 ; 3.75E+02] | 1.01E+01 [7.69E+00 ; 1.34E+01] | 31.5 |
| **N3 (IC50)** | 2.97E+02 [2.29E+02 ; 3.87E+02] | 1.10E+01 [7.67E+00 ; 1.59E+01] | 26.9 |

Table 48 shows the interaction characteristics of Compounds (1) and (5) in H460 cells.

**Table 48**

| Experiment | Ray | f values | Ki (confidence interval at 95%) | Interaction characterization |
|---|---|---|---|---|
| N3 | **Ray 8** | 0.04 | 1.7372 [0.9459 ; 3.1903] | Additivity |
| N2 | **Ray 9** | 0.10 | 0.7189 [0.4787 ; 1.0795] | Additivity |
| N3 | **Ray 9** | 0.11 | 0.7200 [0.4072 ; 1.2730] | Additivity |
| N1 | **Ray 10** | 0.21 | 0.7177 [0.4766 ; 1.0808] | Additivity |
| N2 | **Ray 10** | 0.24 | 0.7328 [0.5205 ; 1.0318] | Additivity |
| N3 | **Ray 10** | 0.27 | 0.9753 [0.6162 ; 1.5438] | Additivity |
| N1 | **Ray 11** | 0.47 | 0.6777 [0.4884 ; 0.9405] | Synergy |
| N2 | **Ray 11** | 0.51 | 0.6608 [0.4894 ; 0.8923] | Synergy |
| N3 | **Ray 11** | 0.55 | 0.2378 [0.1355 ; 0.4172] | Synergy |
| N1 | **Ray 12** | 0.73 | 0.6800 [0.504 ; 0.9166] | Synergy |
| N2 | **Ray 12** | 0.76 | 0.6296 [0.4807 ; 0.8246] | Synergy |
| N3 | **Ray 12** | 0.79 | 0.4957 [0.3146 ; 0.7811] | Synergy |
| N1 | **Ray 13** | 0.90 | 0.7058 [0.5186 ; 0.9607] | Synergy |
| N2 | **Ray 13** | 0.91 | 0.6739 [0.4993 ; 0.9095] | Synergy |
| N3 | **Ray 13** | 0.93 | 0.8401 [0.5394 ; 1.3085] | Additivity |
| N1 | **Ray 14** | 0.96 | 0.9734 [0.7417 ; 1.2775] | Additivity |
| N2 | **Ray 14** | 0.97 | 0.7608 [0.5925 ; 0.9770] | Synergy |

FIG. 16 shows an isobologram of growth inhibition of H460 cells treated with the indicated amounts of Compound (1) in combination with Compound (4) (FIG. 16A) or Compound (5) (FIG. 16B), compared to additive growth inhibition. As shown in Tables 46 and 48, and FIG. 16, Compound (1) in combination with Compound (5) showed a synergistic effect on H460 cells.

**Compound (1) shows additivity with Compounds (4) and (5) in UM-UC-3 cells.**

Table 49 shows the IC50 estimations for each compound alone and relative potency of Compounds (1) and (4) on UM-UC-3 cells.

**Table 49**

| Experiment | Compound 1 | Compound 4 | Relative potency |
|---|---|---|---|
| **N1 (IC50)** | 8.53E+03 [6.54E+03 ; 1.11E+04] | 2.63E+02 [1.53E+02 ; 4.52E+02] | 32.48 |
| **N2 (IC50)** | 6.00E+03 [4.86E+03 ; 7.41E+03] | 7.37E+01 [4.92E+01 ; 1.10E+02] | 81.43 |

Table 50 shows the interaction characteristics of Compounds (1) and (4) in UM-UC-3 cells.

**Table 50**

| Experiment | Ray | f values | Ki (confidence interval at 95%) | Interaction characterization |
|---|---|---|---|---|
| N2 | Ray 9 | 0.04 | 1.7681 [0.9837; 3.1780] | Additivity |
| N1 | Ray 9 | 0.09 | 1.2061 [0.5751; 2.5293] | Additivity |
| N2 | Ray 10 | 0.11 | 1.0148 [0.5706; 1.8047] | Additivity |
| N1 | Ray 10 | 0.24 | 0.7708 [0.4036; 1.4723] | Additivity |
| N2 | Ray 11 | 0.29 | 0.6674 [0.3921; 1.1360] | Additivity |
| N1 | Ray 11 | 0.51 | 0.6154 [0.3304; 1.1462] | Additivity |
| N2 | Ray 12 | 0.55 | 0.9561 [0.7191; 1.2711] | Additivity |
| N1 | Ray 12 | 0.75 | 0.7632 [0.4881; 1.1933] | Additivity |
| N2 | Ray 13 | 0.80 | 0.8798 [0.6518; 1.1875] | Additivity |
| N1 | Ray 13 | 0.91 | 0.9695 [0.6166; 1.5244] | Additivity |
| N2 | Ray 14 | 0.92 | 0.9271 [0.6730; 1.2770] | Additivity |
| N1 | Ray 14 | 0.97 | 0.9708 [0.6301; 1.4957] | Additivity |

Table 51 shows the IC50 estimations for each compound alone and relative potency of Compounds (1) and (5) on UM-UC-3 cells.

**Table 51**

| Experiment | Compound 1 | Compound 5 | Relative potency |
|---|---|---|---|
| **N1 (IC50)** | 6.95E+03 [5.65E+03 ; 8.55E+03] | 2.24E+02 [1.31E+02 ; 3.85E+02] | 31.0 |
| **N2 (IC50)** | 5.40E+03 [4.54E+03 ; 6.43E+03] | 2.34E+01 [1.65E+01 ; 3.33E+01] | 230.5 |
| **N3 (IC50)** | 5.75E+03 [4.52E+03 ; 7.31E+03] | 2.30E+01 [1.43E+01 ; 3.70E+01] | 249.7 |

Table 52 shows the interaction characteristics of Compounds (1) and (5) in UM-UC-3 cells.

**Table 52**

| Experiment | Ray | f values | Ki (confidence interval at 95%) | Interaction characterization |
|---|---|---|---|---|
| N1 | **Ray 8** | 0.03 | 1.0778 [0.4454 ; 2.6084] | Additivity |
| N2 | **Ray 10** | 0.04 | 0.9437 [0.5595 ; 1.5917] | Additivity |
| N3 | **Ray 10** | 0.04 | 1.1040 [0.5600 ; 2.1765] | Additivity |
| N1 | **Ray 9** | 0.10 | 0.9785 [0.4360 ; 2.1962] | Additivity |
| N3 | **Ray 11** | 0.12 | 0.8211 [0.4481 ; 1.5044] | Additivity |
| N2 | **Ray 11** | 0.13 | 0.8543 [0.5354 ; 1.3630] | Additivity |
| N1 | **Ray 10** | 0.24 | 1.4840 [0.6757 ; 3.2592] | Additivity |
| N3 | **Ray 12** | 0.29 | 0.6503 [0.3812 ; 1.1094] | Additivity |
| N2 | **Ray 12** | 0.30 | 0.8290 [0.5545 ; 1.2395] | Additivity |

(continued)

| Experiment | Ray | f values | Ki (confidence interval at 95%) | Interaction characterization |
|---|---|---|---|---|
| N1 | **Ray 11** | 0.52 | 0.8182 [0.4922 ; 1.3602] | Additivity |
| N3 | **Ray 13** | 0.57 | 0.7347 [0.4836 ; 1.1160] | Additivity |
| N2 | **Ray 13** | 0.59 | 0.8713 [0.6481 ; 1.1715] | Additivity |
| N1 | **Ray 12** | 0.76 | 0.7628 [0.5120 ; 1.1364] | Additivity |
| N3 | **Ray 14** | 0.80 | 0.7493 [0.5542 ; 1.0131] | Additivity |
| N2 | **Ray 14** | 0.81 | 0.9370 [0.7408 ; 1.1852] | Additivity |
| N1 | **Ray 13** | 0.91 | 0.9704 [0.6774 ; 1.3900] | Additivity |
| N3 | **Ray 15** | 0.93 | 0.8492 [0.624 ; 1.1551] | Additivity |
| N2 | **Ray 15** | 0.94 | 1.2578 [0.9872 ; 1.6024] | Additivity |

FIG. 17 shows an isobologram of growth inhibition of UM-UC-3 cells treated with the indicated amounts of Compound (1) in combination with Compound (4) (FIG. 17A) or Compound (5) (FIG. 17B), compared to additive growth inhibition. As shown in Tables 50 and 52, and FIG. 17, Compound (1) in combination with Compound (4) or Compound (5) showed additivity on UM-UC-3 cells.

While there have been shown and described what are at present considered the preferred embodiments of the invention, those skilled in the art may make various changes and modifications which remain within the scope of the appended claims.

## Claims

**1.** A method of treating a subject, wherein the subject has cancer, comprising administering to the subject an effective amount of Compound (1)

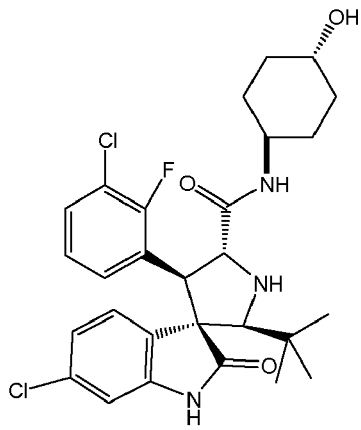

(1)

or a pharmaceutically acceptable salt thereof and Compound (4)

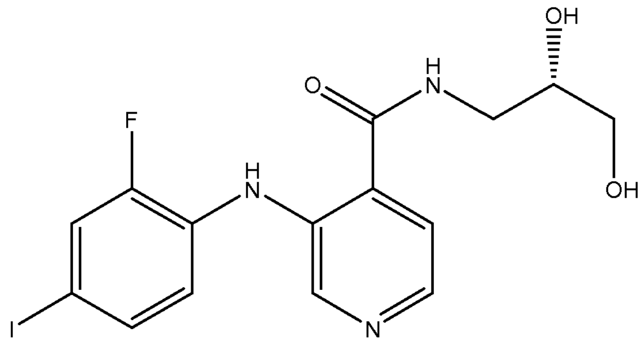

(4)

or a pharmaceutically acceptable salt thereof.

**2.** The method of claim l, wherein Compound (1) and/or Compound (4) are administered with a pharmaceutically acceptable carrier.

**3.** The method of claim 1, wherein Compound (1) and Compound (4) are administered in separate dosage forms.

**4.** The method of claim 1, wherein Compound (1) and Compound (4) are administered in a single dosage form.

**5.** The method of claim 1, wherein Compound (1) and Compound (4) are administered at different times.

**6.** The method of claim 1, wherein Compound (1) and Compound (4) are administered at substantially the same time.

**7.** The method of any one or claims 1-6, wherein the cancer is a mutant Kras expressing colorectal cancer.

**8.** The method of any of claims 1-6, wherein said cancer is selected from the group consisting of: mutant Nras expressing melanoma, mutant Nras expressing bladder cancer, and mutant Braf expressing melanoma.

**9.** The method of claim 8, wherein the effective amount achieves a synergistic effect in the treatment of the subject in need thereof.

**10.** A combination for use in treating cancer, the combination comprising a therapeutically effective amount of Compound (1), or a pharmaceutically acceptable salt thereof, and Compound (4), or a pharmaceutically acceptable salt thereof.

**11.** The combination of claim 10, wherein Compound (1), or a pharmaceutically acceptable salt thereof, and Compound (4), or a pharmaceutically acceptable salt thereof, are in separate dosage forms.

**12.** The combination of claim 11, wherein Compound (1), or a pharmaceutically acceptable salt thereof, and Compound (4), or a pharmaceutically acceptable salt thereof, are in a single dosage form.

**13.** The method of any one or claims 10-12, wherein the cancer is a mutant Kras expressing colorectal cancer.

**14.** The method of any one of claims 10-12, wherein said cancer is selected from the group consisting of: mutant Nras expressing melanoma, mutant Nras expressing bladder cancer, and mutant Braf expressing melanoma.

**15.** The method of claim 14, wherein the therapeutically effective amount achieves a synergistic effect in the treatment of the subject in need thereof.

**16.** A kit comprising: (A) Compound (1), or a pharmaceutically acceptable salt thereof; (B) Compound (4), or a pharmaceutically acceptable salt thereof; and (C) instructions for use.

**17.** Use of a combination comprising a therapeutically effective amount of Compound (1), or a pharmaceutically acceptable salt thereof, and Compound (4), or a pharmaceutically acceptable salt thereof, for the preparation of a medicament for use in treatment of a cancer selected from the group consisting of: mutant Kras expressing colorectal cancer, mutant Nras expressing melanoma, mutant Nras expressing bladder cancer, and mutant Braf expressing

melanoma.

**18.** A composition comprising a compound of the formula (1):

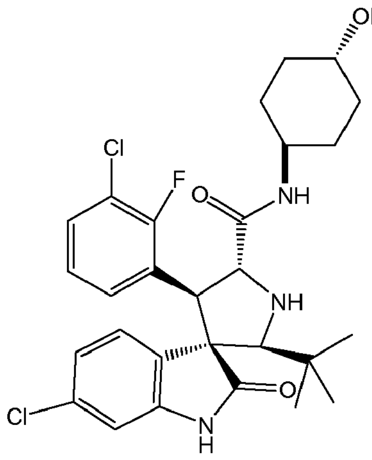

(1)

or a pharmaceutically acceptable salt thereof, and a compound of the formula (4):

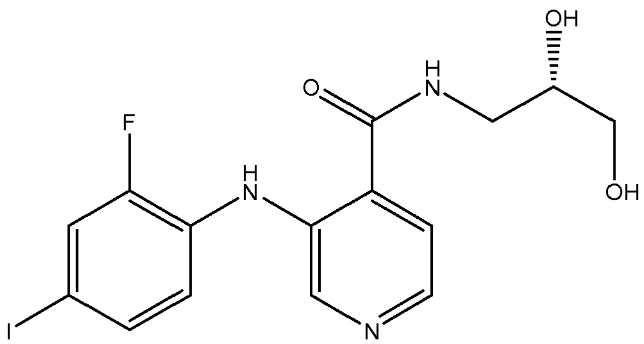

(4)

or a pharmaceutically acceptable salt thereof.

**19.** The composition of claim 18, further comprising a pharmaceutically acceptable carrier.

**20.** The composition of claim 18, wherein said compound of formula (1) and said compound of formula (4) are present in amounts that produce a synergistic effect in the treatment of cancer in a subject in need thereof.

**21.** The composition of claim 20, wherein said cancer is selected from the group consisting of mutant Nras expressing melanoma, mutant Nras expressing bladder cancer, and mutant Braf expressing melanoma.

**22.** A method of treating cancer in a subject in need thereof, comprising administering to the subject an effective amount of Compound (1)

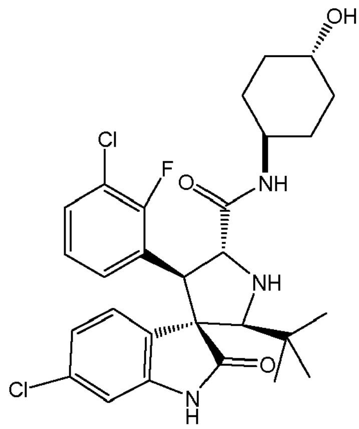

(1)

or a pharmaceutically acceptable salt thereof, and Compound (4)

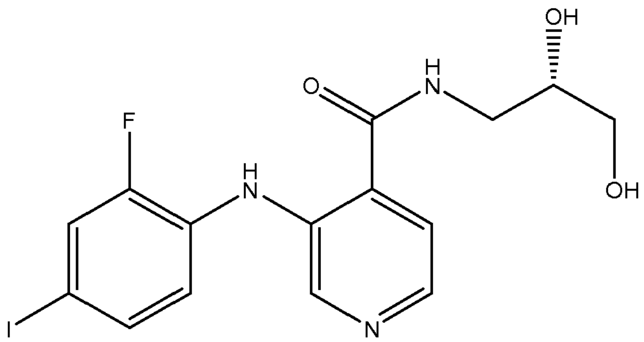

(4)

or a pharmaceutically acceptable salt thereof, wherein the cancer is selected from the group consisting of: mutant Kras expressing colorectal cancer, mutant Nras expressing melanoma, mutant Nras expressing bladder cancer, and mutant Braf expressing melanoma.

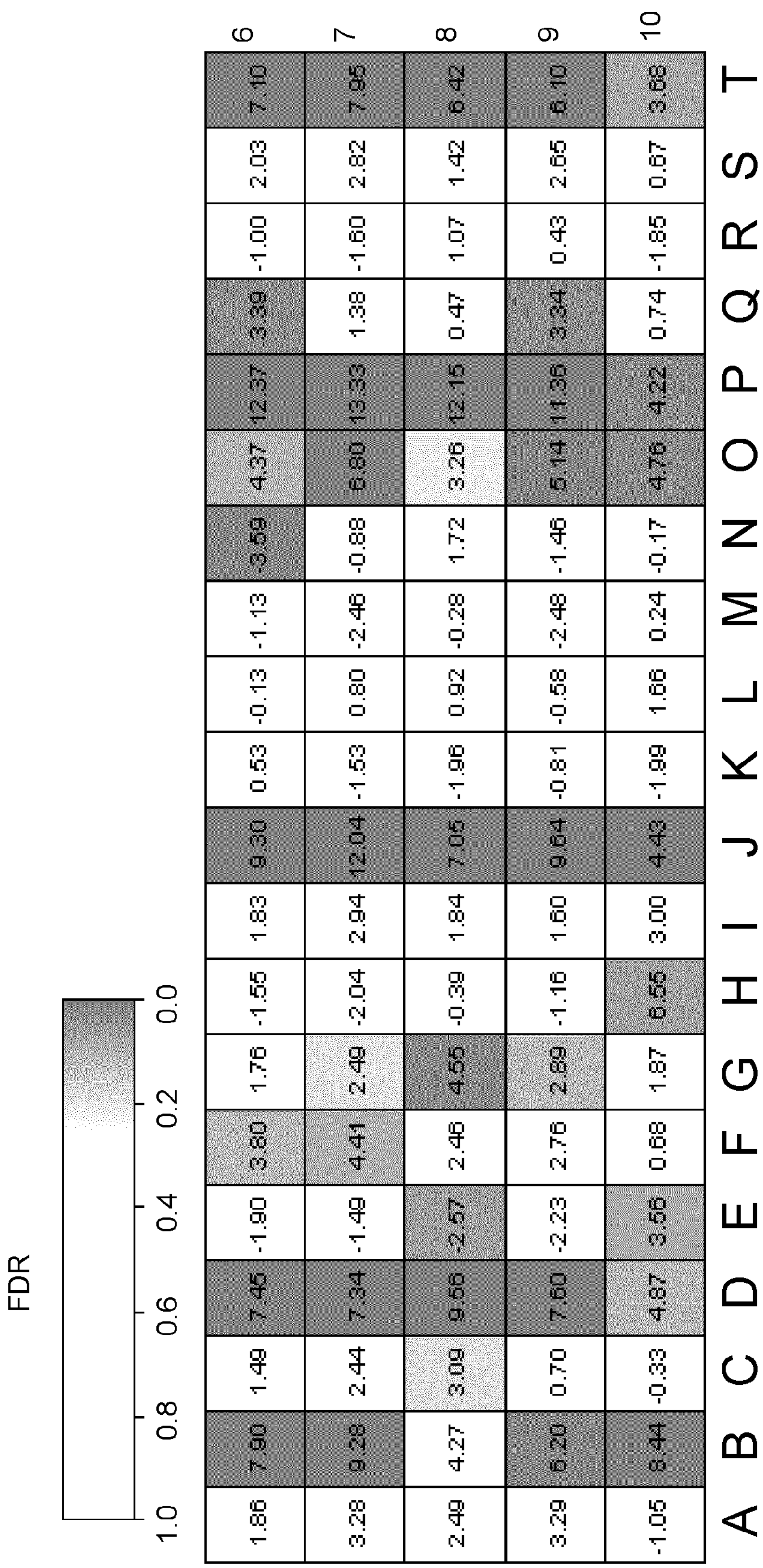
FDR
1.0
0.8
0.6
0.4
0.2
0.0
6
7
8
9
10
A 1.86 3.28 2.49 3.29 -1.05
B 7.90 9.28 4.27 6.20 8.44
C 1.49 2.44 3.09 0.70 -0.33
D 7.45 7.34 9.56 7.60 4.87
E -1.90 -1.49 -2.57 -2.23 3.56
F 3.80 4.41 2.46 2.76 0.68
G 1.76 2.49 4.55 2.89 1.87
H -1.55 -2.04 -0.39 -1.16 6.55
I 1.83 2.94 1.84 1.60 3.00
J 9.30 12.04 7.05 9.64 4.43
K 0.53 -1.53 -1.96 -0.81 -1.99
L -0.13 0.80 0.92 -0.58 1.66
M -1.13 -2.46 -0.28 -2.48 0.24
N -3.59 -0.88 1.72 -1.46 -0.17
O 4.37 6.80 3.26 5.14 4.76
P 12.37 13.33 12.15 11.36 4.22
Q 3.39 1.38 0.47 3.34 0.74
R -1.00 -1.60 1.07 0.43 -1.85
S 2.03 2.82 1.42 2.65 0.67
T 7.10 7.95 6.42 6.10 3.68

FIG. 1

# FIG. 2

A

Growth Inhibition (%)

| | | | | | |
|---|---|---|---|---|---|
| 101 | 142 | 149 | 168 | 185 | 185 |
| 87 | 109 | 114 | 118 | 160 | 176 |
| 63 | 80 | 80 | 75 | 105 | 107 |
| 58 | 65 | 53 | 63 | 77 | 75 |
| 25 | 52 | 37 | 56 | 54 | 53 |
| - | 25 | 37 | 50 | 48 | 64 |
| 0 | .12 | .37 | 1.1 | 3.3 | 9.9 |

Compound (1) (uM)

B

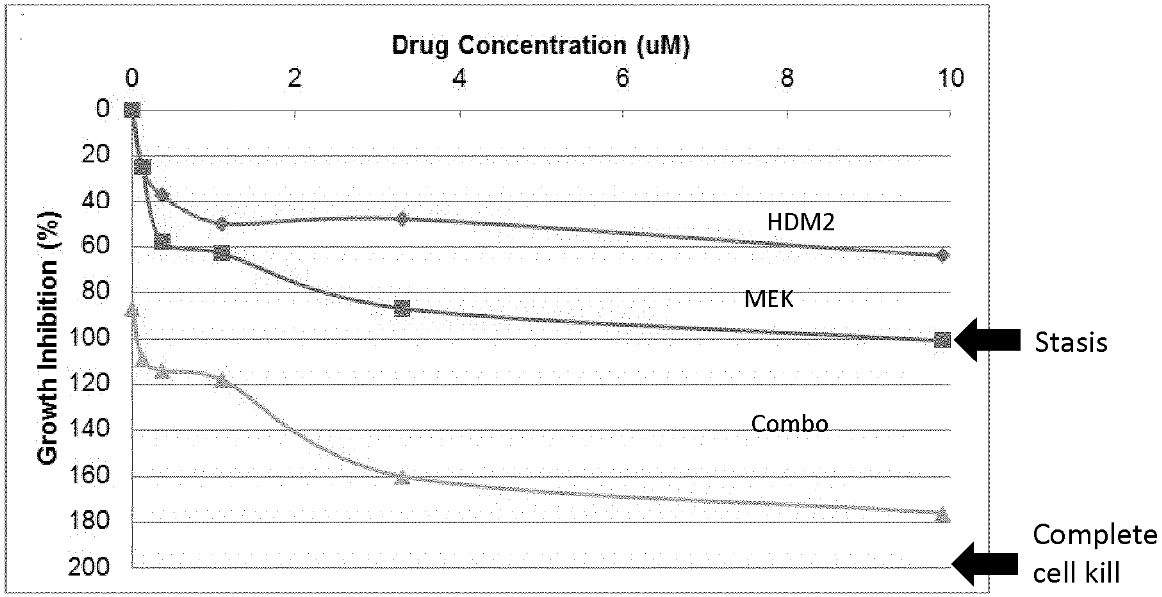

FIG. 3

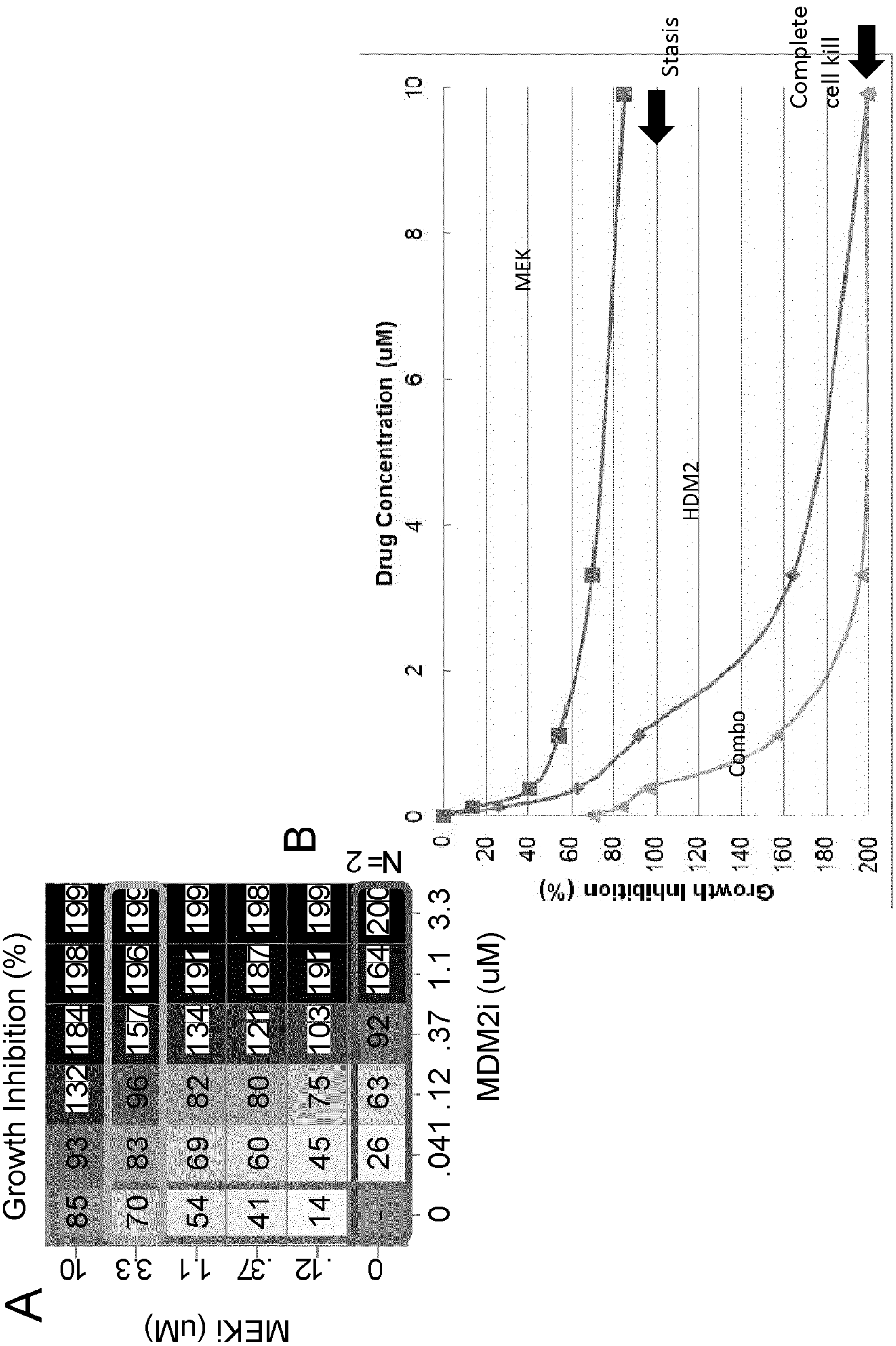
A
Growth Inhibition (%)
MEKi (uM)
10
3.3
1.1
.37
.12
0
85 93 132 184 198 199
70 83 96 157 196 199
54 69 82 134 191 199
41 60 80 121 187 198
14 45 75 103 191 199
- 26 63 92 164 200
0 .041 .12 .37 1.1 3.3
MDM2i (uM)
B
N= 2
Drug Concentration (uM)
0 2 4 6 8 10
Growth Inhibition (%)
0 20 40 60 80 100 120 140 160 180 200
MEK
HDM2
Combo
Stasis
Complete cell kill

FIG. 4

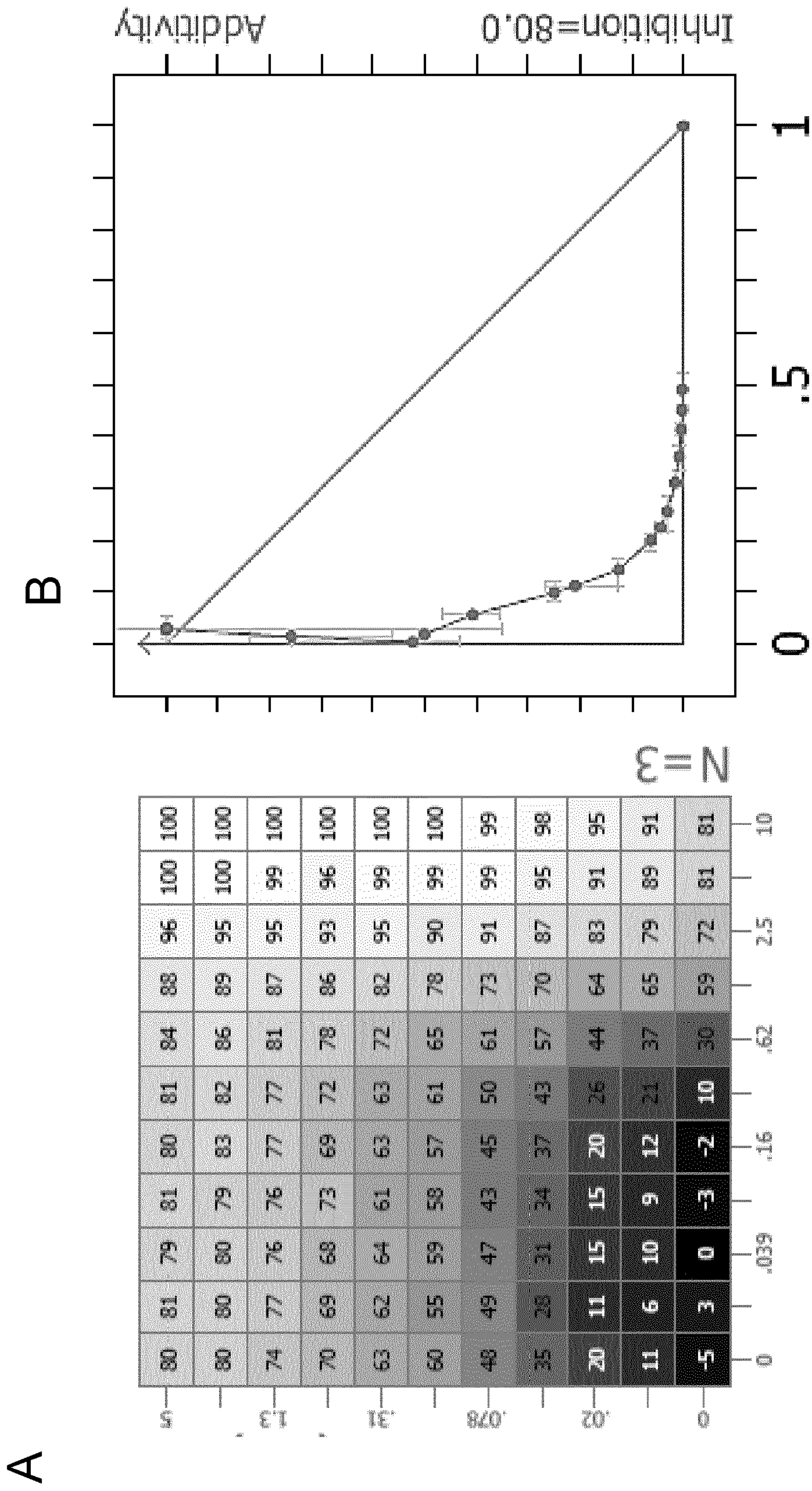
A
B
N=3
Additivity
Inhibition=80.0
0
.5
1
5
1.3
.31
.078
.02
0
.039
.16
.62
2.5
10

FIG. 5

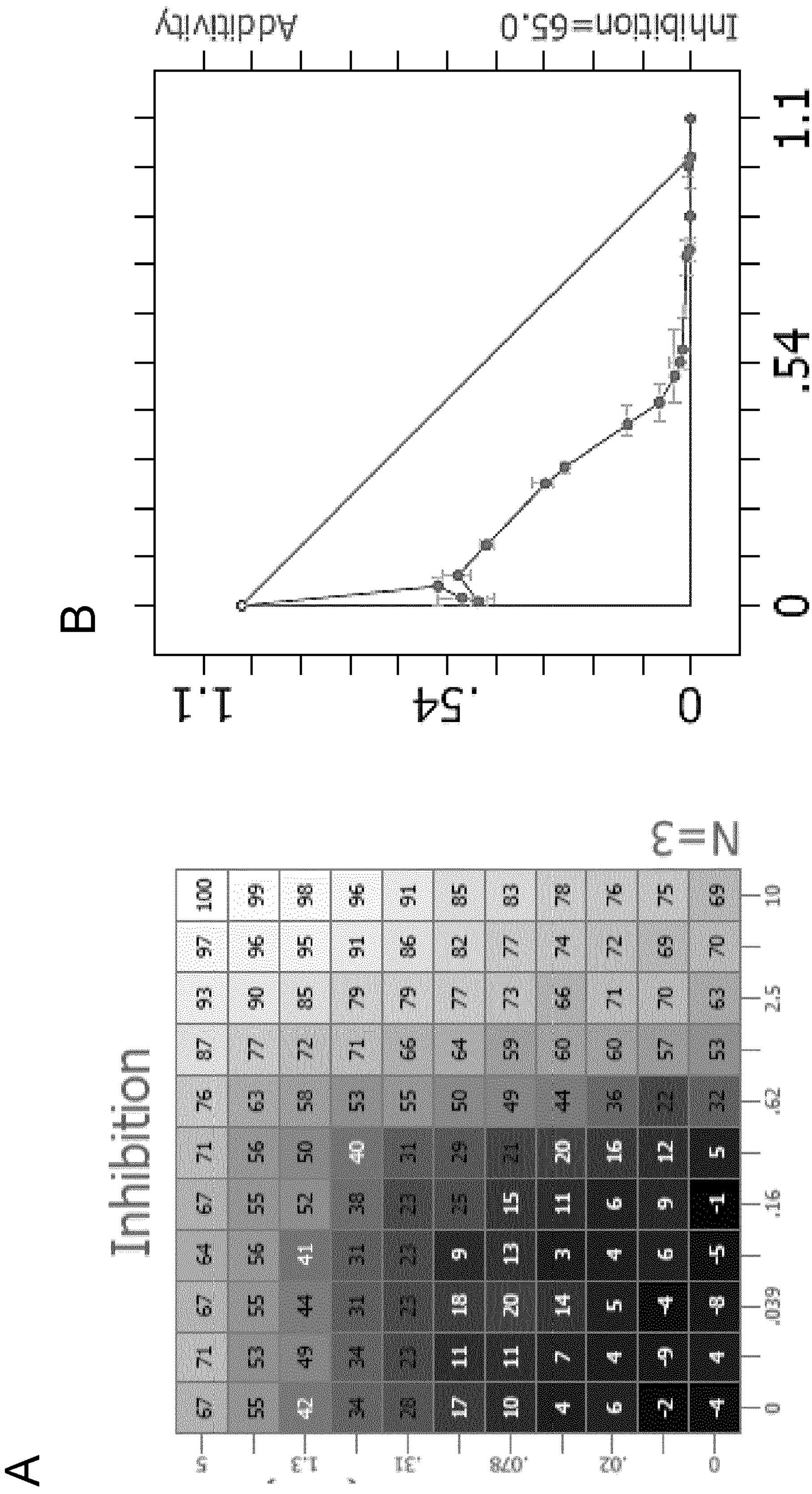
A
Inhibition
N=3
5
1.3
.31
.078
.02
0
.039
.16
.62
2.5
10
B
Additivity
Inhibition=65.0
1.1
.54
0

FIG. 6

A

B

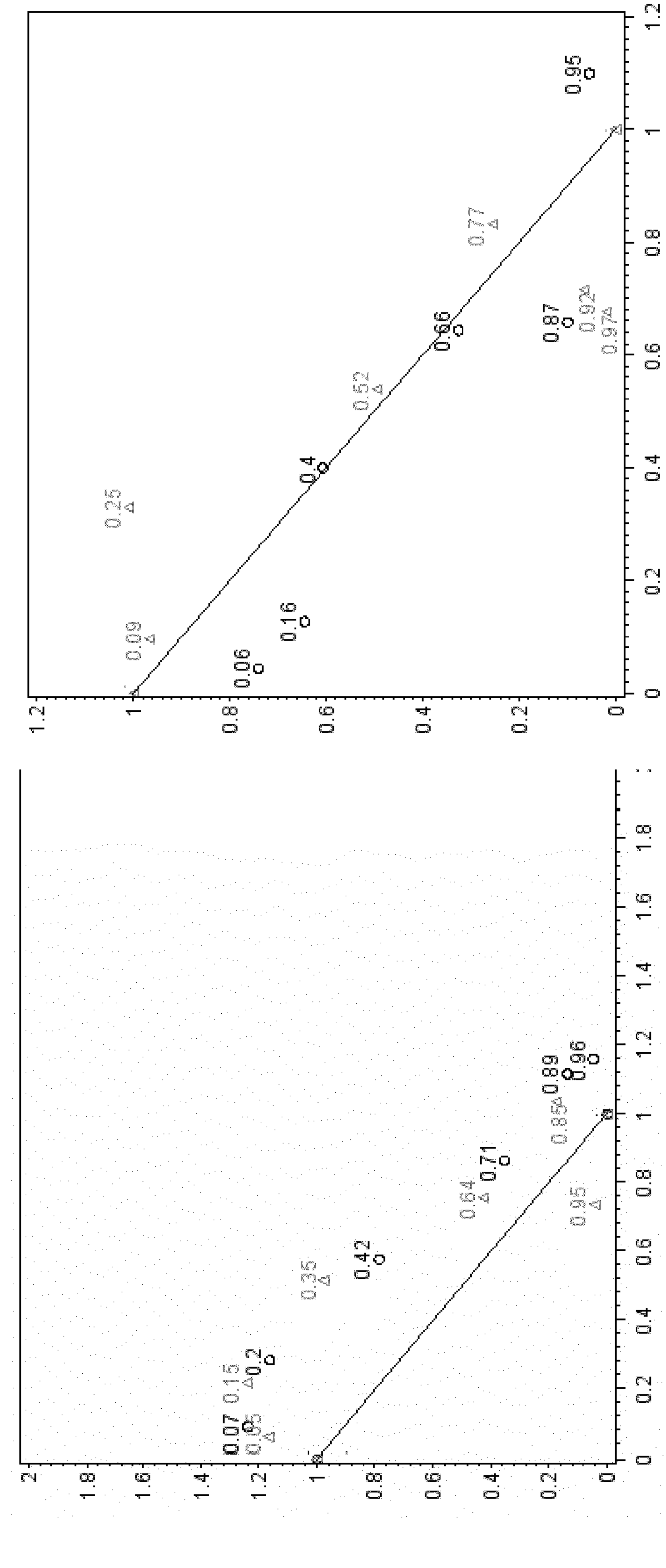
0.07
0.05
0.15
0.2
0.35
0.42
0.64
0.71
0.85
0.89
0.96
0.95
0.09
0.06
0.16
0.25
0.4
0.52
0.66
0.77
0.87
0.92
0.97
0.95
0
0.2
0.4
0.6
0.8
1
1.2
1.4
1.6
1.8
2

FIG. 7

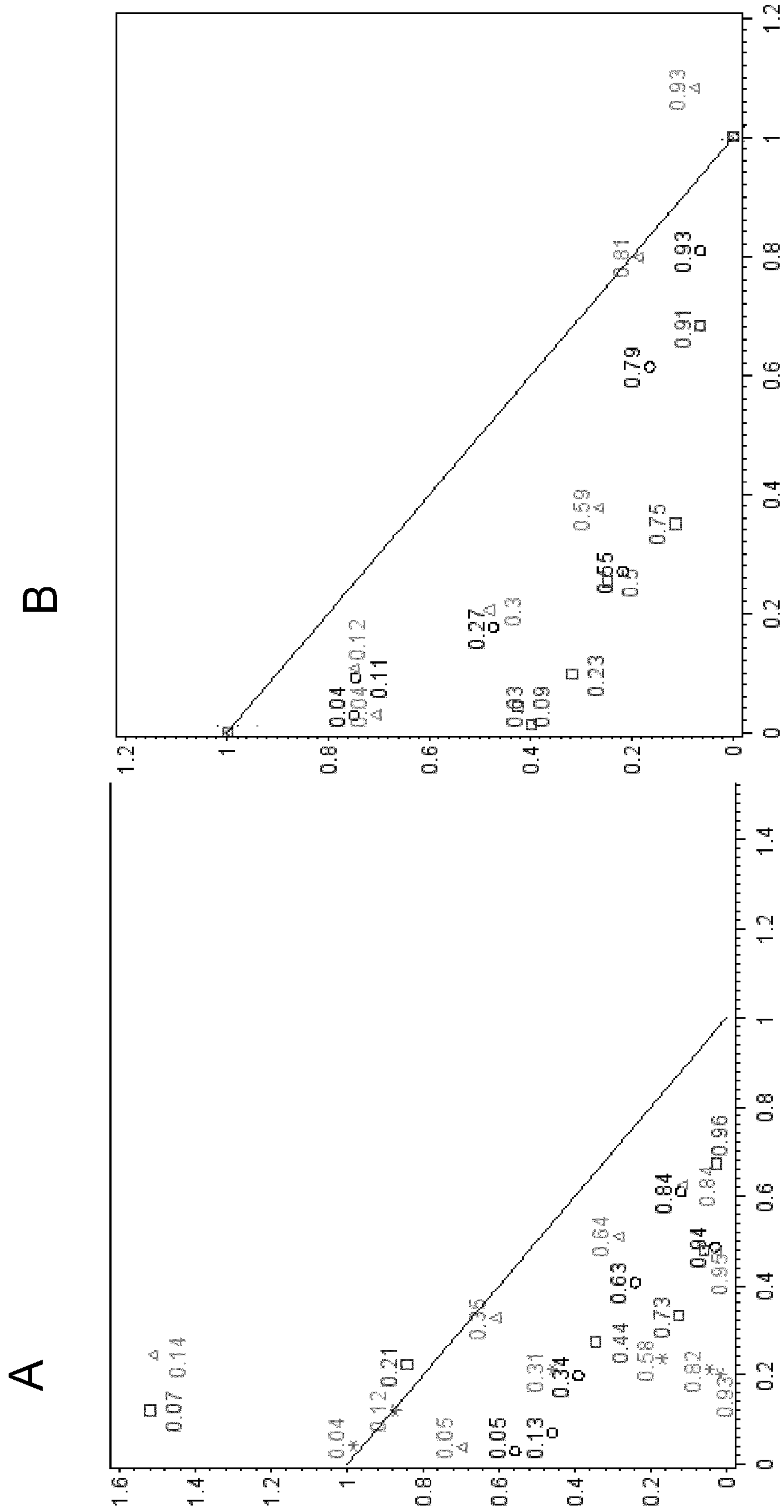

FIG. 8

A

B

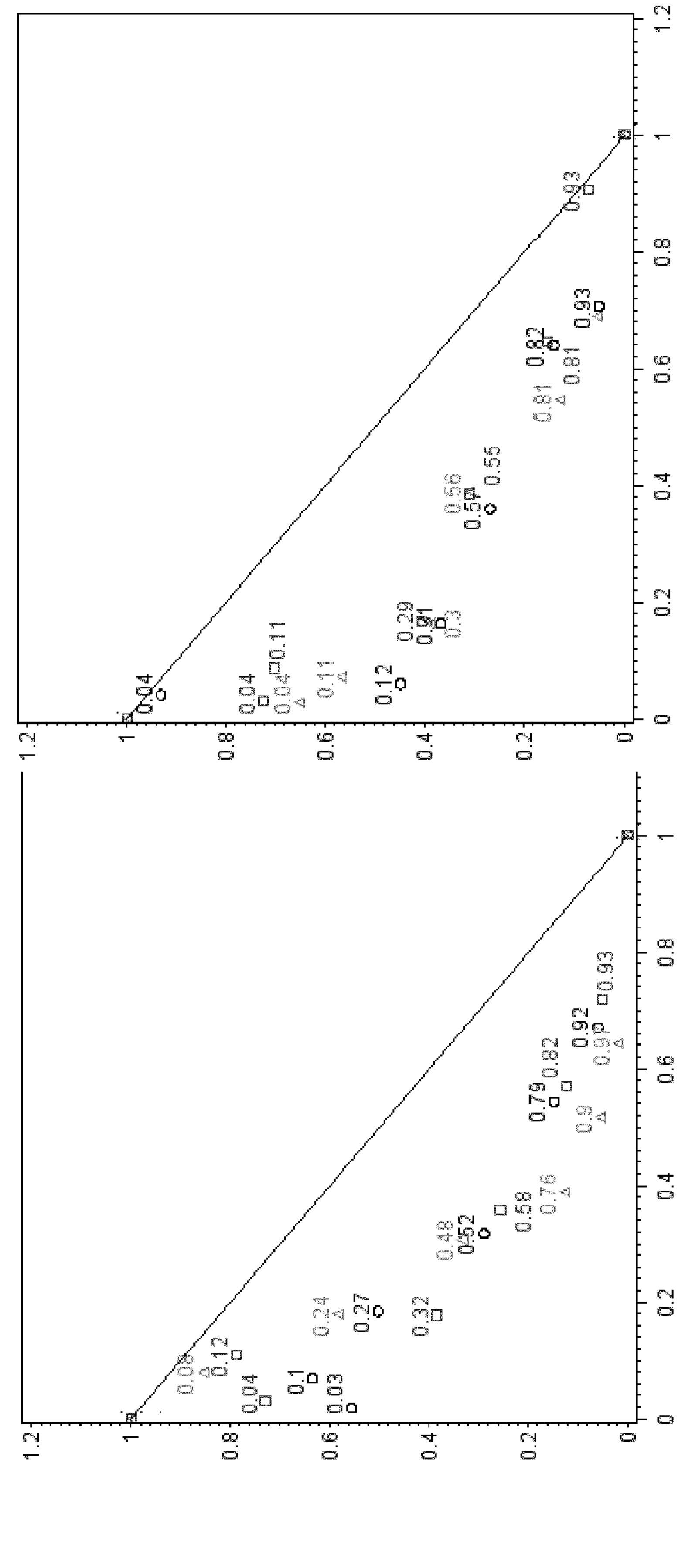

FIG. 9

A

B

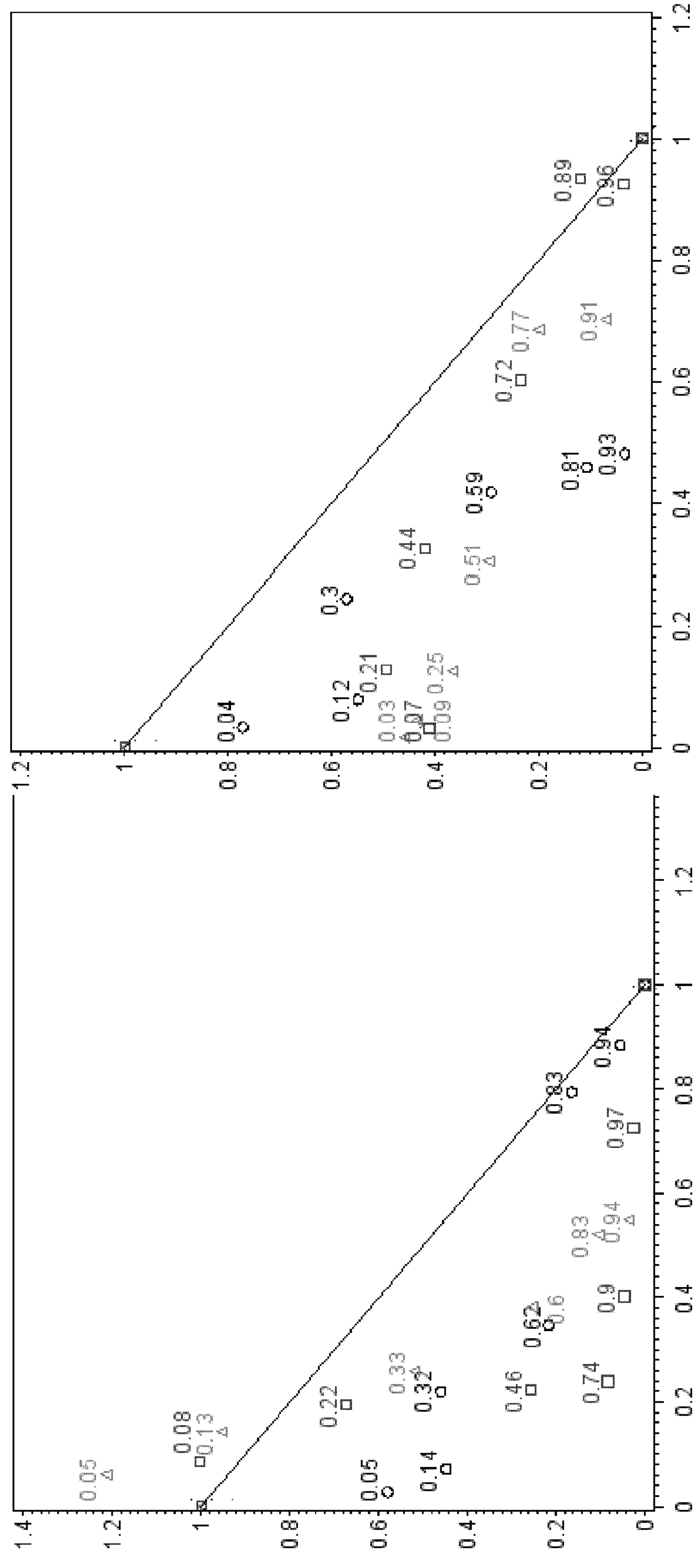

0
0.2
0.4
0.6
0.8
1
1.2
1.4
0.05
0.08
0.13
0.22
0.33
0.32
0.14
0.05
0.46
0.62
0.6
0.74
0.9
0.83
0.94
0.97
0.83
0.94
0.04
0.12
0.21
0.03
0.07
0.09
0.25
0.3
0.44
0.51
0.59
0.81
0.93
0.72
0.77
0.91
0.89
0.96

FIG. 10

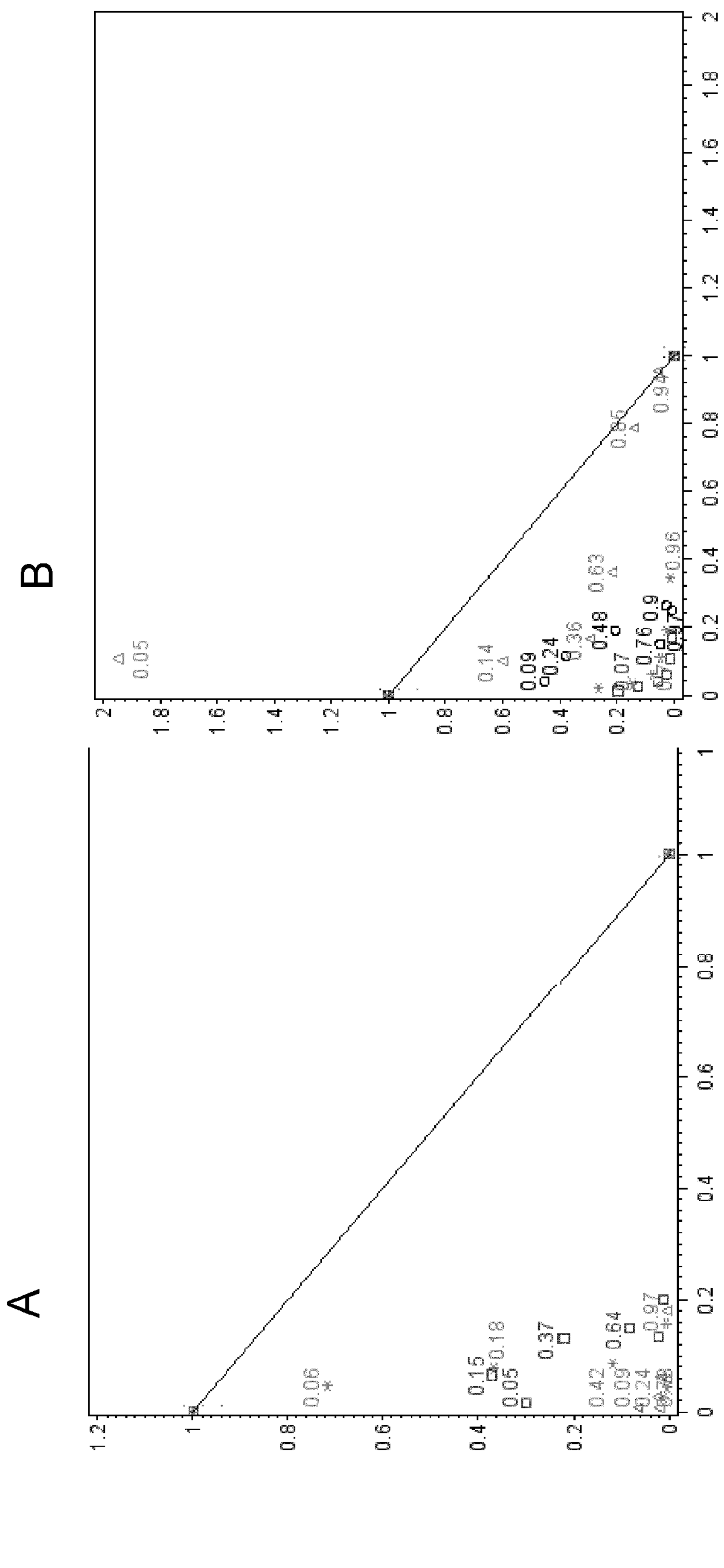

FIG. 11

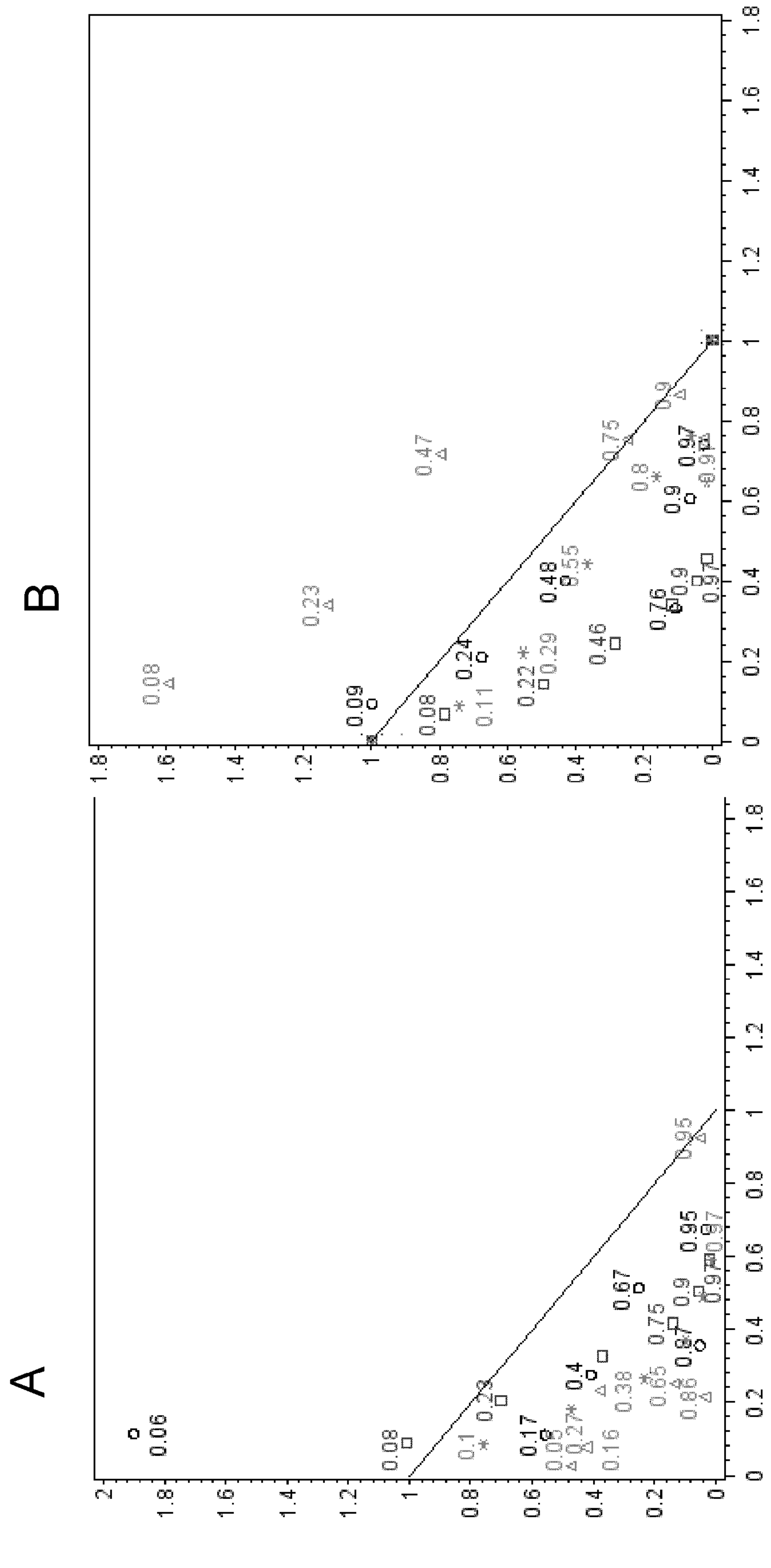

FIG. 12

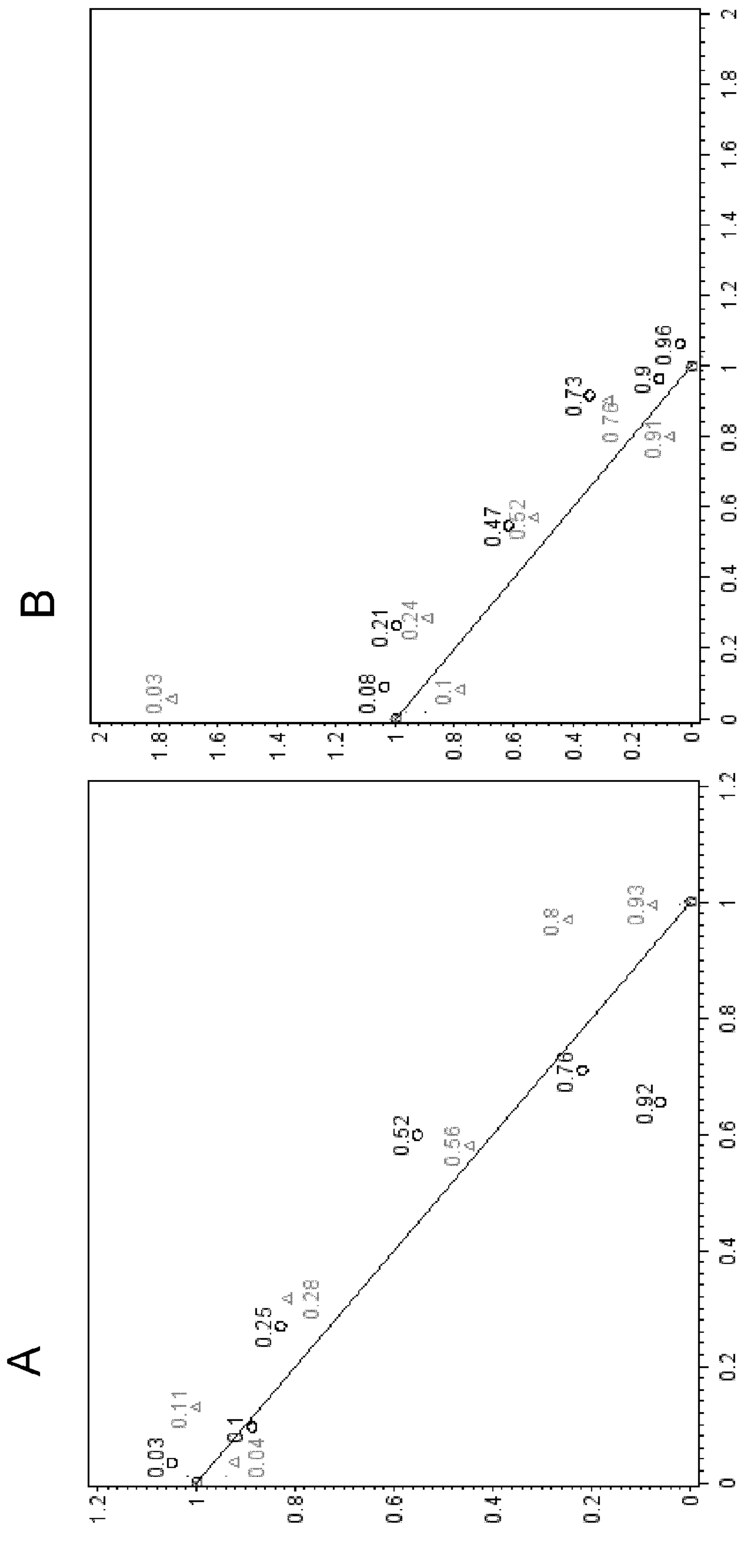

FIG. 13

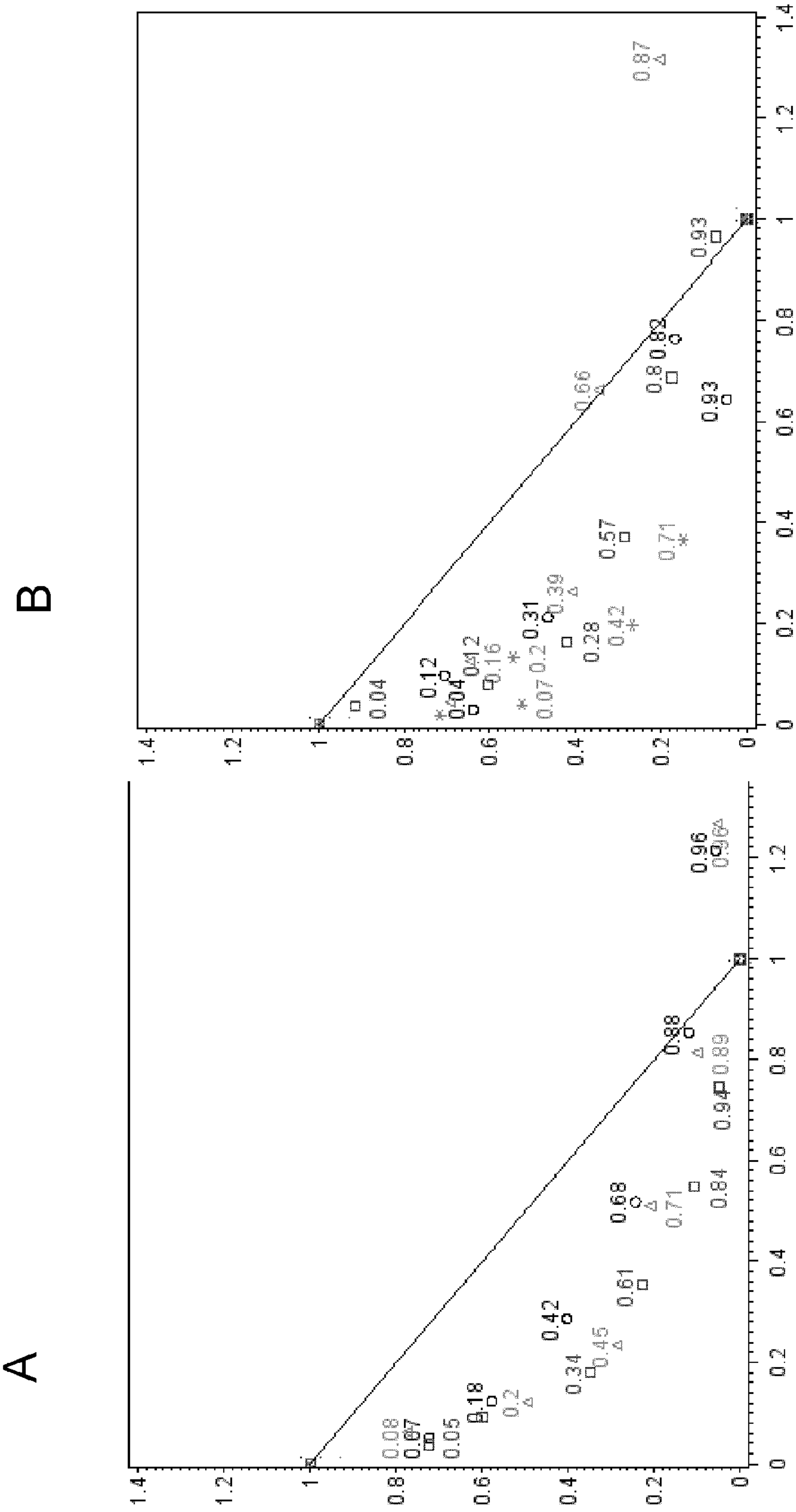

FIG. 14

A

B

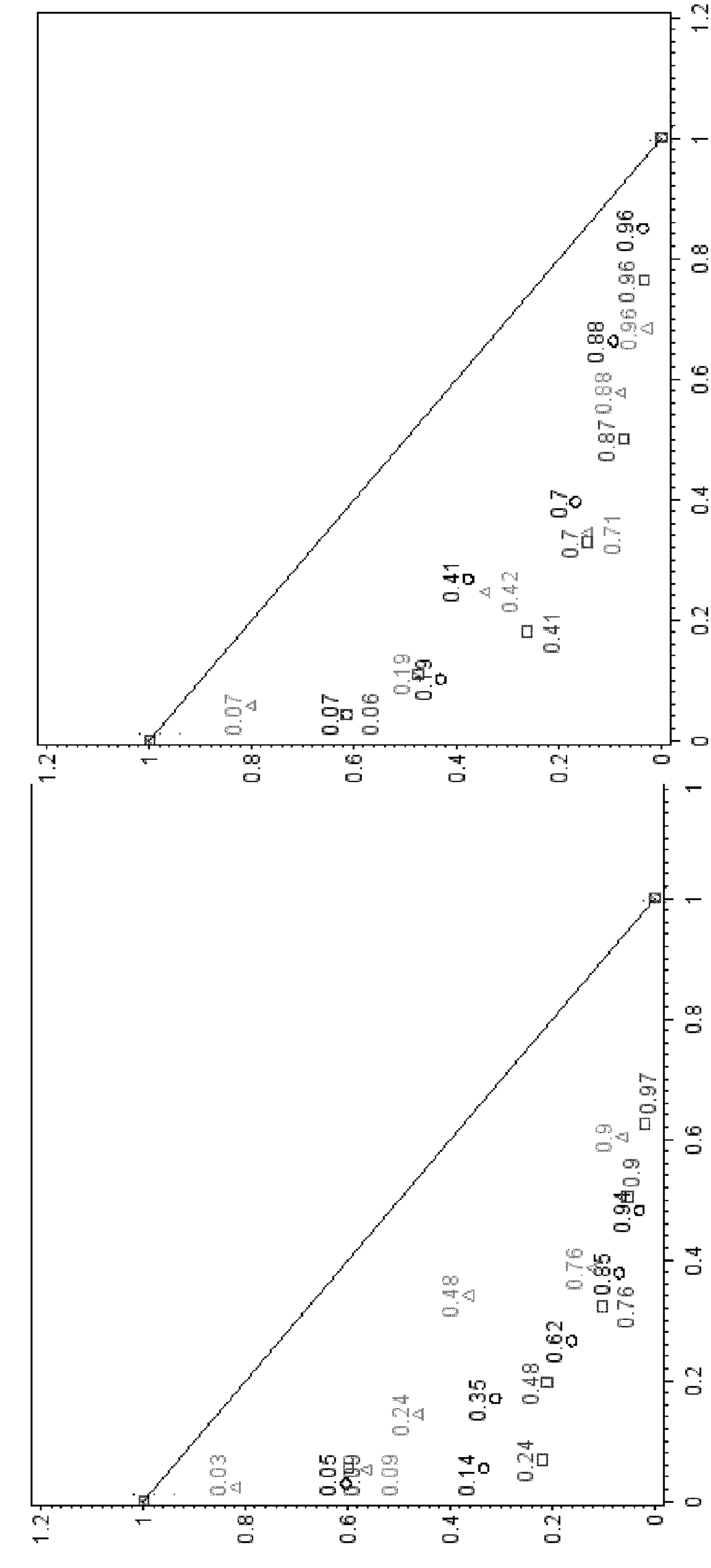

FIG. 15

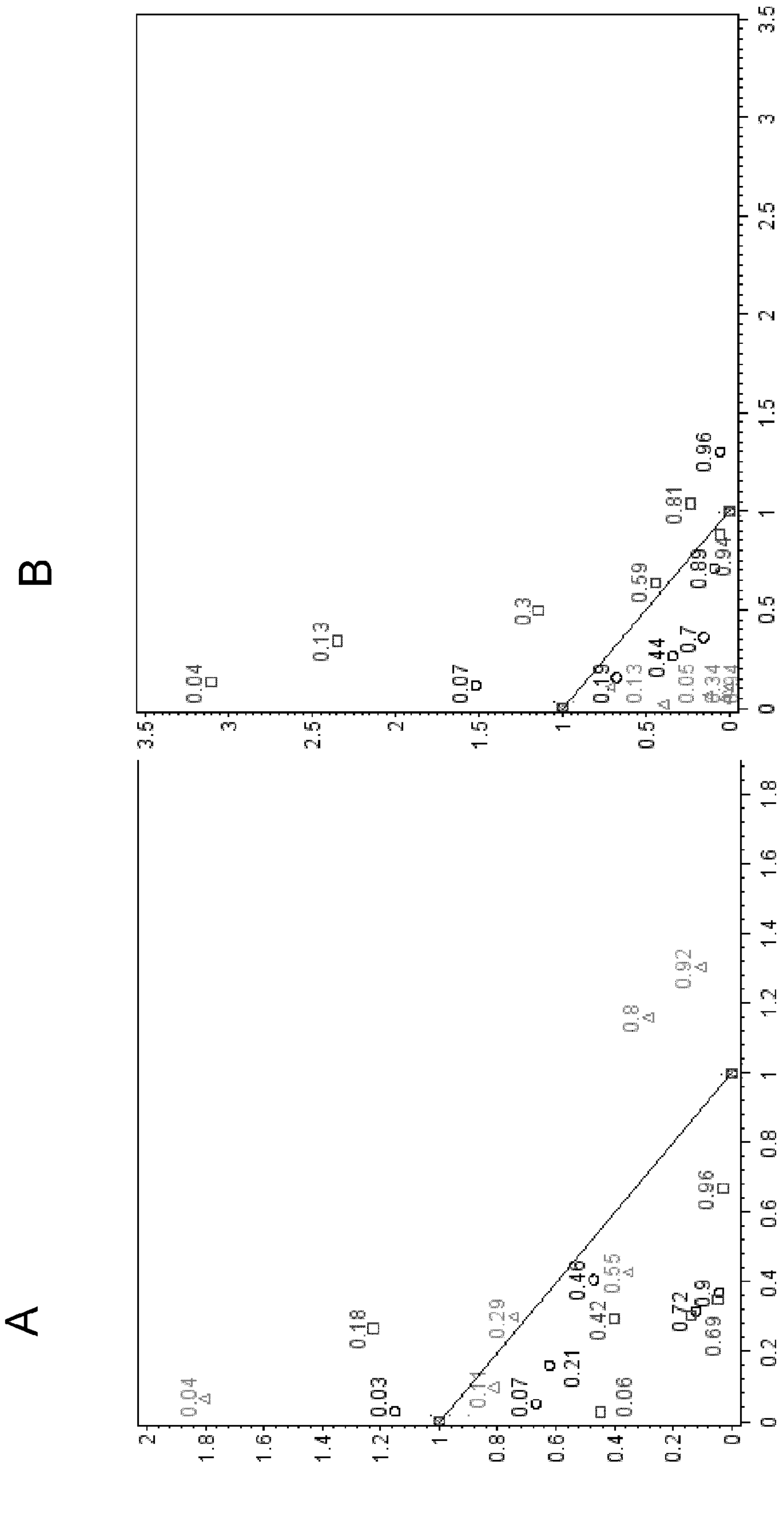

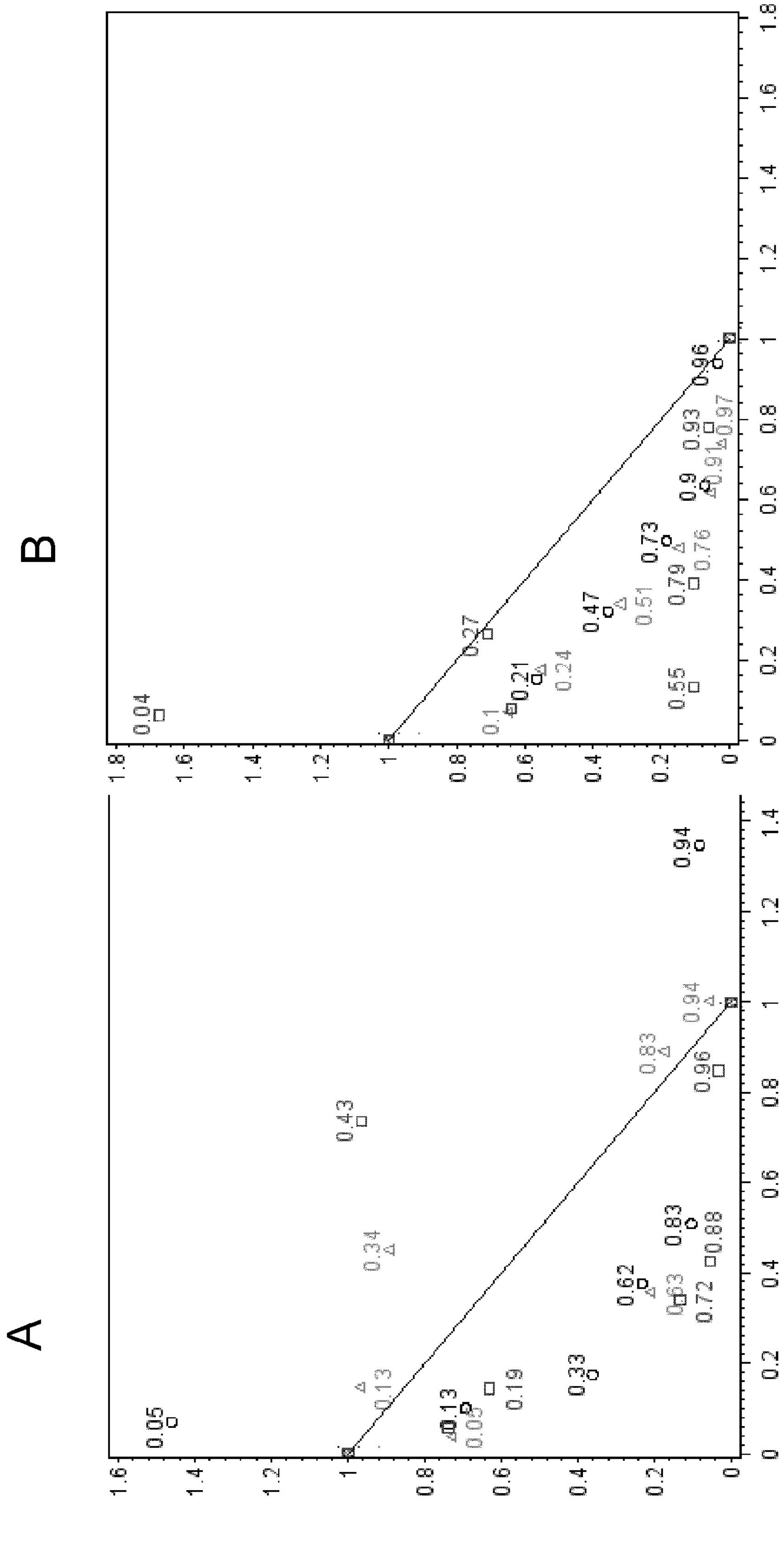

FIG. 16

FIG. 17

A B

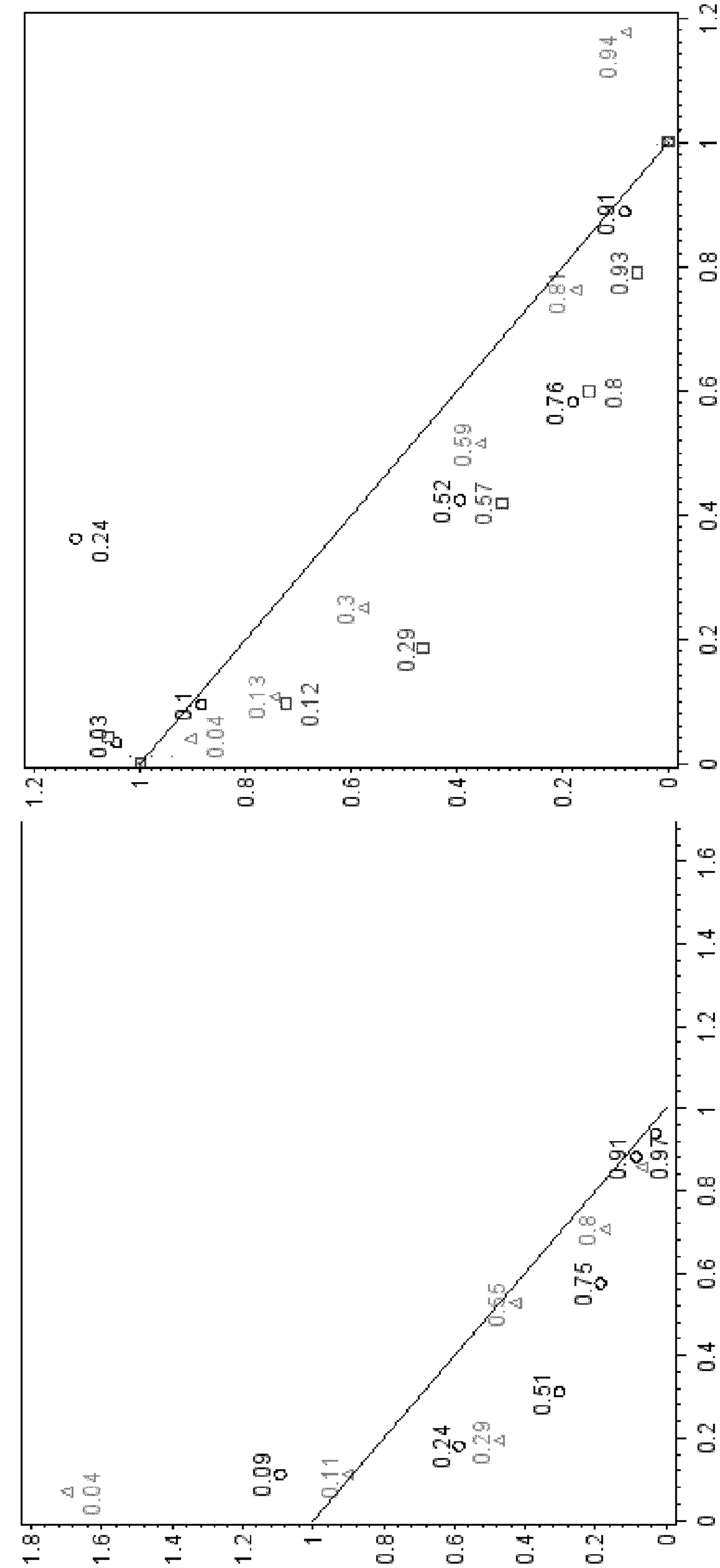

Europäisches Patentamt
European Patent Office
Office européen des brevets

# EUROPEAN SEARCH REPORT

Application Number
EP 13 30 5008

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JI ZHENYU ET AL: "p53 rescue through HDM2 antagonism suppresses melanoma growth and potentiates MEK inhibition.", THE JOURNAL OF INVESTIGATIVE DERMATOLOGY FEB 2012, vol. 132, no. 2, February 2012 (2012-02), pages 356-364, XP002694638, ISSN: 1523-1747 * the whole document * ----- | 1-22 | INV. A61K31/404 A61K31/455 A61K31/44 A61P35/00 |
| Y | ZHANG WEIGUO ET AL: "MEK inhibitor AZD6244 induces cell growth arrest and synergizes nutlin-3a-mediated cell death by upregulating p53 and PUMA levels in acute myelogenous leukemia", BLOOD, vol. 110, no. 11, Part 1, November 2007 (2007-11), page 201A, XP002694639, & 49TH ANNUAL MEETING OF THE AMERICAN-SOCIETY-OF-HEMATOLOGY; ATLANTA, GA, USA; DECEMBER 08 -11, 2007 ISSN: 0006-4971 * abstract * ----- | 1-22 | |
| X | WO 2008/005266 A2 (SCHERING CORP [US]; WANG YAOLIN [US]; ZHANG RUMIN [US]; MA YAO [US]; L) 10 January 2008 (2008-01-10) * page 1, lines 5-11 * * page 9, lines 3-10 * * page 10, lines 3, 16, 22-23 * * page 11, lines 25-27 * ----- -/-- | 1-22 | |

TECHNICAL FIELDS SEARCHED (IPC)
A61K
A61P

1

The present search report has been drawn up for all claims

| Place of search | Date of completion of the search | Examiner |
|---|---|---|
| Munich | 28 March 2013 | Houyvet-Landriscina |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 13 30 5008

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | WO 2012/065022 A2 (UNIV MICHIGAN [US]; ASCENTA THERAPEUTICS INC [US]; SANOFI SA [FR]; WAN) 18 May 2012 (2012-05-18)<br>* page 3, paragraph 9-10 - page 4 *<br>* page 6, paragraph 21 *<br>* page 73, paragraph 240 *<br>* page 83, paragraph 269 - page 84, paragraph 271 *<br>* page 94, paragraph 284 *<br>* table 2A *<br>* page 169; example 11 *<br>----- | 1-22 | |
| Y,D | WO 2006/045514 A1 (APPLIED RESEARCH SYSTEMS [NL]; ABEL ULRICH [DE]; DEPPE HOLGER [DE]; FE) 4 May 2006 (2006-05-04)<br>* page 1, paragraph 1-2 *<br>* page 3, paragraph 5 *<br>* page 22, paragraph 2-3 *<br>* page 76; example 115 *<br>----- | 1-22 | |
| Y | J. YOON ET AL: "MEK1/2 Inhibitors AS703026 and AZD6244 May Be Potential Therapies for KRAS Mutated Colorectal Cancer That Is Resistant to EGFR Monoclonal Antibody Therapy",<br>CANCER RESEARCH,<br>vol. 71, no. 2,<br>15 January 2011 (2011-01-15), pages 445-453, XP055058022,<br>ISSN: 0008-5472, DOI: 10.1158/0008-5472.CAN-10-3058<br>* abstract *<br>* page 445 - page 446, column 1, paragraph 1 *<br>* page 450, column 2 *<br>-----<br>-/-- | 1-22 | TECHNICAL FIELDS SEARCHED (IPC) |

The present search report has been drawn up for all claims

1

| Place of search | Date of completion of the search | Examiner |
|---|---|---|
| Munich | 28 March 2013 | Houyvet-Landriscina |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................................................
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 13 30 5008

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | SOLIT DAVID B ET AL: "BRAF mutation predicts sensitivity to MEK inhibition", NATURE: INTERNATIONAL WEEKLY JOURNAL OF SCIENCE, NATURE PUBLISHING GROUP, UNITED KINGDOM, vol. 439, no. 7074, 19 January 2006 (2006-01-19), pages 358-362, XP002547208, ISSN: 0028-0836, DOI: 10.1038/NATURE04304 * abstract * ----- | 1-22 | |

TECHNICAL FIELDS SEARCHED (IPC)

The present search report has been drawn up for all claims

1

| Place of search | Date of completion of the search | Examiner |
|---|---|---|
| Munich | 28 March 2013 | Houyvet-Landriscina |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 13 30 5008

This annex lists the patent family members relating to the patent documents cited in the above-mentioned European search report. The members are as contained in the European Patent Office EDP file on
The European Patent Office is in no way liable for these particulars which are merely given for the purpose of information.

28-03-2013

| Patent document cited in search report | Publication date | Patent family member(s) | Publication date |
|---|---|---|---|
| WO 2008005266 A2 | 10-01-2008 | CA 2656393 A1 | 10-01-2008 |
| | | CL 19202007 A1 | 22-02-2008 |
| | | CN 101511361 A | 19-08-2009 |
| | | EP 2037919 A2 | 25-03-2009 |
| | | JP 2009542664 A | 03-12-2009 |
| | | TW 200811139 A | 01-03-2008 |
| | | US 2008004286 A1 | 03-01-2008 |
| | | WO 2008005266 A2 | 10-01-2008 |
| WO 2012065022 A2 | 18-05-2012 | US 2012122947 A1 | 17-05-2012 |
| | | UY 33725 A | 29-06-2012 |
| | | WO 2012065022 A2 | 18-05-2012 |
| WO 2006045514 A1 | 04-05-2006 | AR 051248 A1 | 03-01-2007 |
| | | AU 2005298932 A1 | 04-05-2006 |
| | | BR PI0518192 A | 04-11-2008 |
| | | CA 2582247 A1 | 04-05-2006 |
| | | CN 101065358 A | 31-10-2007 |
| | | EA 200700902 A1 | 26-10-2007 |
| | | EP 1802579 A1 | 04-07-2007 |
| | | JP 2008517024 A | 22-05-2008 |
| | | KR 20070067727 A | 28-06-2007 |
| | | US 2009093462 A1 | 09-04-2009 |
| | | US 2011224192 A1 | 15-09-2011 |
| | | US 2012295889 A1 | 22-11-2012 |
| | | WO 2006045514 A1 | 04-05-2006 |
| | | ZA 200703912 A | 25-09-2008 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2012065022 A
- WO 06045514 A
- WO 2005121142 A
- WO 03077914 A
- WO 2002006213 A
- RO 5126766
- US 20100004233 A


### Non-patent literature cited in the description


- **D. B. SOLIT et al.** *Nature,* 2006, vol. 439, 358-362
- **HAURA et al.** *Clin Cancer Res,* 2010, vol. 16 (8), 2450-2457
- **BALADULA et al.** *Invest New Drugs,* 2011, vol. 29, 1114-1121
- Remington's Pharmaceutical Sciences. Mack Publishing Company, 1985
- **S. M. BERGE et al.** Pharmaceutical Salts. *J. Pharm. Sci.,* 1977, vol. 66, 1-19
- **HOLFORD, N. H. G. ; SCHEINER, L. B.** *Clin. Pharmacokinet.,* 1981, vol. 6, 429-453
- **LOEWE, S. ; MUISCHNEK, H.** *Arch. Exp. Pathol Pharmacol.,* 1926, vol. 114, 313-326
- **CHOU, T. C. ; TALALAY, P.** *Adv. Enzyme Regul.,* 1984, vol. 22, 27-55
- Remington's Pharmaceutical Sciences. Mack Publishing Company, 1990
- **ARONOV et al.** *J.Med. Chem.,* 2009, vol. 52, 6362-6368
- **ZHANG et al.** *Bioor. Med. Chem. Let.,* 2000, vol. 10, 2825-2828
- **R. STRAETEMANS.** *Biometrical journal,* 2005, 47